# EUROPEAN PATENT SPECIFICATION

(11) **EP 3 433 250 B1**
(45) Date of publication and mention of the grant of the patent: **08.09.2021**
(21) Application number: 17710962.6
(22) Date of filing: 16.03.2017
(51) Int. Cl.: C07D 405/14, C07D 413/14, A01N 43/40, C07D 213/75, C07D 213/89, C07D 401/04, C07D 409/14, C07D 417/14

(54) **HERBICIDES**
HERBIZIDE
HERBICIDES

(30) Priority: 23.03.2016 GB 201604979; 15.04.2016 GB 201606639
(43) Date of publication of application: 30.01.2019
(73) Proprietor: Syngenta Participations AG, 4058 Basel (CH)
(72) Inventor: CARTER, Neil, Brian, Bracknell Berkshire RG42 6EY (GB); BRIGGS, Emma, Bracknell Berkshire RG42 6EY (GB); MORRIS, James, Alan, Bracknell Berkshire RG42 6EY (GB); MORRIS, Melloney, Bracknell Berkshire RG42 6EY (GB); TATE, Joseph, Andrew, Bracknell Berkshire RG42 6EY (GB); WAILES, Jeffrey, Steven, Bracknell Berkshire RG42 6EY (GB); WILLIAMS, John, Bracknell Berkshire RG42 6EY (GB)
(74) Representative: SYNGENTA IP
(86) International application number: PCT/EP2017/056286
(87) International publication number: WO 2017/162522

(56) References cited:
- WO-A1-2015/052076
- JP-A- 2014 208 631
- US-A1- 2013 005 574

## Description

The present invention relates to herbicidally active pyridino-/pyrimidino-pyridine derivatives, as well as to processes and intermediates used for the preparation of such derivatives. The invention further extends to herbicidal compositions comprising such derivatives, as well as to the use of such compounds and compositions in controlling undesirable plant growth: in particular the use in controlling weeds, in crops of useful plants.

Certain pyrido-pyridine and pyrimidino-pyridine derivatives are known from JP2014-208631, where they are stated to have activity as insecticidal agents, and in particular miticidal agents.

The present invention is based on the finding that pyridino-pyridine, and pyrimidino-pyridine, derivatives of Formula (I) as defined herein, exhibit surprisingly good herbicidal activity. Thus, according to the present invention there is provided the use of a compound of Formula (I) 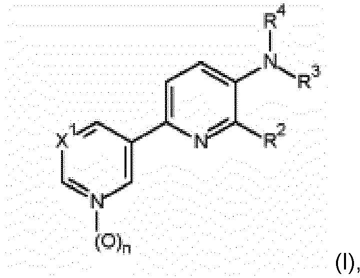 or a salt thereof, wherein,
X¹ is N or CR¹;
R¹ is selected from the group consisting of hydrogen, halogen, cyano, C₁-C₆alkyl, C₃-C₆cycloalkyl, C₂-C₆alkenyl, C₂-C₆alkynyl, C₁-C₆alkoxy, -C(O)OC₁-C₆alkyl, -S(O)ₚC₁-C₆alkyl, NR⁶R⁷, C₁-C₆haloalkoxy and C₁-C₆haloalkyl;
R² is selected from the group consisting of halogen, cyano, nitro, C₁-C₆alkyl, C₁-C₆haloalkyl, C₂-C₆alkenyl, C₂-C₆alkynyl, C₃-C₆cycloalkyl, -C(O)OC₁-C₆alkyl, -S(O)ₚ(C₁-C₆alkyl), C₁-C₆alkoxy, C₁-C₆haloalkoxy and phenyl;
R³ is -C(O)X²R¹²;
X² is O or NR¹⁰;
when X² is O, R¹² is selected from the group consisting of C₁-C₆alkyl, CᵣalkoxyCₛalkyl, C₁-C₆haloalkyl, CᵣalkoxyCₛhaloalkyl, CᵣalkylthioCₛalkyl, C₂-C₆alkenyl, C₂-C₆alkynyl, and -(CR^{a}R^{b})_{q}R¹¹;
when X² is NR¹⁰, R¹² is selected from the group consisting of hydrogen, C₁-C₆alkyl, C₁-C₆alkoxy, C₁-C₆haloalkyl, C₁-C₆haloalkoxy, CᵣalkylthioCₛalkyl, C₂-C₆alkenyl, C₂-C₆alkynyl, and -(CR^{a}R^{b})_{q}R¹¹;
R¹⁰ is selected from the group consisting of hydrogen, C₁-C₆alkyl, C₃-C₆cycloalkyl;
or, R¹⁰ and R¹² together with the nitrogen atom to which they are joined, can form a 5-, 6-, or 7-membered ring, optionally containing 1 to 3 additional heteroatoms each independently selected from O, N or S, wherein when said ring contains a ring sulphur said ring sulphur is in the form S(O)ₚ ;
R⁴ is selected from the group consisting of hydrogen, C₁-C₆alkyl, C₁-C₆alkoxy, C₁-C₆haloalkyl, C₁-C₆haloalkoxy, C₃-C₆cyloalkyl, C₃-C₆alkenyl, C₃-C₆alkynyl, -C(O)R⁹ and - (CR^{a}R^{b})_{q}R⁵;
R^{a} is hydrogen or C₁-C₂ alkyl;
R^{b} is hydrogen or C₁-C₂ alkyl;
R⁵ is cyano, -C(O)OC₁-C₆alkyl, -C₃-C₆cycloalkyl, -aryl or -heteroaryl wherein said aryl and heteroaryl are optionally substituted by 1 to 3 independent R⁸;
R⁶ and R⁷ are independently selected from the group consisting of hydrogen and C₁-C₆alkyl;
each R⁸ is independently selected from the group consisting of halogen, C₁-C₆ alkyl and C₁-C₆alkoxy-, C₁-C₆ haloalkyl, C₁-C₆ haloalkoxy-, cyano and S(O)ₚ(C₁-C₆alkyl);
R⁹ is selected from the group consisting of hydrogen, C₁-C₆alkyl, C₁-C₆alkoxy, C₁-C₆haloalkyl, C₁-C₆haloalkoxy, C₂-C₆alkenyl, C₂-C₆alkynyl, and -(CR^{a}R^{b})_{q}R¹¹;
or R⁴ and R¹⁰ together with the atoms to which they are joined form a 5-7 membered ring system optionally containing from 1 to 3 additional heteroatoms independently selected from S, O and N;
or R⁴ and R¹² together with the atoms to which they are joined form a 5-7 membered ring system optionally containing from 1 to 3 additional heteroatoms independently selected from S, O and N;
R¹¹ is cyano, -C₃-C₆cycloalkyl, or an -aryl, -heteroaryl or -heterocyclyl ring, wherein said ring is optionally substituted by 1 to 3 independent R⁸, and wherein when said ring contains a ring sulphur, said ring sulphur is in the form S(O)ₚ;
n is 0 or 1;
p is 0, 1, or 2;
q is 0, 1, 2, 3, 4, 5 or 6;
r is 1, 2, 3, 4, or 5, s is 1, 2, 3, 4, or 5, and the sum of r+s is less than or equal to 6,
as a herbicide,
with the proviso that the compound of Formula (I) is not
(i) *tert*-butyl N-[2-methyl-6-(3-pyridyl)-3-pyridyl]carbamate.

Compounds of formula (I) may exist as different geometric isomers, or in different tautomeric forms. This invention covers the use of all such isomers and tautomers, and mixtures thereof in all proportions, as well as isotopic forms such as deuterated compounds.

It may be the case that compounds of formula (I) may contain one or more asymmetric centers and may thus give rise to optical isomers and diastereomers. While shown without respect to stereochemistry, the present invention includes the use of all such optical isomers and diastereomers as well as the racemic and resolved, enantiomerically pure R and S stereoisomers and other mixtures of the R and S stereoisomers and agrochemically acceptable salts thereof.

Each alkyl moiety either alone or as part of a larger group (such as alkoxy, alkylthio, alkoxycarbonyl, alkylcarbonyl, alkylaminocarbonyl, ordialkylaminocarbonyl, *et al.*) may be straight-chained or branched. Typically, the alkyl is, for example, methyl, ethyl, n-propyl, isopropyl, n-butyl, sec-butyl, isobutyl, *tert-butyl,* n-pentyl, neopentyl, or n-hexyl. The alkyl groups are generally C₁-C₆ alkyl groups (except where already defined more narrowly), but are preferably C₁-C₄ alkyl or C₁-C₃ alkyl groups, and, more preferably, are C₁-C₂ alkyl groups (such as methyl).

Alkenyl and alkynyl moieties can be in the form of straight or branched chains, and the alkenyl moieties, where appropriate, can be of either the (*E*)- or (Z)-configuration. Alkenyl and alkynyl moieties can contain one or more double and/or triple bonds in any combination; but preferably contain only one double bond (for alkenyl) or only one triple bond (for alkynyl).

The alkenyl or alkynyl moieties are typically C₂-C₄ alkenyl or C₂-C₄ alkynyl, more specifically ethenyl (vinyl), prop-2-enyl, prop-3-enyl (allyl), ethynyl, prop-3-ynyl (propargyl), or prop-1-ynyl. Preferably, the term cycloalkyl refers to cyclopropyl, cyclobutyl, cyclopentyl or cyclohexyl.

In the context of the present specification the term "aryl" preferably means phenyl.

Heteroaryl groups and heteroaryl rings (either alone or as part of a larger group, such as heteroaryl-alkyl-) are ring systems containing at least one heteroatom and can be in mono- or bi-cyclic form. Typically "heteroaryl" is as used in the context of this invention includes furyl, thienyl, pyrrolyl, pyrazolyl, imidazolyl, triazolyl, oxazolyl, isoxazolyl, thiazolyl, isothiazolyl, oxadiazolyl, pyridyl, pyrimidinyl, pyridazinyl, pyrazinyl, and triazinyl rings, which may or may not be substituted as described herein.

The term "heterocyclyl" as used herein, encompasses ring systems containing at least one heteroatom and that are typically in monocyclic form. Preferably, heterocyclyl groups will contain up to two heteroatoms which will preferably be chosen from nitrogen, oxygen and sulfur. Where a heterocycle contains sulfur as a heteroatom it may be in oxidized form i.e. in the form -S(O)ₚ- where p is an integer of 0, 1 or 2 as defined herein. Such heterocyclyl groups are preferably 3- to 8-membered, and more preferably 3- to 6-membered rings. Examples of heterocyclic groups include oxetanyl, thietanyl, and azetidinyl groups. Such heterocyclyl rings may or may not be substituted as described herein.

Halogen (or halo) encompasses fluorine, chlorine, bromine or iodine. The same correspondingly applies to halogen in the context of other definitions, such as haloalkyl or halophenyl.

Haloalkyl groups having a chain length of from 1 to 6 carbon atoms are, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl and 2,2,2-trichloroethyl, heptafluoron-propyl and perfluoro-n-hexyl.

Alkoxy groups preferably have a chain length of from 1 to 6 carbon atoms. Alkoxy is, for example, methoxy, ethoxy, propoxy, isopropoxy, n-butoxy, isobutoxy, sec-butoxy or tert-butoxy or a pentyloxy or hexyloxy isomer, preferably methoxy and ethoxy. It should also be appreciated that two alkoxy substituents may be present on the same carbon atom.

Haloalkoxy is, for example, fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy or 2,2,2-trichloroethoxy, preferably difluoromethoxy, 2-chloroethoxy or trifluoromethoxy.

C₁-C₆ alkyl-S- (alkylthio) is, for example, methylthio, ethylthio, propylthio, isopropylthio, n-butylthio, isobutylthio, sec-butylthio or tert-butylthio, preferably methylthio or ethylthio.

C₁-C₆ alkyl-S(O)- (alkylsulfinyl) is, for example, methylsulfinyl, ethylsulfinyl, propylsulfinyl, isopropylsulfinyl, n-butylsulfinyl, isobutylsulfinyl, sec-butylsulfinyl or tertbutylsulfinyl, preferably methylsulfinyl or ethylsulfinyl.

C₁-C₆ alkyl-S(O)₂- (alkylsulfonyl) is, for example, methylsulfonyl, ethylsulfonyl, propylsulfonyl, isopropylsulfonyl, n-butylsulfonyl, isobutylsulfonyl, sec-butylsulfonyl ortertbutylsulfonyl, preferably methylsulfonyl or ethylsulfonyl.

Compounds of formula (I) may form, and/or be used as, agronomically acceptable salts with amines (for example ammonia, dimethylamine and triethylamine), alkali metal and alkaline earth metal bases or quaternary ammonium bases. Among the alkali metal and alkaline earth metal hydroxides, oxides, alkoxides and hydrogen carbonates and carbonates used in salt formation, emphasis is to be given to the hydroxides, alkoxides, oxides and carbonates of lithium, sodium, potassium, magnesium and calcium, but especially those of sodium, magnesium and calcium. The corresponding trimethylsulfonium salt may also be used.

Compounds of formula (I) may also form (and /or be used as) agronomically acceptable salts with various organic and/or inorganic acids, for example, acetic, propionic, lactic, citric, tartaric, succinic, fumaric, maleic, malonic, mandelic, malic, phthalic, hydrochloric, hydrobromic, phosphoric, nitric, sulfuric, methanesulfonic, naphthalenesulfonic, benzenesulfonic, toluenesulfonic, camphorsulfonic, and similarly known acceptable acids, when the compound of formula (I) contains a basic moiety.

Where appropriate compounds of formula (I) may also be in the form of/used as an N-oxide.

Compounds of formula (I) may also be in the form of/used as hydrates which may be formed during the salt formation.

Preferred values of X¹, X², R¹, R², R³, R⁴, R⁵, R⁶, R⁷, R⁸, R⁹, R¹⁰, R¹¹, R^{a}, R^{b}, n, p, q r and s are as set out below, and a compound of formula (I) according to the invention may comprise any combination of said values. The skilled person will appreciate that values for any specified set of embodiments may combined with values for any other set of embodiments where such combinations are not mutually exclusive.

The skilled man will also appreciate that the values or r and s in the definitions CᵣalkoxyCₛalkyl and CᵣalkoxyCₛhaloalkyl are such that the length of the carbon chain within the substituent does not exceed 6. Preferred values of r are 1, 2, or 3. Preferred values for s are 1, 2, or 3. In various embodiments r is 1, s is 1; or, r is 1, s is 2; or r is 1, s is 3; or r is 2, s is 1; r is 2, s is 2; or r is 2, s is 3; or r is 3, s is 1; or r is 3, s is 2, r is 3, s is 3. Particularly preferred substituents thus include methoxymethyl, and ethoxymethyl.

In one particular embodiment of the present invention, X¹ is N.

In another embodiment of the present invention, X¹ is CR¹ and R¹ is preferably selected from the group consisting of hydrogen, cyano, halogen, C₁-C₃alkyl, C₃-C₄alkynyl, C₁-C₃alkoxy, C₁-C₃haloalkyl, C₁-C₃haloalkoxy, and C₁-C₃thioalkyl. More preferably R¹ is selected from hydrogen, cyano, chloro, fluoro, methyl, propynyl, methoxy, trifluoromethyl, difluoromethoxy and thiomethyl. More preferably still, R¹ is selected from the group consisting of hydrogen, cyano, fluoro, chloro, methoxy-, difluoromethyl and trifluoromethyl. Most preferably R¹ is fluoro.

Preferably R² is selected from the group consisting of halogen, cyano, C₁-C₆alkyl, C₁-C₆haloalkyl, C(O)OC₁-C₆alkyl and phenyl. More preferably R² is chloro, cyano, methyl, trifluoromethyl, methoxy, -C(O)OCH₃ or phenyl.

In one set of embodiments R² is selected from the group consisting of halogen, cyano, nitro, C₁-C₆alkyl, C₁-C₆haloalkyl, C₂-C₆alkenyl, C₂-C₆alkynyl, C₃-C₆cycloalkyl, - C(O)OC₁-C₆alkyl, -S(O)ₚ(C₁-C₆alkyl), C₁-C₆alkoxy, and C₁-C₆haloalkoxy, and is preferably halogen, C₁-C₆alkyl or C₁-C₆haloalkyl, more preferably chloro, methyl or trifluoromethyl.

Where X² is O (i.e. where R³ is -C(O)OR¹²), R¹² is preferably selected from the group consisting of hydrogen, C₁-C₆alkyl, CᵣalkoxyCₛalkyl, C₁-C₆haloalkyl, CᵣalkoxyCₛhaloalkyl, CᵣalkylthioCₛalkyl, C₂-C₆alkenyl, C₂-C₆alkynyl, and -(CR^{a}R^{b})_{q}R¹¹. In such embodiments, R¹² is preferably C₁-C₄alkyl, C₁-C₄ haloalkyl, C₁-C₃alkoxyC₁-C₃alkyl, C₁-C₃alkylthioC₁-C₃alkyl, or CR^{a}R^{b}_{q}R¹¹, wherein q is 0, 1 or 2, R^{a} and R^{b} are each hydrogen, and R¹¹ is cyano, C₃-C₆cycloalkyl, a 5- or 6-membered heterocycle containing 1 or 2 heteroatoms independently selected from O and S wherein said S is in the form S(O)ₚ, or phenyl optionally substituted by 1-3 R⁸.

In one set of embodiments, R³ is -C(O)OC₁-C₆alkyl, and preferably selected from the group consisting of is -C(O)O-ethyl, -C(O)O-*iso*-propyl and -C(O)O-*tert*-butyl.

Where X² is NR¹⁰ (i.e. where R³ is -C(O)NR¹⁰R¹²) it is preferred that R¹⁰ is hydrogen or C₁-C₆alkyl (in particular methyl), or that it forms a 5-7 membered (preferably 5- or 6-membered) ring system optionally containing from 1 to 3 additional heteroatoms independently selected from S, O and N, in conjunction either with R⁴ and the atoms to which R¹⁰ and R⁴ are joined, or in conjunction with R¹² and the nitrogen atom to which R¹⁰ and R¹² are joined. In embodiments where R⁴ and R¹⁰ are joined, the skilled man will appreciate that the ring system may appear as a substituted ring system bearing a substituent on the nitrogen atom of group NR¹⁰, by virtue of substituent R¹². In these embodiments it is preferred that R¹² is hydrogen, or C₁-C₆ alkyl; preferably hydrogen or C₁-C₃ alkyl; and more preferably hydrogen or methyl.

In embodiments where R¹⁰ and R¹² together with the nitrogen atom to which they are joined form a ring system, it is preferred that said ring system is 5- or 6-membered. Where the ring system is 5-membered, it will preferably contain 0 or 1 additional heteroatom independently selected from O, N, or S in the form of S(O)ₚ. More preferably the 1 additional heteroatom will be S in the form of S(O)ₚ. Where the ring system is 6-membered, it will preferably contain 0 or 1 additional heteroatom independently selected from O, N, or S in the form of S(O)ₚ. More preferably the 1 additional heteroatom will be O or N.

Where R¹⁰ does not form a ring with either R⁴ or R¹², and is hydrogen or C₁-C₆alkyl (preferably hydrogen or methyl), it is preferred that R¹² is C₁-C₄alkyl, C₁-C₃alkoxy, - (CH₂)₃SCH₃, C₁-C₃haloalkyl, C₃-C₆alkynyl, or (CR^{a}R^{b})_{q}R¹¹. In such embodiments where R¹² is (CR^{a}R^{b})_{q}R¹¹, it is particularly preferred that q is 0 or 1. It is further preferred that R¹¹ in such embodiments is an optionally substituted ring system selected from the group consisting of C₃-C₆cycloalkyl, isoxazolyl, phenyl, pyridyl, pyrimidinyl, tetrahydropyranyl and morpholinyl, which, when substituted, is substituted by 1-3 independent R₈.

Preferably R⁴ is selected from the group consisting of hydrogen, methyl, ethyl, allyl, but-2-yn-1-yl, C(O)R⁹ where R⁹ is preferably C₁-C₆alkoxy, and -(CH₂)_{q}R⁵ wherein q is 1 and R⁵ is selected from the group consisting of c-propyl, -CO₂methyl, and phenyl optionally substituted by 1-2 groups R⁸, wherein each R⁸ is independently C₁-C₃alkyl or halogen (more preferably in such embodiments R⁸ is methyl or fluoro). In one embodiment where R⁴ is -(CH₂)_{q}R⁵, R⁴ is the group -CH₂-2,4-difluorophenyl. In further embodiments where R⁴ is -(CH₂)_{q}R⁵, R⁴ is -ethyl-cyclopropyl, or ethyl-difluoro-benzyl.

In particularly preferred embodiments R⁴ is selected from the group consisting of hydrogen, methyl and butoxycarbonyl.

In an alternative embodiment of the present invention, R⁴ and R¹⁰ together with the atoms to which they are joined form a 5-7 membered ring system optionally containing from 1 to 3 heteroatoms independently selected from S, O and N, as described *supra.*

In one particular embodiment R⁶ and R⁷ are both hydrogen. In another embodiment R⁶ is hydrogen and R⁷ is C₁-C₆alkyl (e.g., methyl or ethyl). In another embodiment, R⁶ and R⁷ are both C₁-C₆alkyl.

Preferably R⁹ is C₁-C₆alkyl, preferably ethyl, propyl (in particular iso-propyl) or butyl (in particular *tert*-butyl).

Preferably R¹¹ is selected from the group consisting of C₃-C₆cycloalkyl, phenyl optionally substituted by 1-3 R⁸, a 5- or 6-membered unsubstituted heteroaryl or 5- or 6-membered unsubstituted heterocyclyl ring, and a 5- or 6-membered heteroaryl or 5- or 6-membered heterocyclyl ring, each substituted by 1-3 R⁸. When said phenyl, heterocyclyl or heteroaryl ring is substituted, it is preferably substituted by 1 or 2 R⁸.

Preferably each R⁸ is independently selected from halogen, C₁-C₃-alkyl or C₁-C₃haloalkyl. More preferably each R⁸ is independently selected from methyl, ethyl, chloro or fluoro, more preferably still methyl or chloro.

In one set of embodiments R¹¹ is cyclopropyl, cyclobutyl, cyclopentyl, cyclohexyl, phenyl, or phenyl substituted by 1-3 R⁸.

Table 1 below provides 113 specific examples of herbicidal compounds of Formula (I) for use according to the invention.

**Table 1 Specific examples of compounds of Formula (I)^{#} next to an entry denotes that a compound was isolated as a TFA salt.**

| **Entry No** | **n** | **X₁** | **R₂** | **R₃** | **R₄** |
|---|---|---|---|---|---|
| B1 | 1 | C-F | CF₃ | 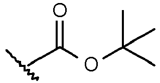 | CH₃ |
| B2 | 0 | C-F | CF₃ | 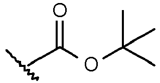 | 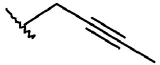 |
| B3 | 0 | C-F | CF₃ | 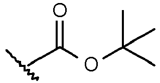 | 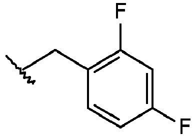 |
| B4 | 0 | C-CN | CF₃ | 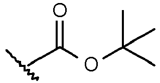 | CH₃ |
| B5 | 0 | C-CF₂H | CF₃ | 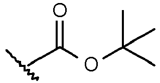 | H |
| B6 | 0 | C-CI | CF₃ | 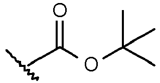 | H |
| B7 | 0 | C-CN | CF₃ | 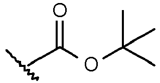 | H |
| B9 | 0 | C-F | CF₃ | 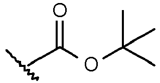 | 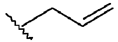 |
| B10 | 0 | C-F | CF₃ | 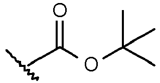 | 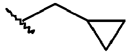 |
| B11 | 0 | C-F | CF₃ | 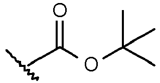 | CH₂CO₂CH₃ |
| B12 | 0 | C-SCH₃ | CH₃ | 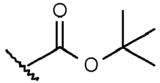 | H |
| B13^{#} | 0 | C-OCF₂H | CH₃ | 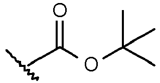 | H |
| B14 | 0 | C-F | CF₃ | 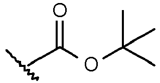 | CH₂CH₃ |
| B15 | 0 | C-F | CF₃ | 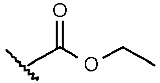 | H |
| B16 | 0 | C-F | CF₃ | 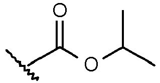 | H |
| B17 | 0 | C-CF₃ | CF₃ | 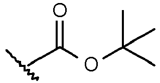 | CH₃ |
| B18 | 0 | C-CH₃ | CF₃ | 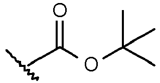 | CH₃ |
| B19 | 0 | C-F | CH₃ | 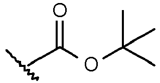 | H |
| B20 | 0 | C-CN | CH₃ | 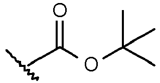 | H |
| B21 | 0 | C-OCH₃ | CF₃ | 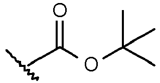 | CH₃ |
| B22 | 0 | C-H | CF₃ | 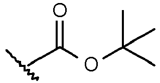 | CH₃ |
| B23 | 0 | C-OCH₃ | CH₃ | 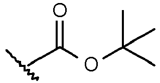 | H |
| B24 | 0 | C-CF₃ | CH₃ | 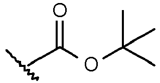 | H |
| B25 | 0 | C-H | CH₃ | 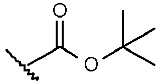 | H |
| B26 | 0 | N | CH₃ | 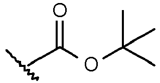 | CH₃ |
| B27 | 0 | N | Cl | 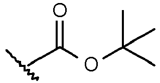 | H |
| B28 | 0 | N | CH₃ | 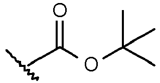 | H |
| B29 | 0 | C-F | CF₃ | 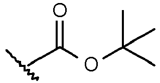 | CH₃ |
| B30 | 0 | C-F | CF₃ | 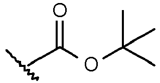 | H |
| B31 | 0 | N | CF₃ | 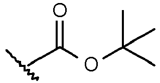 | CH₃ |
| B32 | 0 | N | CF₃ | 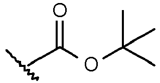 | H |
| B33 | 0 | C-C≡C-CH₃ | CH₃ | 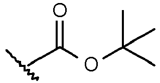 | H |
| B34 | 0 | C-F | CN | 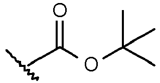 | H |
| B35 | 0 | N | CF₃ | 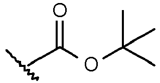 | CH₂CH=CH₂ |
| B36 | 0 | C-OCF₃ | CF₃ | 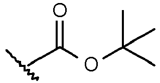 | H |
| B37 | 0 | C-F | CF₃ | 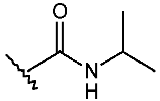 | H |
| B38 | 0 | C-F | CF₃ | 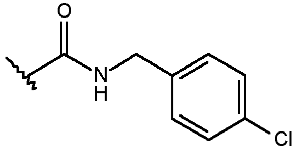 | H |
| B39 | 0 | C-F | CF₃ | 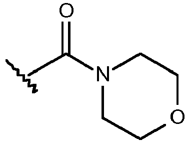 | H |
| B40 | 0 | C-F | CF₃ | 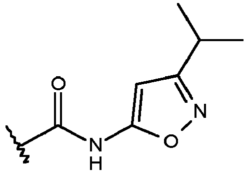 | H |
| B41 | 0 | C-F | CF₃ | 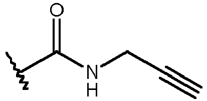 | H |
| B42 | 0 | C-F | CF₃ | 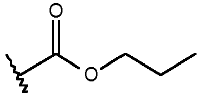 | H |
| B43 | 0 | C-F | CF₃ | 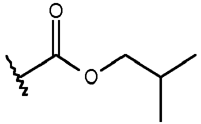 | H |
| B44 | 0 | N | CF₃ | 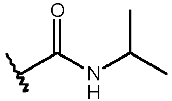 | H |
| B45 | 0 | C-F | CF₃ | 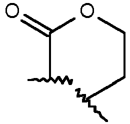 | |
| B46 | 0 | C-F | CF₃ | 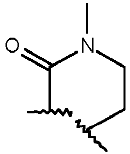 | |
| B47 | 0 | C-F | CF₃ | 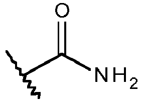 | H |
| B48 | 0 | C-F | CF₃ | 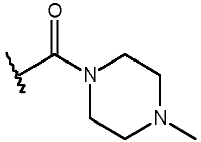 | H |
| B49 | 0 | C-F | CF₃ | 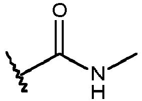 | H |
| B50 | 0 | C-F | CF₃ | 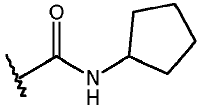 | H |
| B51 | 0 | C-F | CF₃ | 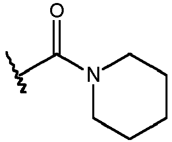 | H |
| B52 | 0 | C-F | CF₃ | 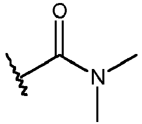 | H |
| B53 | 0 | C-F | CF₃ | 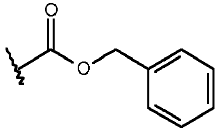 | H |
| B54 | 0 | C-F | CF₃ | 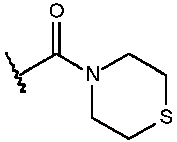 | H |
| B55 | 0 | N | CF₃ | 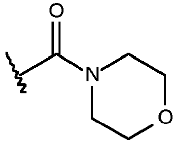 | H |
| B56 | 0 | C-F | CF₃ | 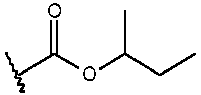 | H |
| B57 | 0 | C-F | CF₃ | 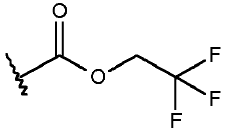 | H |
| B58 | 0 | C-F | CF₃ | 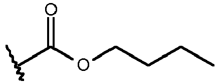 | H |
| B59 | 0 | C-F | CF₃ | 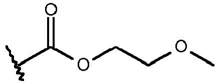 | H |
| B60 | 0 | C-F | CF₃ | 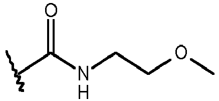 | H |
| B61 | 0 | C-F | CF₃ | 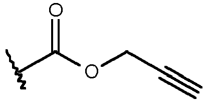 | H |
| B62 | 0 | C-F | CF₃ | 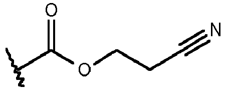 | H |
| B63 | 0 | C-F | CF₃ | 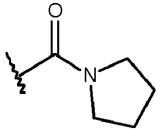 | H |
| B64 | 0 | C-F | CF₃ | 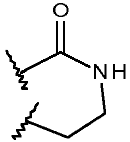 | |
| B65 | 0 | C-F | CF₃ | 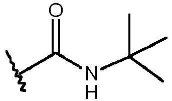 | H |
| B66 | 0 | C-F | CF₃ | 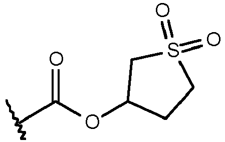 | H |
| B67 | 0 | C-F | CF₃ | 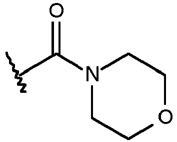 | CH₃ |
| B68 | 0 | C-F | CF₃ | 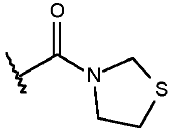 | H |
| B69 | 0 | C-F | CF₃ | 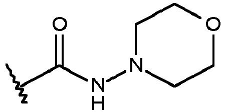 | H |
| B70 | 0 | C-F | CF₃ | 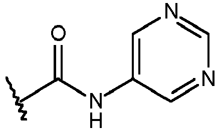 | H |
| B71 | 0 | C-F | CF₃ | 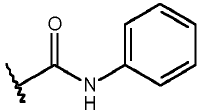 | H |
| B72 | 0 | C-F | CF₃ | 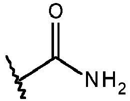 | CH₃ |
| B73 | 0 | C-F | CF₃ | 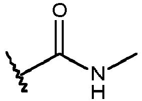 | CH₃ |
| B74 | 0 | C-F | CF₃ | 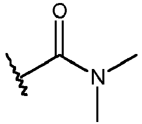 | CH₃ |
| B75 | 0 | C-F | CF₃ | 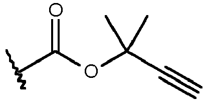 | H |
| B76 | 0 | C-F | CO₂CH₃ | 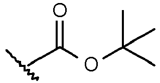 | 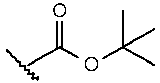 |
| B77 | 0 | C-F | CO₂CH₃ | 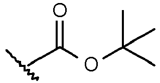 | H |
| B78 | 0 | C-F | OCH₃ | 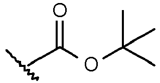 | H |
| B79 | 0 | C-F | CF₃ | 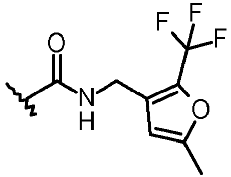 | H |
| B80 | 0 | C-F | CF₃ | 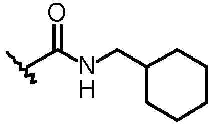 | H |
| B81 | 0 | C-F | CF₃ | 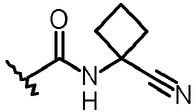 | H |
| B83 | 0 | C-F | CF₃ | 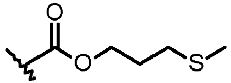 | H |
| B85 | 0 | C-F | CF₃ | 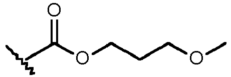 | H |
| B88 | 0 | C-F | CF₃ | 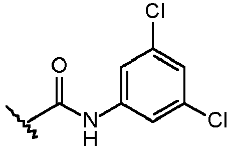 | H |
| B89 | 0 | C-F | CF₃ | 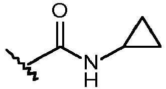 | H |
| B90 | 0 | C-F | CF₃ | 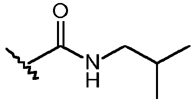 | H |
| B91 | 0 | C-F | CF₃ | 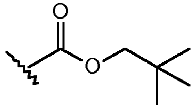 | H |
| B92 | 0 | C-F | CF₃ | 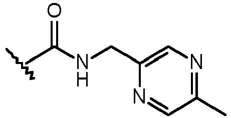 | H |
| B93 | 0 | C-F | CF₃ | 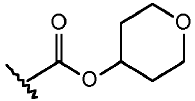 | H |
| B94 | 0 | C-F | CF₃ | 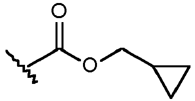 | H |
| B95 | 0 | C-F | CF₃ | 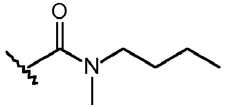 | H |
| B96 | 0 | C-F | CF₃ | 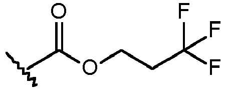 | H |
| B97 | 0 | C-F | CF₃ | 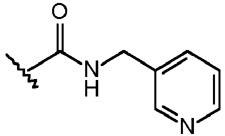 | H |
| B98 | 0 | C-F | CF₃ | 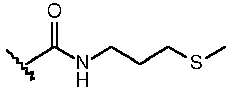 | H |
| B102 | 0 | C-F | CF₃ | 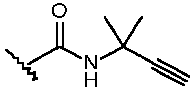 | H |
| B104 | 0 | C-F | CF₃ | 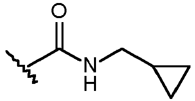 | H |
| B105 | 0 | C-F | CF₃ | 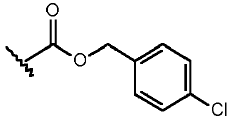 | H |
| B106 | 0 | C-F | CF₃ | 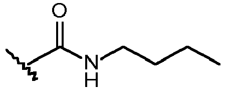 | H |
| B107 | 0 | C-F | CF₃ | 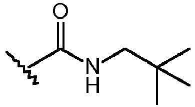 | H |
| B108 | 0 | C-F | CF₃ | 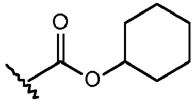 | H |
| B109 | 0 | C-F | OCH₃ | 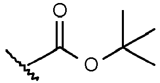 | 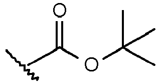 |
| B110 | 0 | C-F | CF₃ | 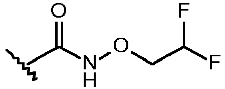 | H |
| B111 | 0 | C-F | CF₃ | 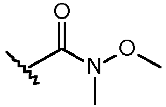 | H |
| B112 | 0 | C-F | Ph | 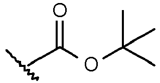 | H |
| B113 | 0 | C-F | CF₃ | 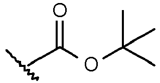 | 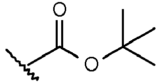 |
| B114 | 0 | C-F | CN | 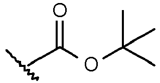 | 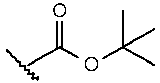 |
| B115 | 0 | C-F | CN | 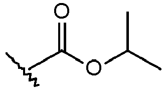 | H |
| B116 | 0 | C-F | CF₃ | 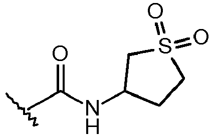 | H |
| B117 | 0 | C-F | CF₃ | 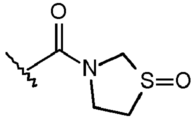 | H |
| B118 | 0 | C-F | CF₃ | 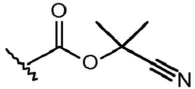 | H |
| B119 | 0 | C-F | CF₃ | 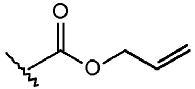 | H |
| B121 | 0 | C-F | CF₃ | 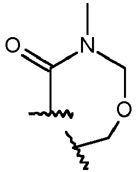 | |
| B123 | 0 | C-F | CF₃ | 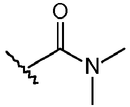 | CH₂CH=CH₂ |
| B125 | 0 | CF | CF₃ | 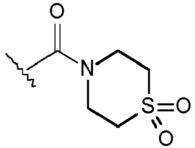 | H |

Compounds of Formula (I) may be prepared according to the following schemes, in which the substituents X¹, X², R¹, R², R³, R⁴, R⁵, R⁶, R⁷, R⁸, R⁹, R¹⁰, R¹¹, R¹², R^{a}, R^{b}, n, p, q, r and s have (unless otherwise stated explicitly) the definitions described hereinbefore, using techniques known to the person skilled in the art of organic chemistry. General methods for the production of compounds of formula (I) are described below. The starting materials used for the preparation of the compounds of the invention may be purchased from the usual commercial suppliers or may be prepared by known methods. The starting materials as well as the intermediates may be purified before use in the next step by state of the art methodologies such as chromatography, crystallization, distillation and filtration.

Typical abbreviations used throughout are as follows:
Ac = acetyl
app = apparent
BINAP = 2,2'-bis(diphenylphosphino)-1,1'-binaphthyl
br. = broad
^{t}Bu = tert-butyl
t-BuOH = *tert*-butanol
d = doublet
dd = double doublet
Dba = dibenzylideneacetone
DCM = dichloromethane
DMF = *N*,*N*-dimethylformamide
DMSO = dimethylsulfoxide
DPPA = diphenylphosphoryl azide
Et₃N = triethylamine
Et₂O = diethyl ether
EtOAc = ethyl acetate
EtOH = ethanol
m = multiplet
mCPBA = *meta*-chloro-perbenzoic acid
Me = methyl
MeOH = methanol
Ms = mesylate
Ph = phenyl
q = quartet
RT or rt = room temperature
s = singlet
t = triplet
Tf = triflate
TFA = trifluoroacetic acid
THF = tetrahydrofuran
TMS = tetramethylsilane
tr = retention time

Processes for preparation of compounds, e.g. a compound of formula (I) (which optionally can be an agrochemically acceptable salt thereof), are now described, and form further aspects of the present invention.

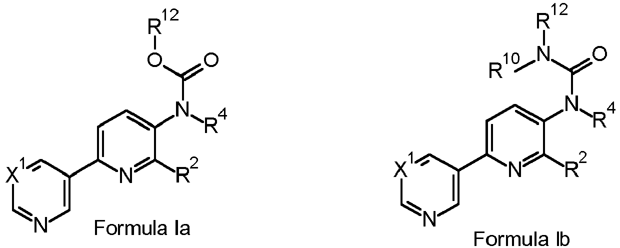

Compounds of Formula la are compounds of Formula I where X² = O, compounds of Formula Ib are compounds of Formula I where X² = NR¹⁰

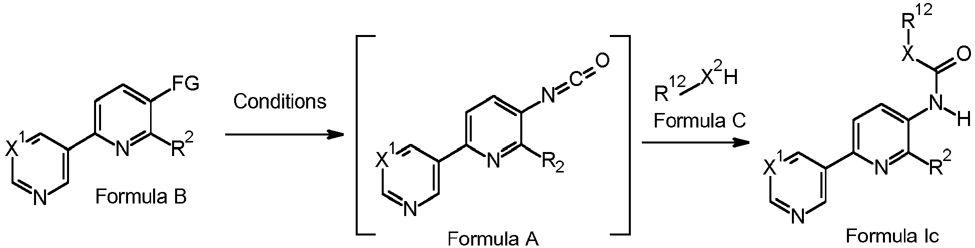

A compound of Formula Ic (a compound of Formula I where R³ is hydrogen) may be prepared from a compound of Formula A by reaction with a compound of Formula C, optionally in the presence of a suitable base and in a suitable solvent. The compound of formula A (isocyanate) may be prepared *in situ* from a suitable compound of Formula B (where FG represents for example a carboxylic acid group) via a Curtius rearrangement with a suitable reagent such as diphenyl phosphoryl azide (see for examples Nissan Chemical Industries Ltd JP2014/208631). Other methods for generating an isocyanate *in situ* are known in the literature. Alternatively the isocyanate can be prepared and isolated before reaction with a compound of Formula C, again methods for such a procedure are known in the literature. Compounds of Formula C are commercially available or can be prepared by methods well known in the literature.

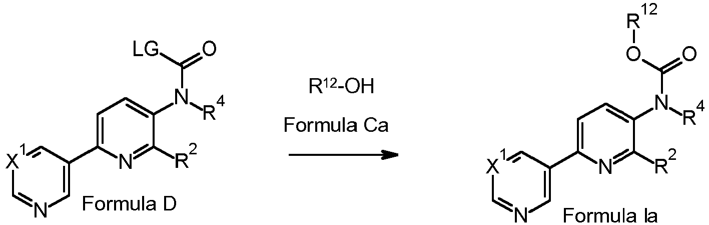

A compound of Formula la may be prepared from a compound of Formula D (where LG is a suitable leaving group, such as Cl (see for example M.C. Fernandez et al Bioorg. Med. Chem. Lett. (2012) 3056) or p-NO₂-phenol (see for example Johnson&Johnson US2006/281768)) by reaction with a compound of Formula Ca (a compound of Formula C where X = O), optionally in the presence of a suitable base in a suitable solvent. Suitable bases include sodium hydride, *N*-ethyl-*N,N*-diisopropylamine, pyridine, 4-dimethylaminopyridine or triethylamine. Suitable solvents may include CH₃CN, THF, DMSO or CH₂Cl₂.

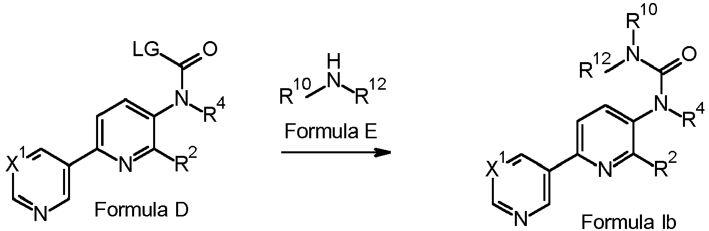

A compound of Formula Ib may be prepared from a compound of Formula D (where LG is a suitable leaving group, such as Cl (see for example Smithkline Beecham Corporation WO2009/058921) or p-NO2-phenol (see for example Johnson&Johnson US2006/281772)) by reaction with a compound of Formula E, optionally in the presence of a suitable base in a suitable solvent. Suitable bases include sodium hydride, N-ethyl-*N,N*-diisopropylamine, pyridine, 4-dimethylaminopyridine or triethylamine. Suitable solvents may include CH₃CN, THF, DMSO or CH₂Cl₂.

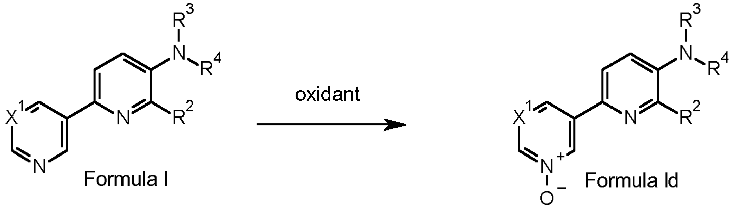

A compound of Formula Id (a compound of Formula I where n = 1) may be prepared from a compound of Formula I (where n = 0) *via* reaction with a suitable oxidant in a suitable solvent. Suitable oxidants may include 3-chloroperbenzoic acid (see for example UCB Pharma WO2012032334). Suitable solvents may include DCM.

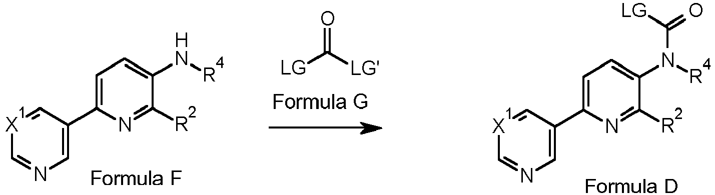

A compound of Formula D may be prepared from a compound of Formula F by reaction with a compound of Formula G (where LG' is a suitable leaving group such as Cl (see for example Smithkline Beecham Corporation WO2009/058921)), optionally in the presence of a suitable base and in a suitable solvent. Suitable bases may include pyridine. Suitable solvents may include CH₂Cl₂.

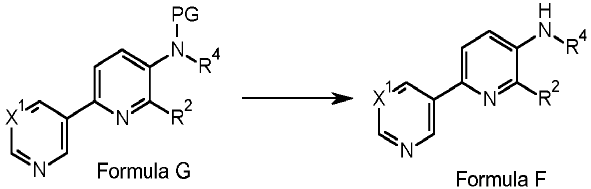

A compound of Formula F may be prepared from a compound of Formula G (where PG is a suitable protecting group such as *tert*-butoxycarbonyl) via a deprotection reaction using a suitable reagent in a suitable solvent. Suitable reagents for removal of a *tert*-butoxycarbonyl group include trifluoroacetic acid (see for example Hoffmann La Roche US2006/183754) or hydrochloric acid (see for example Fujisawa Pharmaceutical Co. Ltd. WO2004/022540). Suitable solvents may include CH₂Cl₂ or EtOAc.

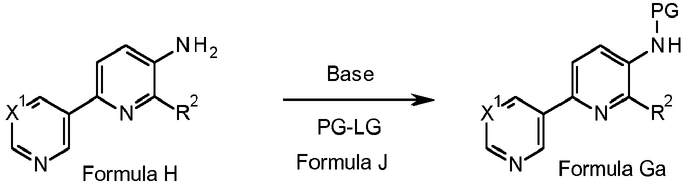

A compound of Formula Ga (a compound of Formula G where R⁴ is H and where PG is a suitable protecting group such as *tert*-butoxycarbonyl) may be prepared from a compound of Formula H via reaction with a compound of Formula J (where LG is a suitable leaving group, such as O^{t}Bu) optionally in the presence of a suitable base and in a suitable solvent. A suitable compound of Formula J may include di-*tert*-butyldicarbonate (see for example Incyte Corporation US2015/175604). Suitable bases may include lithium hexamethyldisilazide. Suitable solvents may include THF. Compounds of Formula J are commercially available or can be prepared by methods well known in the literature.

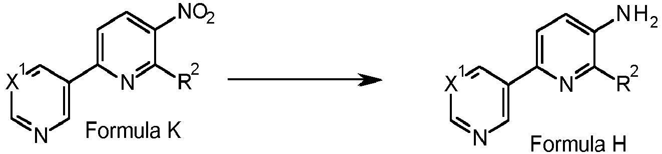

A compound of Formula H may be prepared from a compound of Formula K via a reduction reaction optionally in the presence of a suitable catalyst and/or using a suitable reducing agent in a suitable solvent. Suitable catalysts include palladium on charcoal (see for example Z. Gao et al Bioorg. Med. Chem. Lett. (2013) 6269), Raney nickel (see for example Millenium Pharmaceuticals Ltd WO2010/065134). Suitable reducing agents include hydrogen gas, Fe/HCI (see for example A. Gangee et al J. Med. Chem. (1998) 4533), SnCl₂ (see for example Pharmacia and Upjohn Company WO2004/099201). Suitable solvents include ethanol, methanol, ethyl acetate or water.

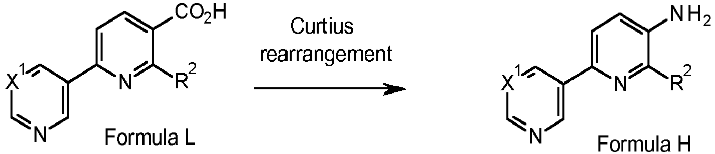

In an alternative approach, a compound of Formula H may be prepared from a compound of Formula L via a Curtius rearrangement using a suitable reagent in a suitable solvent. Suitable reagents include DPPA (see for example Takeda Pharmaceutical Company Ltd WO2008/156757) and suitable solvents include DMF or toluene.

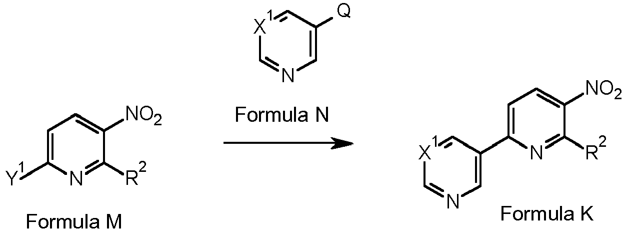

A compound of Formula K may be prepared from a compound of Formula M (where Y¹ is a suitable halogen, such as Cl, Br or I or suitable pseudohalogen, such as OTf) via a cross-coupling reaction with a compound of Formula N (where Q is a suitable coupling group, such as -B(OH)₂ or -B(OR)₂ or -SnR₃) in the presence of a suitable catalyst, optionally in the presence of a suitable base and in a suitable solvent. Suitable catalysts may include Pd(PPh₃)₄ (see for example A.P. Johnson et al, ACS Med. Chem. Lett. (2011) 729) or [1,1'-bis(diphenylphosphino)ferrocene]dichloropalladium(II) (see for example Laboratories Almirall, WO2009/021696). Suitable bases may include K₂CO₃, Na₂CO₃, Cs₂CO₃, K₃PO₄ or CsF. Suitable solvents may include ethylene glycol dimethyl ether, acetonitrile, DMF, ethanol, 1,4-dioxane, tetrahydrofuran and/or water. Compounds of Formula M and of Formula N are commercially available or can be prepared by methods well known in the literature.

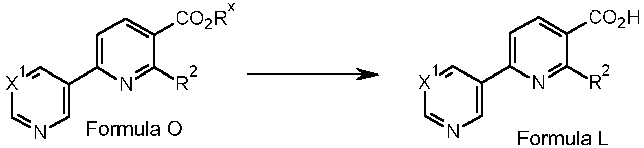

A compound of Formula L may be prepared from a compound of Formula O (where R^{x} is C₁₋₆ alkyl) via a hydrolysis reaction in the presence of a suitable reagent in a suitable solvent. Suitable reagents include NaOH (see for example F. Giordanetto et al Bioorg. Med. Chem. Lett (2014), 2963), LiOH (see for example AstraZeneca AB, WO2006/073361) or KOH (see for example Kowa Co. Ltd EP1627875). Suitable solvents include H₂O, THF, MeOH or EtOH or mixtures thereof.

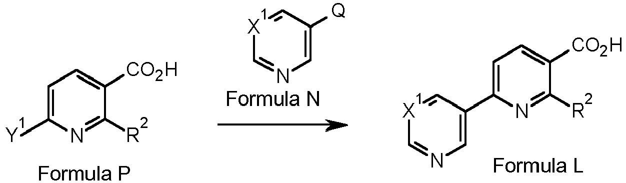

In an alternative approach, a compound of Formula L may be prepared from a compound of Formula P (where Y¹ is a suitable halogen, such as Cl or Br) via a cross-coupling reaction with a compound of Formula N (where Q is a suitable coupling group, such as -B(OH)₂ or -B(OR)₂ or -SnR₃) in the presence of a suitable catalyst, optionally in the presence of a suitable base and in a suitable solvent. Suitable catalysts may include Pd(PPh₃)₄ (see for example Pfizer Limited WO2009/153720) or [1,1'-bis(diphenylphosphino)ferrocene]dichloropalladium(II) (see for example AstraZeneca AB, WO2009/075160). Suitable bases may include K₂CO₃, Na₂CO₃, Cs₂CO₃, K₃PO₄ or CsF. Suitable solvents may include ethylene glycol dimethyl ether, acetonitrile, DMF, ethanol, 1,4-dioxane, tetrahydrofuran and/or water. Compounds of Formula E are commercially available or can be prepared by methods well known in the literature.

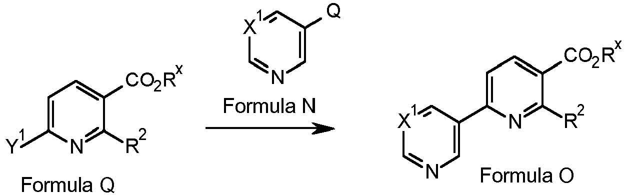

A compound of Formula O may be prepared from a compound of Formula Q (where Y¹ is a suitable halogen, such as Cl or Br) via a cross-coupling reaction with a compound of Formula N (where Q is a suitable coupling group, such as -B(OH)₂ or - B(OR)₂ or -SnR₃) in the presence of a suitable catalyst, optionally in the presence of a suitable base and in a suitable solvent. Suitable catalysts may include Pd(PPh₃)₄ (see for example Pfizer Limited WO2009/153720) or [1,1'-bis(diphenylphosphino)ferrocene]dichloropalladium(II) (see for example Cytokinetics Incorporated WO2008/016643). Suitable bases may include K₂CO₃, Na₂CO₃, Cs₂CO₃, K₃PO₄ or CsF. Suitable solvents may include ethylene glycol dimethyl ether, acetonitrile, DMF, ethanol, 1,4-dioxane, tetrahydrofuran and/or water. Compounds of Formula E are commercially available or can be prepared by methods well known in the literature.

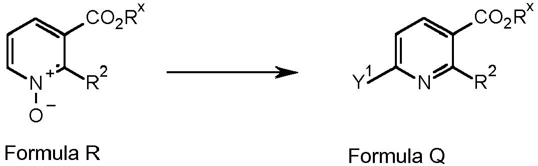

A compound of Formula Q (where Y¹ is a suitable halogen, such as Br or Cl) may be prepared from a compound of Formula R via a halogenation reaction using a suitable reagent, optionally in a suitable solvent. Suitable reagents may include POCl₃ (see for example Takeda Pharmaceutical Co. Ltd. US2011/152273). Suitable solvents may include DCM or DCE.

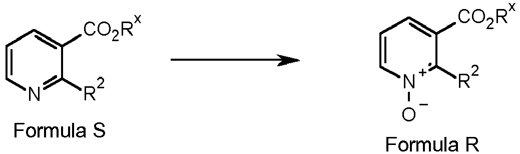

A compound of Formula R may be prepared from a compound of Formula S via an oxidation reaction using a suitable oxidising reagent in a suitable solvent. Suitable oxidants may include 3-chloroperbenzoic acid (see for example Trius Therapeutics Inc. US2012/023875) or urea hydrogen peroxide complex/trifluoroacetic anhydride (see Takeda Pharmaceutical Co. Ltd. US2011/152273). Suitable solvents include DCM or acetonitrile. Compounds of Formula Q are commercially available or can be prepared by methods well known in the literature.

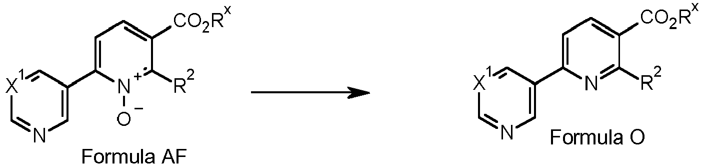

In a yet further alternative approach, a compound of Formula O may be prepared from a compound of Formula AF via a reduction using a suitable reducing agent optionally in a suitable solvent. Suitable reducing agents include indium/ammonium chloride (see for example J.S. Yadav et al Tet. Lett (2000), 2663) or zinc/ammonium chloride. Suitable solvents may include MeOH, THF or water or combinations thereof.

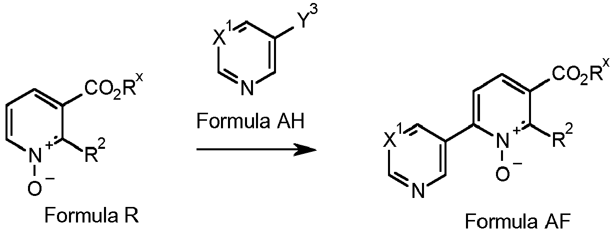

A compound of Formula AF may be prepared from a compound of Formula R *via* a cross-coupling reaction with a compound of Formula AH (where Y³ is a suitable halogen, such as Cl, Br or I or suitable pseudohalogen, such as OTf) in the presence of a suitable catalyst, optionally in the presence of a suitable base and in a suitable solvent. Suitable catalysts include Pd(OAc)₂/tri(tert-butyl)phosphonium tetrafluoroboronate (see for example F. Glorius et al JACS (2013) 12204). A suitable base is K₂CO₃. A suitable solvent is toluene. Compounds of Formula AH are commercially available or can be prepared by methods well known in the literature.

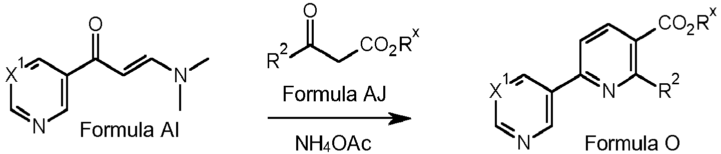

In a yet further alternative approach, compounds of Formula O may be prepared from compounds of Formula Al by reaction with compounds of Formula AJ in the presence of ammonium acetate (see for example F. Hoffmann-La Roche WO2008/034579). Compounds of Formula AJ are commercially available or can be prepared by methods well known in the literature.

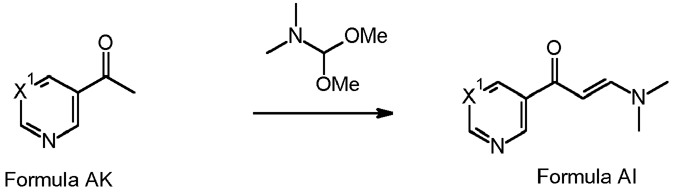

Compounds of Formula Al may be prepared from compounds of Formula AK by reaction with dimethyl formamide dimethylacetal (see for example F. Hoffmann-La Roche WO2008/034579). Compounds of Formula AK are commercially available or can be prepared by methods well known in the literature.

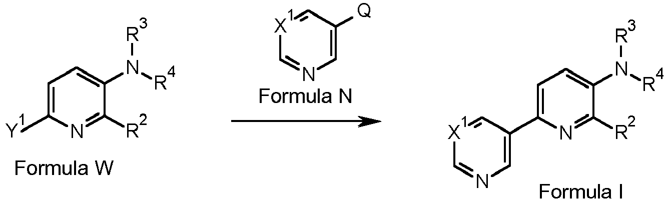

In a further alternative approach, a compound of Formula I may be prepared from a compound of Formula W (where Y¹ is a suitable halogen, such as Cl, Br or I or a suitable pseudohalogen, such as OTf) via a cross-coupling reaction with a compound of Formula N (where Q is a suitable coupling group, such as -B(OH)₂ or -B(OR)₂ or -SnR₃) in the presence of a suitable catalyst, optionally in the presence of a suitable base and in a suitable solvent. Suitable catalysts may include Pd(PPh₃)₄ (see for example Vertex Pharmaceuticals Ltd. WO2011087776 orS.M. Bromidge et al J. Med. Chem. (2000) 1123), Pd₂Cl₂(PPh₃)₂ (see for example Abbott Laboratories US2012245124), [1,1'-bis(diphenylphosphino)ferrocene]dichloropalladium(II) (see for example Dow Agro Sciences US2013005574). Suitable bases may include K₂CO₃ or CsF. Suitable solvents may include ethylene glycol dimethyl ether, acetonitrile, DMF, ethanol, 1,4-dioxane and/or water. Compounds of Formula N are commercially available or can be prepared by methods well known in the literature.

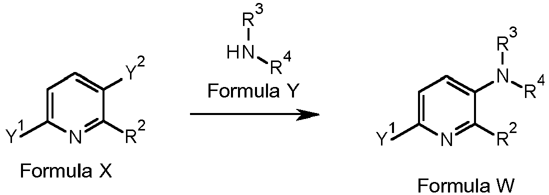

A compound of Formula W may be prepared from a compound of Formula X (where Y² is a suitable halogen, such as Br or I) via reaction with a compound of Formula Y, optionally in the presence of a suitable catalyst and optionally in the presence of a suitable base and in a suitable solvent. Suitable catalyst/ligand systems include Pd₂dba₃/BINAP (see for example Y-Q. Long et al Org. and Biomol. Chem. (2012) 1239). Suitable bases include NaO^{t}Bu. Suitable solvents include toluene or tetrahydrofuran Compounds of Formula Y and of Formula X are commercially available or can be prepared by methods well known in the literature.

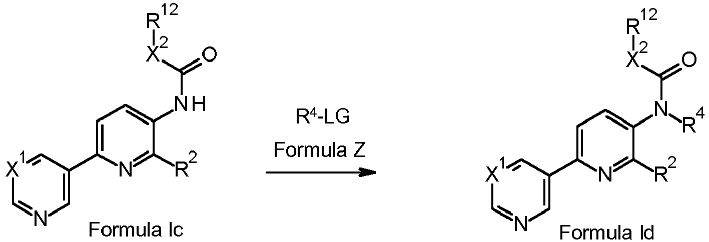

In a further alternative approach a compound of Formula Id (a compound of Formula I where R⁴ is not hydrogen) may be prepared from a compound of Formula Ic (a compound of Formula I where R⁴ is hydrogen) via an alkylation reaction with a compound of Formula Z in the presence of a suitable base and in a suitable solvent. Suitable bases may include sodium hydride (see for example Smithkline Beecham Corporation WO2007/019098) or sodium hexamethyldisilazide (see for example Gilead Sciences Inc. US2010/022508). Suitable solvents may include THF and/or DMF. Compounds of Formula Z are commercially available or may be prepared by methods well known in the literature.

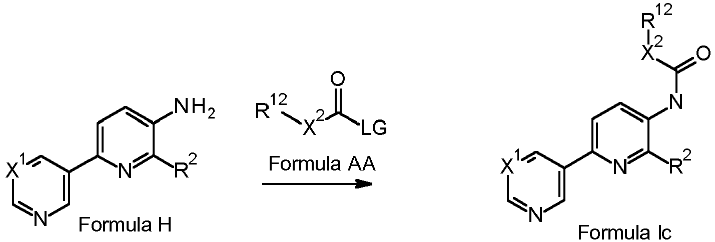

A compound of Formula Ic may be prepared from a compound of Formula H via an acylation reaction with a compound of Formula AA (where LG = a suitable leaving group such as Cl) optionally in the presence of a suitable base and in a suitable solvent. Suitable bases may include NaOH (see for example Array Biopharma Inc. WO2014/078408) or pyridine (see for example Incyte Corporation US2014/200216). Suitable solvents may include acetone, THF, EtOAc and/or water. Compounds of Formula AA are commercially available or may be prepared by methods well known in the literature.

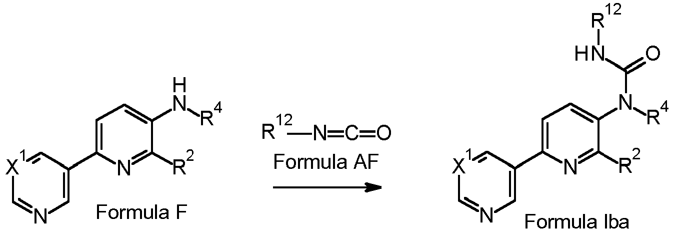

In an alternative approach a compound of Formula Iba (a compound of Formula Ib where R⁹ is hydrogen) may be prepared from a compound of Formula F *via* reaction with a compound of Formula AF optionally in the presence of a suitable base and in a suitable solvent. Suitable bases may include triethylamine or pyridine. Suitable solvents may include dichloromethane, toluene or tetrahydrofuran. Compounds of Formula AF are commercially available or may be prepared by methods well known in the literature.

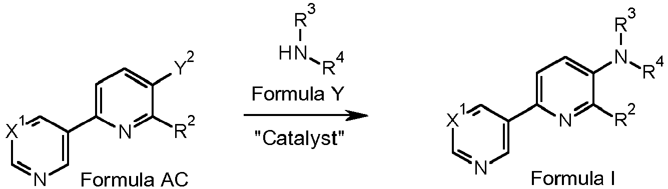

In a yet further alternative approach, a compound of Formula I may be prepared from a compound of Formula AC (where Y² is a suitable halogen, such as Cl, Br or I or a suitable pseudohalogen, such as OTf) *via* cross-coupling with a compound of Formula Y in the presence of a suitable catalyst/ligand, optionally in the presence of a suitable base and in a suitable solvent. Suitable catalyst/ligand combinations may include tris-(dibenzylideneacetone)dipalladium/9,9-dimethyl-4,5-bis(diphenyl-phosphino)xanthene (XantPhos) (see for example F. Hoffmann-La Roche WO2011/154327), Pd(OAc)₂/2-(dicyclohexylphosphino)-2',4',6'-tri-i-propyl-1,1'-biphenyl (see for example D. Zou *et al* Tet. Lett. (2010) 4445) or copper(I) iodide/1,2-diaminocyclohexane (see for example Novartis AG WO2015/059668). Suitable bases include Cs₂CO₃ or K₃PO₄. Suitable solvents include 1,4-dioxane. Compounds of Formula Y and Formula AC are commercially available or may be prepared by methods well known in the literature.

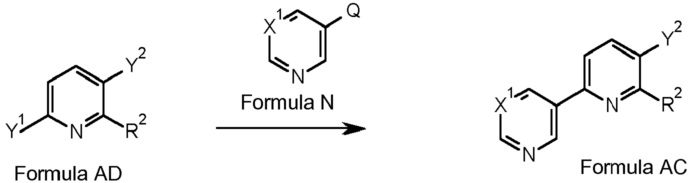

A compound of Formula AC may be prepared from a compound of Formula AD (where Y¹ is a suitable halogen, such as Cl or Br) *via* a cross-coupling reaction with a compound of Formula N (where Q is a suitable coupling group, such as -B(OH)₂ or - B(OR)₂ or -SnR₃) in the presence of a suitable catalyst, optionally in the presence of a suitable base and in a suitable solvent. Suitable catalysts may include Pd(PPh₃)₄ (see for example Vertex Pharmaceuticals Ltd. WO2011087776), Pd₂Cl₂(PPh₃)₂ (see for example Abbott Laboratories US2012245124) or [1,1'-bis(diphenylphosphino)ferrocene]dichloropalladium(II) (see for example Dow Agro Sciences US2013005574). Suitable bases may include K₂CO₃ or CsF. Suitable solvents may include ethylene glycol dimethyl ether, acetonitrile, DMF, ethanol, 1,4-dioxane and/or water. Compounds of Formula AD and of Formula N are commercially available or can be prepared by methods well known in the literature.

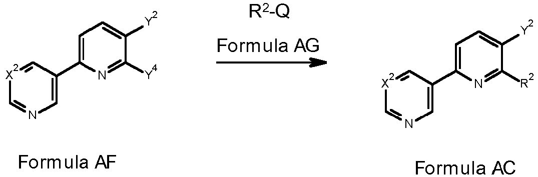

In an alternative approach a compound of Formula AC may be prepared from a compound of Formula AF (where Y⁴ is a suitable halogen, such as Cl) *via* a cross-coupling reaction with a compound of Formula AG (where Q is a suitable coupling group, such as -B(OH)₂ or -B(OR)₂) in the presence of a suitable catalyst (for example XantPhos palladacycle 4^{th} generation), optionally in the presence of a suitable base and in a suitable solvent. Suitable bases may include K₂CO₃. Suitable solvents may include combinations of ethanol, toluene and/or water. Compounds of Formula AG are commercially available or can be prepared by methods well known in the literature.

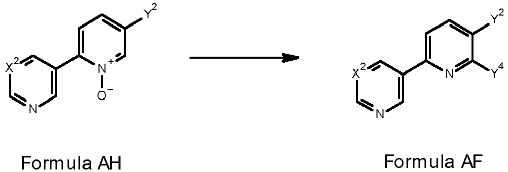

A compound of Formula AF (where Y⁴ is a suitable halogen such as Cl) may be prepared from a compound of Formula AH *via* a halogenation reaction using a suitable reagent, optionally in a suitable solvent. Suitable reagents may include POCl₃.

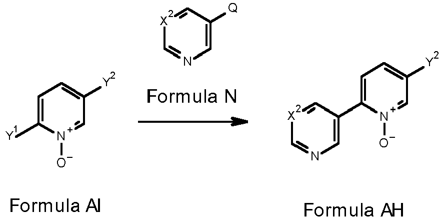

A compound of Formula AH may be prepared from a compound of Formula Al (where Y¹ and Y² are suitable halogens such as Cl) *via* a cross-coupling reaction with a compound of Formula N (where Q is a suitable coupling group, such as -B(OH)₂ or - B(OR)₂) in the presence of a suitable catalyst (for example XantPhos palladacycle 4^{th} generation) optionally in the presence of a suitable base and in a suitable solvent. Suitable bases may include K₂CO₃. Suitable solvents may include combinations of ethanol, toluene and/or water. Compounds of Formula Al and of Formula N are commercially available or may be prepared by methods well known in the literature.

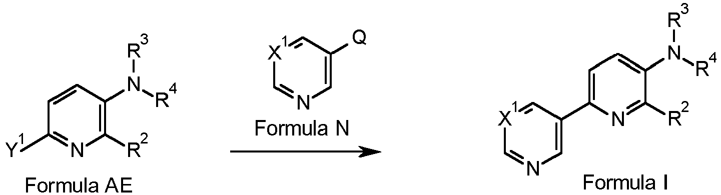

In a further alternative approach, a compound of Formula I may be prepared from a compound of Formula AE (where Y¹ is a suitable halogen, such as Cl, Br or I or a suitable pseudohalogen, such as OTf) via a cross-coupling reaction with a compound of Formula N (where Q is a suitable coupling group, such as -B(OH)₂ or -B(OR)₂ or -SnR₃) in the presence of a suitable catalyst, optionally in the presence of a suitable base and in a suitable solvent. Suitable catalysts may include Pd(PPh₃)₄ (see for example Vertex Pharmaceuticals Ltd. WO2011087776 orS.M. Bromidge et al J. Med. Chem. (2000) 1123), Pd₂Cl₂(PPh₃)₂ (see for example Abbott Laboratories US2012245124), [1,1'-bis(diphenylphosphino)ferrocene]dichloropalladium(II) (see for example Dow Agro Sciences US2013005574). Suitable bases may include K₂CO₃ or CsF. Suitable solvents may include ethylene glycol dimethyl ether, acetonitrile, DMF, ethanol, 1,4-dioxane and/or water. Compounds of Formula N are commercially available or can be prepared by methods well known in the literature.

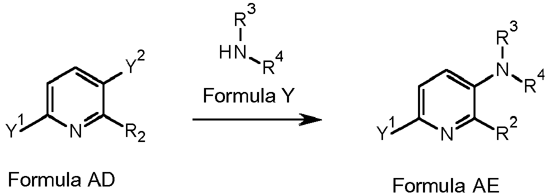

A compound of Formula AE may be prepared from a compound of Formula AD (where Y² is a suitable halogen such as Br or I) via reaction with a compound of Formula Y, optionally in the presence of a suitable catalyst/ligand and optionally in the presence of a suitable base and in a suitable solvent. Suitable catalyst/ligand combinations may include tris-(dibenzylideneacetone)dipalladium/9,9-dimethyl-4,5-bis(diphenylphosphino)xanthene (XantPhos) (see for example F. Hoffmann-La Roche WO2011/154327), Pd(OAc)₂/2-(dicyclohexylphosphino)-2',4',6'-tri-i-propyl-1,1'-biphenyl (see for example D. Zou *et al* Tet. Lett. (2010) 4445) or copper(I) iodide/1,2-diaminocyclohexane (see for example Novartis AG WO2015/059668). Suitable bases include Cs₂CO₃ or K₃PO₄. Suitable solvents include 1,4-dioxane. Compounds of Formula Y and Formula AD are commercially available or may be prepared by methods well known in the literature.

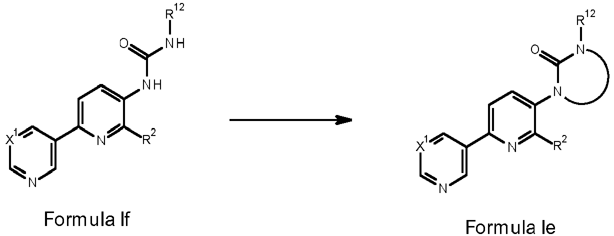

A compound of Formula le (a compound of Formula I where R⁴ and R¹⁰ together with the nitrogen atoms to which they are joined form a 5-, 6-, or 7-membered ring, optionally containing 1 to 3 additional heteroatoms each independently selected from O, N or S) may be prepared from a compound of Formula If (a compound of Formula I where R⁴ and R¹⁰ = H) *via* a cyclisation reaction using a suitable reagent, for example formaldehyde (see for example Nissan Chemical Industries US2012/029187).

The compounds of Formula (I) as described herein may be used as herbicides by themselves, but they are generally formulated into herbicidal compositions using formulation adjuvants, such as carriers, solvents and surface-active agents (SFAs). Thus, the present invention further provides a herbicidal composition comprising a herbicidal compound as described herein and an agriculturally acceptable formulation adjuvant. The composition can be in the form of concentrates which are diluted prior to use, although ready-to-use compositions can also be made. The final dilution is usually made with water, but can be made instead of, or in addition to, water, with, for example, liquid fertilisers, micronutrients, biological organisms, oil or solvents.

Such herbicidal compositions generally comprise from 0.1 to 99 % by weight, especially from 0.1 to 95 % by weight of compounds of Formula (I) and from 1 to 99.9 % by weight of a formulation adjuvant, which preferably includes from 0 to 25 % by weight of a surface-active substance.

The compositions can be chosen from a number of formulation types, many of which are known from the Manual on Development and Use of FAO Specifications for Plant Protection Products, 5th Edition, 1999. These include dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, capsule suspensions (CS) and seed treatment formulations. The formulation type chosen in any instance will depend upon the particular purpose envisaged and the physical, chemical and biological properties of the compound of Formula (I).

Dustable powders (DP) may be prepared by mixing a compound of Formula (I) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of Formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of Formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of Formula (I) and one or more powdered solid diluents or carriers, or from pre-formed blank granules by absorbing a compound of Formula (I) (or a solution thereof, in a suitable agent) in a porous granular material (such as pumice, attapulgite clays, fuller's earth, kieselguhr, diatomaceous earths or ground corn cobs) or by adsorbing a compound of Formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of Formula (I) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of Formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment.

Preparation of an EW involves obtaining a compound of Formula (I) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SFAs, to produce spontaneously a thermodynamically stable isotropic liquid formulation. A compound of Formula (I) is present initially in either the water or the solvent/SFA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of Formula (I). SCs may be prepared by ball or bead milling the solid compound of Formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of Formula (I) may be dry milled and added to water, containing agents hereinbefore described, to produce the desired end product.

Aerosol formulations comprise a compound of Formula (I) and a suitable propellant (for example n-butane). A compound of Formula (I) may also be dissolved or dispersed in a suitable medium (for example water or a water miscible liquid, such as *n-*propanol) to provide compositions for use in non-pressurised, hand-actuated spray pumps.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of Formula (I) and, optionally, a carrier or diluent therefor. The polymeric shell may be produced by either an interfacial polycondensation reaction or by a coacervation procedure. The compositions may provide for controlled release of the compound of Formula (I) and they may be used for seed treatment. A compound of Formula (I) may also be formulated in a biodegradable polymeric matrix to provide a slow, controlled release of the compound.

The composition may include one or more additives to improve the biological performance of the composition, for example by improving wetting, retention or distribution on surfaces; resistance to rain on treated surfaces; or uptake or mobility of a compound of Formula (I). Such additives include surface active agents (SFAs), spray additives based on oils, for example certain mineral oils or natural plant oils (such as soy bean and rape seed oil), and blends of these with other bio-enhancing adjuvants (ingredients which may aid or modify the action of a compound of Formula (I)).

Wetting agents, dispersing agents and emulsifying agents may be SFAs of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di*i*sopropyl- and tri-*i*sopropyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

Herbicidal compositions as described herein may further comprise at least one additional pesticide. For example, the compounds of formula (I) can also be used in combination with other herbicides or plant growth regulators. In a preferred embodiment the additional pesticide is a herbicide and/or herbicide safener. Examples of such mixtures are, in which 'I' represents a compound of Formula (I), I + acetochlor, I + acifluorfen, I + acifluorfen-sodium, I + aclonifen, I + acrolein, I + alachlor, I + alloxydim, I + ametryn, I + amicarbazone, I + amidosulfuron, I + aminopyralid, I + amitrole, I + anilofos, I + asulam, I + atrazine, I + azafenidin, I + azimsulfuron, I + BCPC, I + beflubutamid, I + benazolin, I + bencarbazone, I + benfluralin, I + benfuresate, I + bensulfuron, I + bensulfuron-methyl, I + bensulide, I + bentazone, I + benzfendizone, I + benzobicyclon, I + benzofenap, I + bicyclopyrone, I + bifenox, I + bilanafos, I + bispyribac, I + bispyribac-sodium, I + borax, I + bromacil, I + bromobutide, I + bromoxynil, I + butachlor, I + butamifos, I + butralin, I + butroxydim, I + butylate, I + cacodylic acid, I + calcium chlorate, I + cafenstrole, I + carbetamide, I + carfentrazone, I + carfentrazone-ethyl, I + chlorflurenol, I + chlorflurenolmethyl, I + chloridazon, I + chlorimuron, I + chlorimuron-ethyl, I + chloroacetic acid, I + chlorotoluron, I + chlorpropham, I + chlorsulfuron, I + chlorthal, I + chlorthal-dimethyl, I + cinidon-ethyl, I + cinmethylin, I + cinosulfuron, I + cisanilide, I + clethodim, I + clodinafop, I + clodinafop-propargyl, I + clomazone, I + clomeprop, I + clopyralid, I + cloransulam, I + cloransulam-methyl, I + cyanazine, I + cycloate, I + cyclosulfamuron, I + cycloxydim, I + cyhalofop, I + cyhalofop-butyl,, I + 2,4-D, I + daimuron, I + dalapon, I + dazomet, I + 2,4-DB, I + I + desmedipham, I + dicamba, I + dichlobenil, I + dichlorprop, I + dichlorprop-P, I + diclofop, I + diclofop-methyl, I + diclosulam, I + difenzoquat, I + difenzoquat metilsulfate, I + diflufenican, I + diflufenzopyr, I + dimefuron, I + dimepiperate, I + dimethachlor, I + dimethametryn, I + dimethenamid, I + dimethenamid-P, I + dimethipin, I + dimethylarsinic acid, I + dinitramine, I + dinoterb, I + diphenamid, I + dipropetryn, I + diquat, I + diquat dibromide, I + dithiopyr, I + diuron, I + endothal, I + EPTC, I + esprocarb, I + ethalfluralin, I + ethametsulfuron, I + ethametsulfuron-methyl, I + ethephon, I + ethofumesate, I + ethoxyfen, I + ethoxysulfuron, I + etobenzanid, I + fenoxaprop-P, I + fenoxaprop-P-ethyl, I + fentrazamide, I + ferrous sulfate, I + flamprop-M, I + flazasulfuron, I + florasulam, I + fluazifop, I + fluazifop-butyl, I + fluazifop-P, I + fluazifop-P-butyl, I + fluazolate, I + flucarbazone, I + flucarbazone-sodium, I + flucetosulfuron, I + fluchloralin, I + flufenacet, I + flufenpyr, I + flufenpyr-ethyl, I + flumetralin, I + flumetsulam, I + flumiclorac, I + flumiclorac-pentyl, I + flumioxazin, I + flumipropin, I + fluometuron, I + fluoroglycofen, I + fluoroglycofen-ethyl, I + fluoxaprop, I + flupoxam, I + flupropacil, I + flupropanate, I + flupyrsulfuron, I + flupyrsulfuron-methyl-sodium, I + flurenol, I + fluridone, I + flurochloridone, I + fluroxypyr, I + flurtamone, I + fluthiacet, I + fluthiacet-methyl, I + fomesafen, I + foramsulfuron, I + fosamine, I + glufosinate, I + glufosinate-ammonium, I + glyphosate, I + halauxifen, I + halosulfuron, I + halosulfuron-methyl, I + haloxyfop, I + haloxyfop-P, I + hexazinone, I + imazamethabenz, I + imazamethabenz-methyl, I + imazamox, I + imazapic, I + imazapyr, I + imazaquin, I + imazethapyr, I + imazosulfuron, I + indanofan, I + indaziflam, I + iodomethane, I + iodosulfuron, I + iodosulfuron-methyl-sodium, I + ioxynil, I + isoproturon, I + isouron, I + isoxaben, I + isoxachlortole, I + isoxaflutole, I + isoxapyrifop, I + karbutilate, I + lactofen, I + lenacil, I + linuron, I + mecoprop, I + mecoprop-P, I + mefenacet, I + mefluidide, I + mesosulfuron, I + mesosulfuron-methyl, I + mesotrione, I + metam, I + metamifop, I + metamitron, I + metazachlor, I + methabenzthiazuron, I + methazole, I + methylarsonic acid, I + methyldymron, I + methyl isothiocyanate, I + metolachlor, I + S-metolachlor, I + metosulam, I + metoxuron, I + metribuzin, I + metsulfuron, I + metsulfuron-methyl, I + molinate, I + monolinuron, I + naproanilide, I + napropamide, I + naptalam, I + neburon, I + nicosulfuron, I + n-methyl glyphosate, I + nonanoic acid, I + norflurazon, I + oleic acid (fatty acids), I + orbencarb, I + orthosulfamuron, I + oryzalin, I + oxadiargyl, I + oxadiazon, I + oxasulfuron, I + oxaziclomefone, I + oxyfluorfen, I + paraquat, I + paraquat dichloride, I + pebulate, I + pendimethalin, I + penoxsulam, I + pentachlorophenol, I + pentanochlor, I + pentoxazone, I + pethoxamid, I + phenmedipham, I + picloram, I + picolinafen, I + pinoxaden, I + piperophos, I + pretilachlor, I + primisulfuron, I + primisulfuron-methyl, I + prodiamine, I + profoxydim, I + prohexadione-calcium, I + prometon, I + prometryn, I + propachlor, I + propanil, I + propaquizafop, I + propazine, I + propham, I + propisochlor, I + propoxycarbazone, I + propoxycarbazone-sodium, I + propyzamide, I + prosulfocarb, I + prosulfuron, I + pyraclonil, I + pyraflufen, I + pyraflufen-ethyl, I + pyrasulfotole, I + pyrazolynate, I + pyrazosulfuron, I + pyrazosulfuron-ethyl, I + pyrazoxyfen, I + pyribenzoxim, I + pyributicarb, I + pyridafol, I + pyridate, I + pyriftalid, I + pyriminobac, I + pyriminobac-methyl, I + pyrimisulfan, I + pyrithiobac, I + pyrithiobac-sodium, I + pyroxasulfone, I + pyroxsulam, I + quinclorac, I + quinmerac, I + quinoclamine, I + quizalofop, I + quizalofop-P, I + rimsulfuron, I + saflufenacil, I + sethoxydim, I + siduron, I + simazine, I + simetryn, I + sodium chlorate, I + sulcotrione, I + sulfentrazone, I + sulfometuron, I + sulfometuron-methyl, I + sulfosate, I + sulfosulfuron, I + sulfuric acid, I + tebuthiuron, I + tefuryltrione, I + tembotrione, I + tepraloxydim, I + terbacil, I + terbumeton, I + terbuthylazine, I + terbutryn, I + thenylchlor, I + thiazopyr, I + thifensulfuron, I + thiencarbazone, I + thifensulfuron-methyl, I + thiobencarb, I + topramezone, I + tralkoxydim, I + tri-allate, I + triasulfuron, I + triaziflam, I + tribenuron, I + tribenuron-methyl, I + triclopyr, I + trietazine, I + trifloxysulfuron, I + trifloxysulfuron-sodium, I + trifluralin, I + triflusulfuron, I + triflusulfuron-methyl, I + trihydroxytriazine, I + trinexapac-ethyl, I + tritosulfuron, I + [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetic acid ethyl ester (CAS RN 353292-31-6). The compounds of formula (I) and/or compositions of the present invention may also be combined with herbicidal compounds disclosed in WO06/024820 and/or WO07/096576.

The mixing partners of the compound of Formula (I) may also be in the form of esters or salts, as mentioned e.g. in The Pesticide Manual, Sixteenth Edition, British Crop Protection Council, 2012.

The compound of Formula (I) can also be used in mixtures with other agrochemicals such as fungicides, nematicides or insecticides, examples of which are given in The Pesticide Manual (*supra*)*.*

The mixing ratio of the compound of Formula (I) to the mixing partner is preferably from 1: 100 to 1000:1.

The mixtures can advantageously be used in the above-mentioned formulations (in which case "active ingredient" relates to the respective mixture of compound of Formula I with the mixing partner).

The compounds of Formula (I) as described herein can also be used in combination with one or more safeners. Likewise, mixtures of a compound of Formula (I) as described herein with one or more further herbicides can also be used in combination with one or more safeners. The safeners can be AD 67 (MON 4660), benoxacor, cloquintocet-mexyl, cyprosulfamide (CAS RN 221667-31-8), dichlormid, fenchlorazole-ethyl, fenclorim, fluxofenim, furilazole and the corresponding R isomer, isoxadifen-ethyl, mefenpyr-diethyl, oxabetrinil, N-isopropyl-4-(2-methoxy-benzoylsulfamoyl)-benzamide (CAS RN 221668-34-4). Other possibilities include safener compounds disclosed in, for example, EP0365484 e.g N-(2-methoxybenzoyl)-4-[(methylaminocarbonyl)amino]benzenesulfonamide. Particularly preferred are mixtures of a compound of Formula I with cyprosulfamide, isoxadifen-ethyl, cloquintocet-mexyl and/or N-(2-methoxybenzoyl)-4-[(methyl-aminocarbonyl)amino]benzenesulfonamide.

The safeners of the compound of Formula (I) may also be in the form of esters or salts, as mentioned e.g. in The Pesticide Manual (*supra*)*.* The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048, and the reference to fenchlorazole-ethyl also applies to fenchlorazole, etc.

Preferably the mixing ratio of compound of Formula (I) to safener is from 100:1 to 1:10, especially from 20:1 to 1:1.

The mixtures can advantageously be used in the above-mentioned formulations (in which case "active ingredient" relates to the respective mixture of compound of Formula (I) with the safener).

As described above, compounds of formula (I) and/or compositions comprising such compounds may be used in methods of controlling unwanted plant growth, and in particular in controlling unwanted plant growth in crops of useful plants. Thus, the present invention further provides a method of selectively controlling weeds at a locus comprising crop plants and weeds, wherein the method comprises application to the locus, of a weedcontrolling amount of a compound of formula (I), or a composition as described herein. 'Controlling' means killing, reducing or retarding growth or preventing or reducing germination. Generally the plants to be controlled are unwanted plants (weeds). 'Locus' means the area in which the plants are growing or will grow.

The rates of application of compounds of Formula (I) may vary within wide limits and depend on the nature of the soil, the method of application (pre- or post-emergence; seed dressing; application to the seed furrow; no tillage application etc.), the crop plant, the weed(s) to be controlled, the prevailing climatic conditions, and other factors governed by the method of application, the time of application and the target crop. The compounds of Formula I according to the invention are generally applied at a rate of from 10 to 2000 g/ha, especially from 50 to 1000 g/ha.

The application is generally made by spraying the composition, typically by tractor mounted sprayer for large areas, but other methods such as dusting (for powders), drip or drench can also be used.

Useful plants in which the composition according to the invention can be used include crops such as cereals, for example barley and wheat, cotton, oilseed rape, sunflower, maize, rice, soybeans, sugar beet, sugar cane and turf.

Crop plants can also include trees, such as fruit trees, palm trees, coconut trees or other nuts. Also included are vines such as grapes, fruit bushes, fruit plants and vegetables.

Crops are to be understood as also including those crops which have been rendered tolerant to herbicides or classes of herbicides (e.g. ALS-, GS-, EPSPS-, PPO-, ACCase- and HPPD-inhibitors) by conventional methods of breeding or by genetic engineering. An example of a crop that has been rendered tolerant to imidazolinones, e.g. imazamox, by conventional methods of breeding is Clearfield® summer rape (canola). Examples of crops that have been rendered tolerant to herbicides by genetic engineering methods include e.g. glyphosate- and glufosinate-resistant maize varieties commercially available under the trade names RoundupReady® and LibertyLink®, as well as those where the crop plant has been engineered to over-express homogentisate solanesyltransferase as taught in, for example, WO2010/029311.

Crops are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK® (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO 03/052073 and EP-A-427 529. Examples of transgenic plants comprising one or more genes that code for an insecticidal resistance and express one or more toxins are KnockOut@ (maize), Yield Gard® (maize), NuCOTIN33B® (cotton), Bollgard® (cotton), NewLeaf® (potatoes), NatureGard® and Protexcta®. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crops are also to be understood to include those which are obtained by conventional methods of breeding or genetic engineering and contain so-called output traits (e.g. improved storage stability, higher nutritional value and improved flavour).

Other useful plants include turf grass for example in golf-courses, lawns, parks and roadsides, or grown commercially for sod, and ornamental plants such as flowers or bushes.

The compositions can be used to control unwanted plants (collectively, 'weeds'). The weeds to be controlled include both monocotyledonous (e.g. grassy) species, for example: *Agrostis, Alopecurus, Avena, Brachiaria, Bromus, Cenchrus, Cyperus, Digitaria, Echinochloa, Eleusine, Lolium, Monochoria, Rottboellia, Sagittaria, Scirpus, Setaria and Sorghum;* and dicotyledonous species, for example: *Abutilon, Amaranthus, Ambrosia, Chenopodium, Chrysanthemum, Conyza, Galium, Ipomoea, Kochia, Nasturtium, Polygonum, Sida, Sinapis, Solanum, Stellaria, Veronica, Viola* and *Xanthium.* Weeds can also include plants which may be considered crop plants but which are growing outside a crop area ('escapes'), or which grow from seed left over from a previous planting of a different crop ('volunteers'). Such volunteers or escapes may be tolerant to certain other herbicides.

Preferably the weeds to be controlled and/or growth-inhibited, include monocotyledonous weeds, more preferably grassy monocotyledonous weeds, in particular those from the following genus: *Agrostis, Alopecurus, Apera, Avena, Brachiaria, Bromus, Cenchrus, Cyperus* (a genus of sedges), *Digitaria, Echinochloa, Eleusine, Eriochloa, Fimbristylis* (a genus of sedges), *Juncus* (a genus of rushes), *Leptochloa, Lolium, Monochoria, Ottochloa, Panicum, Pennisetum, Phalaris, Poa, Rottboellia, Sagittaria, Scirpus* (a genus of sedges), *Setaria* and/or *Sorghum,* and/or volunteer corn (volunteer maize) weeds; in particular: *Alopecurus myosuroides* (ALOMY, English name "blackgrass"), *Apera spica-venti, Avena fatua* (AVEFA, English name "wild oats"), *Avena ludoviciana, Avena sterilis, Avena sativa* (English name "oats" (volunteer)), *Brachiaria decumbens, Brachiaria plantaginea, Brachiaria platyphylla* (BRAPP), *Bromus tectorum, Digitaria horizontalis, Digitaria insularis, Digitaria sanguinalis* (DIGSA), *Echinochloa crusgalli* (English name "common barnyard grass", ECHCG), *Echinochloa oryzoides, Echinochloa colona or colonum, Eleusine indica, Eriochloa villosa* (English name "woolly cupgrass"), *Leptochloa chinensis, Leptochloa panicoides, Lolium perenne* (LOLPE, English name "perennial ryegrass"), *Lolium multiflorum* (LOLMU, English name "Italian ryegrass"), *Lolium persicum* (English name "Persian darnel"), *Lolium rigidum, Panicum dichotomiflorum* (PANDI), *Panicum miliaceum* (English name "wild proso millet"), *Phalaris minor, Phalaris paradoxa, Poa annua* (POAAN, English name "annual bluegrass"), *Scirpus maritimus, Scirpus juncoides, Setaria viridis* (SETVI, English name "green foxtail"), *Setaria faberi* (SETFA, English name "giant foxtail"), *Setaria glauca, Setaria lutescens* (English name "yellow foxtail"), *Sorghum bicolor,* and/or *Sorghum halepense* (English name "Johnson grass"), and/or *Sorghum vulgare;* and/or volunteer corn (volunteer maize) weeds.

In one embodiment, grassy monocotyledonous weeds to be controlled comprise weeds from the genus: *Agrostis, Alopecurus, Apera, Avena, Brachiaria, Bromus, Cenchrus, Digitaria, Echinochloa, Eleusine, Eriochloa, Leptochloa, Lolium, Ottochloa, Panicum, Pennisetum, Phalaris, Poa, Rottboellia, Setaria* and/or *Sorghum,* and/or volunteer corn (volunteer maize) weeds; in particular: weeds from the genus *Agrostis, Alopecurus, Apera, Avena, Brachiaria, Bromus, Cenchrus, Digitaria, Echinochloa, Eleusine, Eriochloa, Leptochloa, Lolium, Panicum, Phalaris, Poa, Rottboellia, Setaria,* and/or *Sorghum,* and/or volunteer corn (volunteer maize) weeds.

In a further embodiment, the grassy monocotyledonous weeds are "warm-season" (warm climate) grassy weeds; in which case they preferably comprise (e.g. are): weeds from the genus *Brachiaria, Cenchrus, Digitaria, Echinochloa, Eleusine, Eriochloa, Leptochloa, Ottochloa, Panicum, Pennisetum, Phalaris, Rottboellia, Setaria* and/or *Sorghum,* and/or volunteer corn (volunteer maize) weeds. More preferably, the grassy monocotyledonous weeds, e.g. to be controlled and/or growth-inhibited, are "warm-season" (warm climate) grassy weeds comprising (e.g. being): weeds from the genus *Brachiaria, Cenchrus, Digitaria, Echinochloa, Eleusine, Eriochloa, Panicum, Setaria* and/or *Sorghum,* and/or volunteer corn (volunteer maize) weeds.

In another particular embodiment the grassy monocotyledonous weeds, are "coolseason" (cool climate) grassy weeds; in which case they typically comprise weeds from the genus *Agrostis, Alopecurus, Apera, Avena, Bromus, Lolium* and/or Poa.

Various aspects and embodiments of the present invention will now be illustrated in more detail by way of example. It will be appreciated that modification of detail may be made without departing from the scope of the invention.

### PREPARATION EXAMPLES

Those skilled in the art will appreciate that depending on the nature of the substituents X¹, X², R¹, R², R³, R⁴, R⁵, R⁶, R⁷, R⁸, R⁹, R¹⁰, R¹¹, R^{a}, R^{b}, n, p and q, compounds of Formula I may exist in different interconvertible rotameric forms as described in, for example S.A. Richards and J.C. Hollerton, Essential Practical NMR for Organic Chemistry, John Wiley and sons (2010). For clarity, only the spectroscopic data for the major rotameric form is quoted.

### General Methods

[Pd(IPr*)(cin)Cl] refers to the catalyst below - see Chem. Eur. J. 2012, 18, 4517

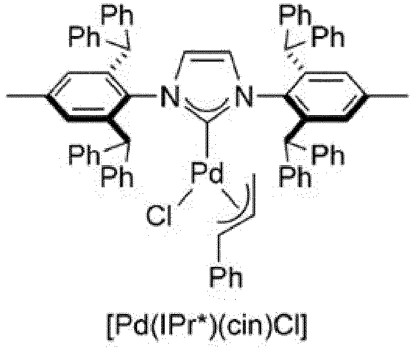

Xantphos palladacycle 4th generation refers to the catalyst below- see Org. Lett. 2014, 16, 4296 and WO13184198.

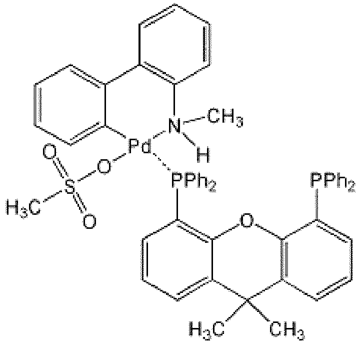

JackiePhos Pd G3 refers to the catalyst below- see J. Am. Chem. Soc., 2009, 131, 16720.

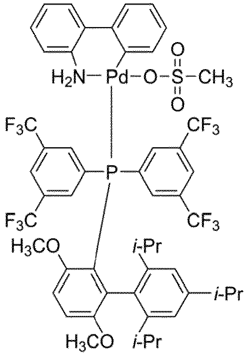

BrettPhos Pd G3 refers to the catalyst below - see *Org. Lett.,* **2014,** 16, 3844.

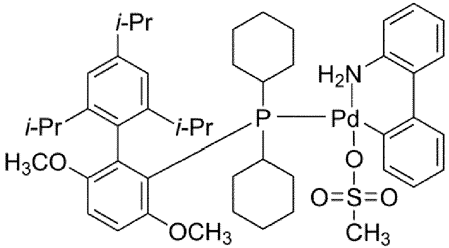

tBuBrettPhos Pd G3 refers to the catalyst below - see *Org. Lett.,* **2013,** *15*, 1394

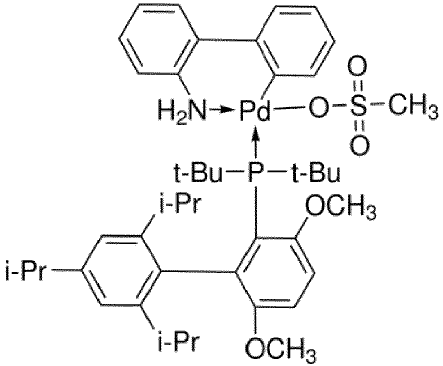

### EXAMPLE P1: Synthesis of tert-butyl N-[6-(5-fluoro-3-pyridyl)-2-(trifluoromethyl)-3-pyridyl]carbamate (Compound B30)

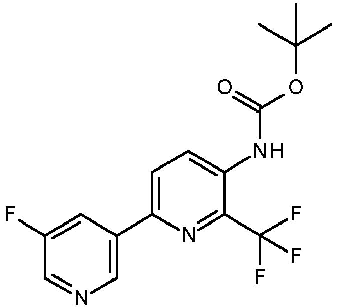

### Step 1: Synthesis of ethyl 1-oxido-2-(trifluoromethyl)pyridin-1-ium-3-carboxylate

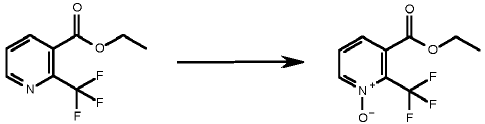

To a stirred suspension of freshly ground urea hydrogen peroxide addition compound (0.099g, 1.05mmol) in DCM (10 mL) at 0°C was added ethyl 2-(trifluoromethyl)pyridine-3-carboxylate (0.1 g, 0.46 mmol) followed by slow addition (ca. 5 minutes) of a solution of trifluoroacetic anhydride (0.13mL, 0.91mmol) in DCM (5 mL). The reaction was allowed to warm to ambient and left stirring overnight. The reaction was washed with 2M aq. sodium carbonate solution (5 mL) and 2M aq sodium metabisulphite solution (2 x 10 mL) and the solvent was removed *in vacuo.* The crude product was purified via flash column chromatography on silica gel using an EtOAc/isohexane gradient as eluent to give the desired product (76mg, 73%) as a thick colourless oil.
¹H NMR (400MHz, CDCl₃) δ 8.28 (1H, d), 7.44 (1H, dd), 7.21 (1H, d), 4.43 (2H, q), 1.44 (3H, t)

### Step 2: Synthesis of ethyl 6-chloro-2-(trifluoromethyl)pyridine-3-carboxylate

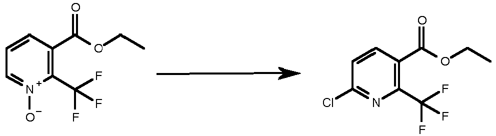

A mixture of ethyl 1-oxido-2-(trifluoromethyl)pyridin-1-ium-3-carboxylate (0.2g, 0.85mmol) and POCl₃ (2mL, 21.24mmol) was heated to 80 °C for 6 hours and then cooled to ambient. The reaction was quenched with 2M aq Na₂CO₃ solution and then extracted with Et₂O (3 x 15 mL). The combined organic extracts were dried over Na₂SO₄ and pre-absorbed onto silica gel for purification *via* flash column chromatography on silica using an EtOAc/isohexane gradient as eluent to give the desired product (0.14g, 61%) as a colourless oil.
¹H NMR (400MHz, CDCl₃) δ 8.09 (d, 1H), 7.60 (d, 1H), 4.43 (q, 2H), 1.43 (t, 3H)

### Step 3: Synthesis of 6-chloro-2-(trifluoromethyl)pyridine-3-carboxylic acid

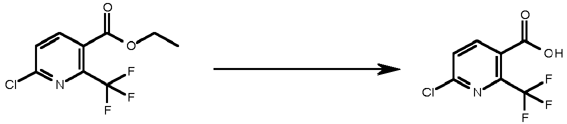

To a solution of ethyl 6-chloro-2-(trifluoromethyl)pyridine-3-carboxylate (190mg, 0.75mmol) in THF (4 mL) and H₂O (2 mL) was added LiOH.H₂O (72mg, 1.72mmol) and the reaction stirred at RT for 3h. The reaction was concentrated under reduced pressure and 2N HCI was added slowly to reach pH 3-4, then extracted with EtOAc (2 x 10 mL). The combined organic extracts were dried over MgSO₄ and concentrated to dryness under reduced pressure to give the desired product (170mg, quant) as a white solid.
¹H NMR (400MHz, CDCl₃) δ 8.12 (1H, d), 7.62 (1H, d)

### Step 4: Synthesis of tert-butyl N-[6-chloro-2-(trifluoromethyl)-3-pyridyl]carbamate

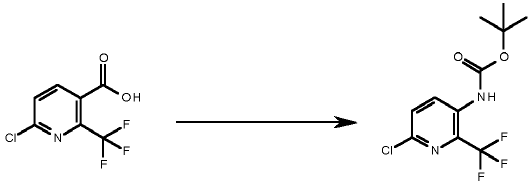

To a stirred solution of 6-chloro-2-(trifluoromethyl)pyridine-3-carboxylic acid (3.0g, 13.3mmol) in *t*-butanol (25 mL) was added triethylamine (2.41 mL, 17.29mmol) and diphenylphosphoryl azide (DPPA) (3.73 mL, 17.29mmol). The reaction was heated at 90°C for 2hrs and then was allowed to cool to RT overnight. The reaction mixture was diluted with EtOAc and washed with water (x2), then brine (x1), dried over MgSO₄ and evaporated to dryness under reduced pressure. The crude product was adsorbed onto silica and purified by flash chromatography on silica using a gradient from 5-50% EtOAc in isohexane as eluent to give the desired product (3.24g, 82%) as a colourless oil.
¹H NMR (400 MHz, CDCl₃) δ 8.64 (d, 1H), 7.48 (d, 1H), 6.89 (br.s, 1H), 1.52 (s, 9H)

### Step 5: Synthesis of tert-butyl N-[6-(5-fluoro-3-pyridyl)-2-(trifluoromethyl)-3-pyridyl]carbamate

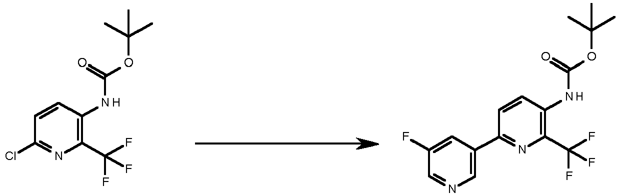

To a stirred suspension of (5-fluoro-3-pyridyl)boronic acid (1.70g, 12mmol), Xantphos palladacycle 4th generation (0.2g, 0.21mmol) and tert-butyl N-[6-chloro-2-(trifluoromethyl)-3-pyridyl]carbamate (2.50g, 8.4mmol) in a mixture of ethanol (6.8 mL) and toluene (25 mL) was added K₂CO₃ (8.4 mL of a 2M solution in water, 17mmol). The reaction mixture was heated at reflux for 3hrs. The reaction mixture was cooled to room temperature and concentrated to dryness. The residue was adsorbed onto silica and purified by flash chromatography on silica using a gradient from 5-100% EtOAc/isohexane as eluent to give the desired compound (2.57g, 85%).
¹H NMR (400MHz, CDCl₃) δ 9.02 (dd, 1H), 8.79 (d, 1H), 8.52 (d, 1H), 8.12 (m, 1H), 7.94 (d, 1H), 7.01 (br.s, 1H), 1.56 (s, 9H)

### EXAMPLE P2: Synthesis of tert-butyl N-[6-pyrimidin-5-yl-2-(trifluoromethyl)-3-pyridyl]carbamate (Compound B32)

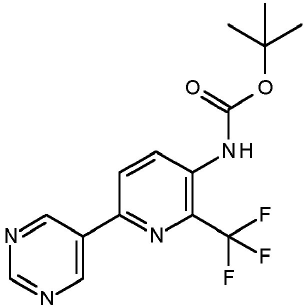

### Step 1: Synthesis of tert-butyl N-[6-pyrimidin-5-yl-2-(trifluoromethyl)-3-pyridyl]carbamate

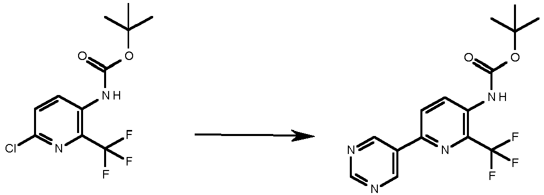

To a stirred suspension of tert-butyl N-[6-chloro-2-(trifluoromethyl)-3-pyridyl]carbamate (2.0g, 6.74mmol), pyrimidin-5-ylboronic acid (1.25g, 10.1mmol) and [Pd(IPr*)(cin)Cl) (0.395g, 0.34mmol) in ethanol (50mL) was added K₂CO₃ (2.07g, 14.8mmol). This mixture was then heated at reflux for 2 hrs. The reaction mixture was adsorbed directly onto silica and purified by flash chromatography on silica using a gradient from 5-100% EtOAc/isohexane as eluent to give the desired product (1.98g, 86%) as a pale yellow solid.
¹H NMR (400MHz, CDCl₃) δ 9.33 (s, 2H), 9.27 (s, 1H), 8.81 (d, 1H), 7.92 (d, 1H), 7.02 (br.s, 1H), 1.54 (s, 9H)

### EXAMPLE P3: Synthesis of tert-butyl N-[6-(5-fluoro-3-pyridyl)-2-(trifluoromethyl)-3-pyridyl]-N-methyl-carbamate (Compound B29)

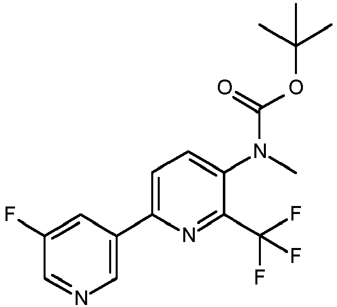

### Step 1: Synthesis of tert-butyl N-[6-(5-fluoro-3-pyridyl)-2-(trifluoromethyl)-3-pyridyl]-N-methyl-carbamate

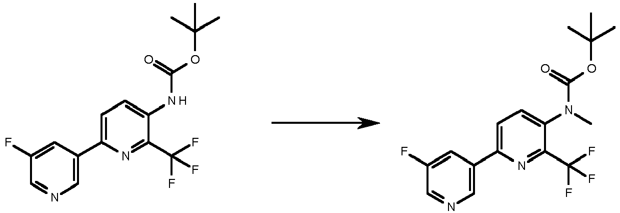

A solution of tert-butyl N-[6-pyrimidin-5-yl-2-(trifluoromethyl)-3-pyridyl]carbamate (422mg, 1.24mmol) in N,N-dimethylformamide (4.2mL) was cooled to 5°C (ice bath), under nitrogen. Sodium hydride (60% dispersion in mineral oil) (1.49mmol, 0.060g) was added in one portion. This mixture was allowed to warm to room temperature and stir for 1 hr, then iodomethane (1.860mmol) was added and the reaction mixture stirred for a further 2hrs. The reaction mixture was diluted carefully with water and extracted with EtOAc (x3). The organics were combined, washed with brine, dried over MgSO₄ and concentrated to give a yellow gum. The crude product was adsorbed directly onto silica and purified by flash chromatography on silica using a gradient from 5-100% EtOAc in isohexane as eluent to give the desired product (354mg, 81%) as a gum.
1H NMR (400MHz, CDCl₃, major rotamer) δ 9.07 (s, 1H), 8.57 (d, 1H), 8.20 (br.d, 1H), 8.01 (d, 1H), 7.76 (d, 1H), 3.22 (s, 3H), 1.33 (s, 9H)

### EXAMPLE P4: Synthesis of ethyl N-[6-(5-fluoro-3-pyridyl)-2-(trifluoromethyl)-3-pyridyl]carbamate (compound B15)

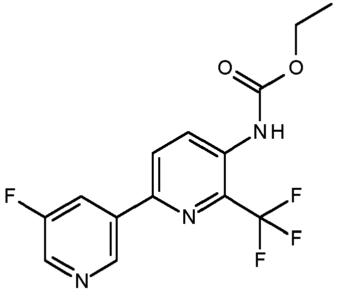

### Step 1: Synthesis of Synthesis of 6-(5-fluoro-3-pyridyl)-2-(trifluoromethyl)pyridin-3-amine

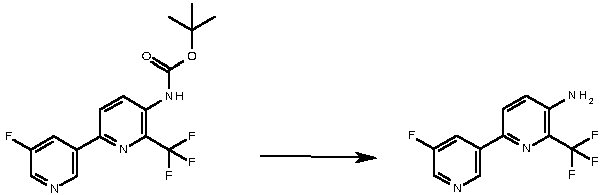

Trifluoroacetic acid (1.4mL, 18mmol) was added to tert-butyl N-[6-(5-fluoro-3-pyridyl)-2-(trifluoromethyl)-3-pyridyl]carbamate (685mg, 1.92mmol) in DCM (7mL) and the reaction mixture was heated at reflux for 3h before being allowed to cool to room temperature. The reaction mixture was partitioned between 2M NaOH (so pH of aqueous was greater than 12) and DCM. The aqueous layer was extracted twice with DCM and the combined organic extracts were dried over MgSO₄ and dry loaded onto celite. Purification by flash chromatography on silica using a gradient of 0-30% EtOAc in isohexane as eluent gave the desired compound (472mg, 96%) as a white solid.
¹H NMR (400MHz, CDCl₃) δ 8.93 (m, 1H), 8.45 (d, 1H), 8.12-8.00 (m, 1H), 7.75 (d 1H), 7.21 (d, 1H), 4.38 (br.s, 2H)

### Step 2: Synthesis of ethyl N-[6-(5-fluoro-3-pyridyl)-2-(trifluoromethyl)-3-pyridyl]carbamate

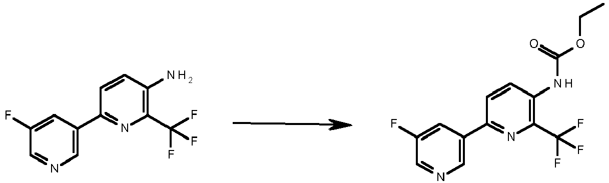

To a stirred solution of 6-(5-fluoro-3-pyridyl)-2-(trifluoromethyl)pyridin-3-amine (100mg, 0.39mmol) and triethylamine (0.065mL, 0.47mmol) in DCM (2mL) at room temperature was added ethyl chloroformate (0.045mL, 0.47 mmol). The reaction mixture was stirred at rt overnight. A further 0.05mL of ethyl chloroformate and 0.07mL of triethylamine was added to the reaction mixture, together with 5mg of DMAP and it was heated to 40 °C for 8 hours and then left to stand at room temperature overnight. A further 0.20mL of ethyl chloroformate was added to the reaction mixture and the reaction mixture heated at 40 °C for 7 hours and then left to stand overnight at room temperature. The reaction mixture was quenched slowly with water, and then extracted three times with DCM. The combined organic layers were washed with brine and then dried over MgSO₄ and dry loaded onto celite. Purification by flash chromatography on silica using a 0-30% EtOAc in isohexane gradient as eluent gave the desired product (48mg, 38%) as a white solid.
¹H NMR (400MHz, CDCl₃) δ 9.03 (d, 1H), 8.79 (d, 1H), 8.53 (d, 1H), 8.12 (m, 1H), 7.97 (d, 1H), 7.14 (br.s, 1H), 4.30 (q, 2H), 1.37 (t, 3H)

### EXAMPLE P5: Synthesis of isopropyl N-[6-(5-fluoro-3-pyridyl)-2-(trifluoromethyl)-3-pyridyl]carbamate (compound B16)

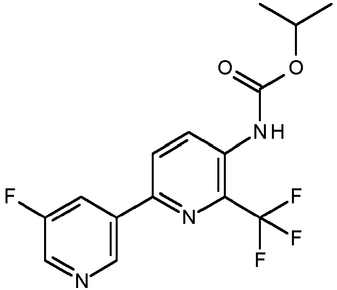

### Step 1: Synthesis of isopropyl N-[6-chloro-2-(trifluoromethyl)-3-pyridyl]carbamate

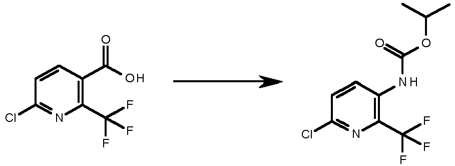

To a solution of 6-chloro-2-(trifluoromethyl)pyridine-3-carboxylic acid (300mg, 1.33mmol) in propan-2-ol (5mL) was added DPPA (0.42g, 1.73mmol) and triethylamine (0.24mL, 1.73mmol). The reaction mixture was heated at 70 °C for 2.5 hours before being allowed to cool to room temperature and stand overnight. The reaction mixture was dry loaded onto celite and purified by column chromatography on silica using a gradient of 0-20% EtOAc in isohexane as eluent to give the desired product (278mg, 74%) as a colourless oil.
¹H NMR (400MHz, CDCl₃) δ 8.66 (d, 1H), 7.50 (d, 1H), 6.98 (br.s, 1H), 5.04 (m, 1H), 1.34 (d, 6H).

### Step 2: Synthesis of isopropyl N-[6-(5-fluoro-3-pyridyl)-2-(trifluoromethyl)-3-pyridyl]carbamate

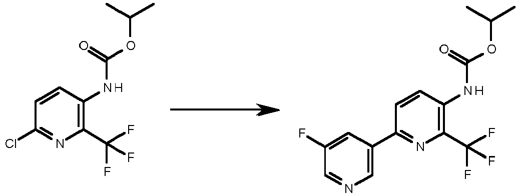

To a suspension of isopropyl N-[6-chloro-2-(trifluoromethyl)-3-pyridyl]carbamate (100mg, 0.35mmol), (5-fluoro-3-pyridyl)boronic acid (75mg, 0.53 mmol) and [Pd(IPr*)(cin)Cl) (20mg, 0.018mmol) in ethanol (3mL) was added potassium carbonate (109mg 0.78mmol). The mixture was then heated to 80 °C for 2 h. The mixture was filtered and then concentrated *in vacuo* onto celite. Purification by flash chromatography on silica using a 20% EtOAc in isohexane gradient as eluent, followed by a second round of purification by column chromatography on silica using a 0-15% EtOAc in isohexane gradient as eluent gave the desired compound (45 mg, 37%) as a white solid.
¹H NMR (400MHz, CDCl₃) δ 9.02 (d, 1H), 8.80 (d, 1H), 8.52 (d, 1H), 8.12 (m, 1H), 7.97 (d, 1H), 7.09 (br.s, 1H), 5.07 (m, 1H), 1.36 (d, 6H)

### EXAMPLE P6: Synthesis of 1-[6-(5-fluoro-3-pyridyl)-2-(trifluoromethyl)-3-pyridyl]-3-isopropyl-urea (compound B37)

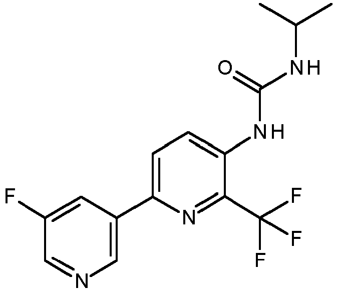

### Step 1: Synthesis of 1-[6-(5-fluoro-3-pyridyl)-2-(trifluoromethyl)-3-pyridyl]-3-isopropyl-urea

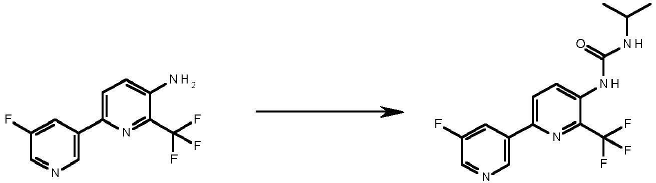

To a stirred solution of 6-(5-fluoro-3-pyridyl)-2-(trifluoromethyl)pyridin-3-amine (200mg, 0.78mmol) in DCM (10mL) was added pyridine (0.252mL, 3.11mmol), DMAP (0.010g, 0.07mmol) and 4-nitrophenyl chloroformate (0.313g, 1.56mmol). The reaction was stirred at room temperature overnight and then isopropylamine (0.334mL, 3.89mmol) was added. The reaction was stirred at room temperature for a further 72h, evaporated to dryness under reduced pressure and purified by flash chromatography on SiO₂ using an EtOAc/isohexane gradient as eluent to give the desired compound (117mg, 44%) as a pale yellow solid.
¹H NMR (400 MHz, CDCl₃) δ 9.01 (m, 1H), 8.81 (d, 1H), 8.50 (d, 1H), 8.13-8.08 (m, 1H), 7.92 (d, 1H), 6.68 (br.s, 1H), 4.69 (br.s, 1H), 4.05-3.94 (m, 1H), 1.25 (m, 6H)

### EXAMPLE P7: Synthesis of tert-butyl N-[2-cyano-6-(5-fluoro-3-pyridyl)-3-pyridyl]carbamate (compound B34)

### Step 1: Synthesis of 3-amino-6-(5-fluoro-3-pyridyl)pyridine-2-carbonitrile

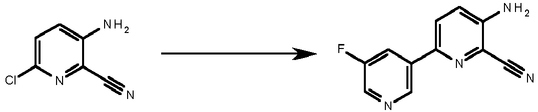

A microwave vial was charged with 3-amino-6-chloro-pyridine-2-carbonitrile (210mg, 1.37mmol), (5-fluoro-3-pyridyl)boronic acid (301 mg, 2.05mmol), potassium carbonate (756mg, 5.47mmol), Pd(PPh₃)₄ (158mg, 0.137mmol) and toluene (5mL). The reaction was heated under microwave irradiation at 150°C for 15 minutes. The reaction mixture was filtered through celite, evaporated to dryness under reduced pressure and purified by flash chromatography on SiO₂ using an EtOAc/isohexane gradient as eluent to give the desired compound (101 mg, 34%) as a pale yellow solid.
¹H NMR (400MHz, CDCl₃) δ 9.31 (s, 1H), 8.83 (s, 1H), 8.58 (s, 1H), 7.77 (d, 1H), 7.23 (d, 1H), 4.47 (s, 2H)

### Step 2: Synthesis of N-[2-cyano-6-(5-fluoro-3-pyridyl)-3-pyridyl]carbamate

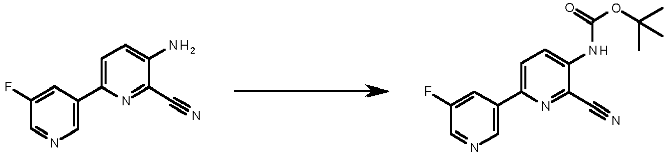

To a stirred solution of 3-amino-6-(5-fluoro-3-pyridyl)pyridine-2-carbonitrile (87mg, F0.41 mmol) in THF (10mL) was added NaHMDS (0.81 mL of 1M solution in THF, 0.81 mmol). The reaction was stirred at room temperature for 30 minutes and then a solution of tert-butoxycarbonyl tert-butyl carbonate (90mg, 0.41 mmol) in THF (2mL) was added in a single portion. The reaction was stirred at room temperature for 3 hours, then H₂O (20mL) was added and the reaction extracted with EtOAc (2 x 20mL). The combined organic extracts were dried over MgSO₄ and evaporated to dryness under reduced pressure. The crude product was purified by flash chromatography over SiO₂ using an EtOAc/isohexane gradient as eluent to give the desired compound (13mg, 10%) as a white solid.
¹H NMR (400MHz, CDCl₃) δ 9.06-8.90 (m, 1H), 8.78 (d, 1H), 8.53 (d, 1H), 8.15-8.00 (m, 1H), 7.95 (d, 1H), 7.18 (br.s, 1H), 1.58 (s, 9H)

### EXAMPLE P8: Synthesis of tert-butyl N-[6-(5-fluoro-1-oxido-pyridin-1-ium-3-yl)-2-(trifluoromethyl)-3-pyridyl]-N-methyl-carbamate (compound B1)

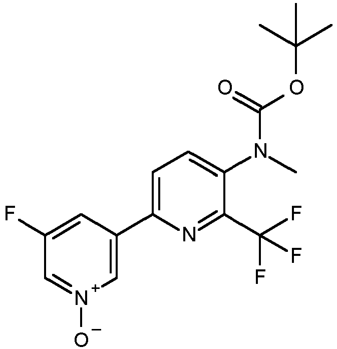

### Step 1: Synthesis of tert-butyl N-[6-(5-fluoro-1-oxido-pyridin-1-ium-3-yl)-2-(trifluoromethyl)-3-pyridyl]-N-methyl-carbamate

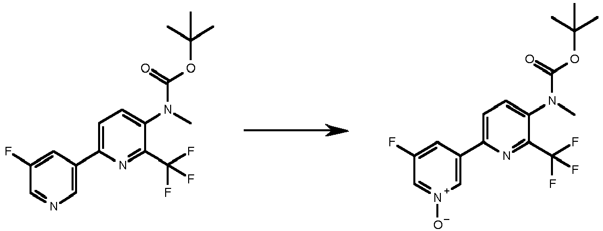

To a stirred solution of tert-butyl N-[6-(5-fluoro-3-pyridyl)-2-(trifluoromethyl)-3-pyridyl]-N-methyl-carbamate (234mg, 0.631 mmol) in CHCl₃ (5mL) was added mCPBA (233mg, 0.95mmol) in a single portion. The reaction was stirred at room temperature for 72h, quenched with saturated aq. NaHCO₃ solution (10mL) and extracted with DCM (2 x 10mL). The combined organic extracts were washed with 10% aq sodium metabisulfite solution (10mL), brine (10mL), dried over MgSO₄ and evaporated to dryness under reduced pressure. The residue was purified by flash chromatography over SiO₂ using a gradient of 0-10% MeOH in DCM as eluent. The crude product was dissolved in DCM (10mL) and washed with saturated aqueous NaHCO₃ solution (3 x 10mL), water (10mL) and brine (10mL). The organic phase was dried over MgSO₄ and evaporated to dryness under reduced pressure to give the desired product (64mg, 26%) as a white solid.
¹H NMR (400MHz, CD₃OD, major rotamer) δ 8.97 (s, 1H), 8.53 (dd, 1H), 8.36 (d, 1H), 8.19 (d, 1H), 8.09 (d, 1H), 3.12 (s, 3H), 1.32 (s, 9H)

### EXAMPLE P9: Synthesis of 3-[6-(5-fluoro-3-pyridyl)-2-(trifluoromethyl)-3-pyridyl]oxazolidin-2-one (compound B45)

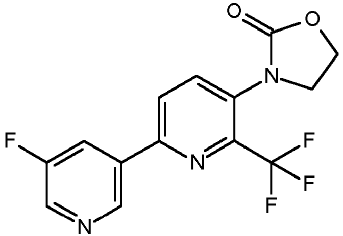

### Step 1: Synthesis of 3-chloro-6-(5-fluoro-3-pyridyl)-2-(trifluoromethyl)pyridine

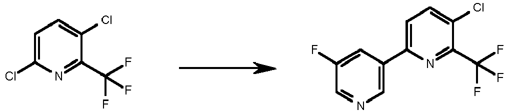

A suspension of 3,6-dichloro-2-(trifluoromethyl)pyridine (2.0g, 9.26 mmol) and (5-fluoro-3-pyridyl) boronic acid (1.44g, 10.19 mmol) in a mixture of EtOH (5.4 mL), toluene (20 mL) and water (9.25 mL) was sparged with N₂ for 30 minutes at RT. K₂CO₃ (2.56g, 18.52 mmol) and Xantphos palladacycle 4th generation (222mg, 0.232 mmol) was added and the reaction heated to 80°C for 2.5 hours. The reaction was allowed to cool to RT, diluted with EtOAc (100 mL) and washed with water (100 mL). The aqueous phase was extracted with further EtOAc (2 x 100 mL). The combined organic extracts were dried over MgSO₄ and evaporated to dryness under reduced pressure. The crude material was purified by flash chromatography on silica gel using an EtOAc/isohexane gradient as eluent to give the desired product (2.16g, 84%) as a pale orange oil which solidified on standing.
¹H NMR (400MHz, CDCl₃) δ 9.03 (s, 1H), 8.58 (s, 1H), 8.15 (d, 1H), 7.98 (d, 1H), 7.92 (d, 1H).

### Step 2: Synthesis of 3-[6-(5-fluoro-3-pyridyl)-2-(trifluoromethyl)-3-pyridyl]oxazolidin-2-one

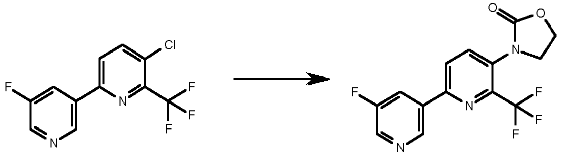

A microwave vial was charged with 3-chloro-6-(5-fluoro-3-pyridyl)-2-(trifluoromethyl)pyridine (100mg, 0.362 mmol), JackiePhos Pd G3 (16.9mg, 0.0145 mmol), Cs₂CO₃ (236mg, 0.723 mmol), oxazolidin-2-one (79mg, 0.904 mmol) and toluene (1 mL), sealed and heated to 150°C for 1 hour under microwave irradiation. The reaction was cooled to RT, diluted with EtOAc (25 mL), filtered through a plug of celite and evaporated to dryness under reduced pressure. The crude material was purified by flash chromatography on silica gel using an EtOAc/isohexane gradient as eluent. The resultant colourless solid was triturated with water, the remaining solid was collected by filtration washed with further water and then dissolved in DCM. The solution was dried over MgSO₄ and evaporated to dryness under reduced pressure to give the desired product (24 mg, 20%) as a colourless solid.
¹H NMR (400MHz, CDCl₃) δ 9.07 (s, 1H), 8.61 (d, 1H), 8.19 (m, 1H), 8.10 (d, 1H), 8.00 (d, 1H), 4.63 (dd, 2H), 4.05 (dd, 2H)

### EXAMPLE P10: Synthesis of methyl 3-[bis(tert-butoxycarbonyl)amino]-6-(5-fluoro-3-pyridyl)pyridine-2-carboxylate (compound B76)

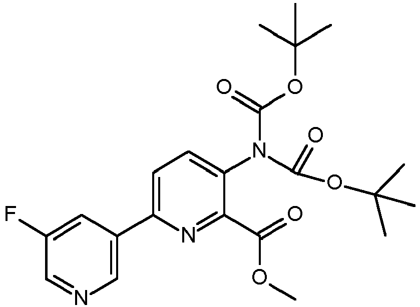

### Step 1: Synthesis of methyl 3-chloro-6-(5-fluoro-3-pyridyl)pyridine-2-carboxylate

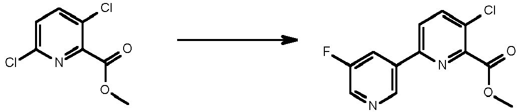

A mixture of methyl 3,6-dichloropyridine-2-carboxylate (1.00g. 4.85 mmol) and (5-fluoro-3-pyridyl)boronic acid (0.752g, 5.34 mmol) in ethanol (2.7 mL), toluene (10.0 mL) and water (4.6 mL) was sparged with N₂ for 30 min at rt. K₂CO₃ (1.342g, 9.71 mmol) and Xantphos palladacycle G4 (0.117 g, 0.121 mmol) were then added and the yellow solution heated to 85°C under an N₂ atmosphere for 2 hours. The reaction was allowed to cool to RT, diluted with EtOAc (50 mL) and washed with water (50 mL). The aqueous phase was further extracted with EtOAc (2 x 50 mL). The combined organics extracts were dried over MgSO₄ and evaporated to dryness under reduced pressure. The crude material was purified by flash chromatography on silica gel using an EtOAc/isohexane gradient as eluent to give the desired product (0.94g, 73%) as a colourless solid.
¹H NMR (400 MHz, CDCl₃) δ 9.00 (s, 1H), 8.54 (d, 1H), 8.14-8.10 (m, 1H), 7.92 (d, 1H), 7.83 (d, 1H), 4.05 (s, 3H).

### Step 2: Synthesis of methyl 3-(benzhydrylideneamino)-6-(5-fluoro-3-pyridyl)pyridine-2-carboxylate

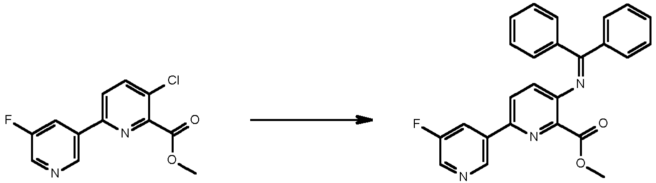

A microwave vial was charged with methyl 3-chloro-6-(5-fluoro-3-pyridyl)pyridine-2-carboxylate (50mg, 0.19 mmol), BrettPhos palladacycle G3 (8.5mg, 0.0094 mmol), BrettPhos (5.1 mg 0.0094 mmol), K₂CO₃ (36mg, 0.26 mmol), benzophenone imine (41 mg, 0.23 mmol) and anhydrous tBuOH (1 mL) and heated for 1 hour at 160°C under microwave irradiation. The reaction was cooled to RT, diluted with DCM (20 mL) and washed with water (20 mL). The aqueous layer was extracted with further portions of DCM (2 x 20 mL) and the combined organic extracts were then dried over MgSO₄ and evaporated to dryness under reduced pressure. The crude product was purified by flash chromatography on silica gel using an EtOAc/isohexane gradient as eluent to give the desired product (29,g, 38%) as a yellow gum.
¹H NMR (400MHz, CDCl₃) δ 8.93 (s, 1H), 8.47 (d, 1H), 8.12-8.06 (m, 1H), 7.78 (br. s, 2H), 7.69 (d, 1H), 7.36 (br m, 8H), 7.11 (d, 1H), 3.92 (s, 3H).

### Step 3: Synthesis of methyl 3-amino-6-(5-fluoro-3-pyridyl)pyridine-2-carboxylate

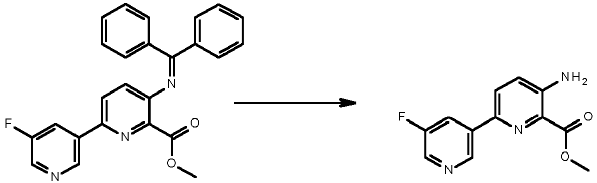

To a stirred solution of methyl 3-(benzhydrylideneamino)-6-(5-fluoro-3-pyridyl)pyridine-2-carboxylate (121 mg, 0.294 mmol) in MeOH (3 mL) were added sodium acetate trihydrate (96mg, 0.706 mmol) and hydroxylamine hydrochloride (37mg, 0.529 mmol) and the reaction stirred at RT for 2 hours. Further sodium acetate trihydrate (40 mg) and hydroxylamine hydrochloride (15 mg) were added and the reaction stirred at RT for 16 hours. The reaction was diluted in DCM (20 mL) and washed with aq. NaOH (0.1 M, 20 mL). The aqueous layer was extracted with further DCM (2 x 20 mL) and the combined organics were then dried over MgSO₄ and evaporated to dryness under reduced pressure. The crude product was purified by flash chromatography on silica gel using an EtOAc/isohexane gradient as eluent to give the desired product (57mg, 78%) as an offwhite solid.
¹H NMR (400 MHz, 2:1 d4-MeOH:d6-DMSO) δ 9.08 (s, 1H), 8.51 (d, 1H), 8.26-8.23 (m, 1H), 8.00 (d, 1H), 7.41 (d, 1H), 4.30 (s, 3H).

### Step 4: Synthesis of methyl 3-[bis(tert-butoxycarbonyl)amino]-6-(5-fluoro-3-pyridyl)pyridine-2-carboxylate (B76)

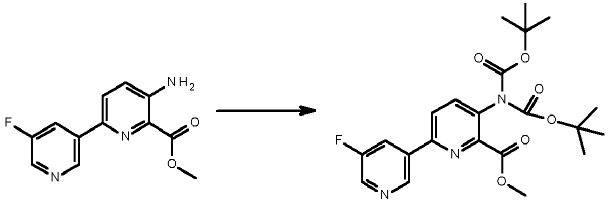

To a stirred suspension of methyl 3-amino-6-(5-fluoro-3-pyridyl)pyridine-2-carboxylate (30mg, 0.12 mmol), DMAP (1.5 mg) and pyridine (0.04 mL, 0.49 mmol) in dichloromethane (1 mL) was added di-tert-butyl dicarbonate (53mg, 0.24 mmol). The reaction was stirred at RT for 2 hours and then further DMAP (14 mg) and 1 mL acetonitrile were added. The reaction was stirred at RT for a further 3 hours and then additional tert-butoxycarbonyl tert-butyl carbonate (53 mg) was added. The reaction was stirred at RT for 17 hours and then diluted in DCM (20 mL) and washed with water (20 mL). The aqueous phase was extracted with further DCM (2 x 20 mL) and then the combined organic extracts were dried over MgSO₄ and evaporated to dryness under reduced pressure. The crude product was purified by flash chromatography on silica gel using an EtOAc/isohexane gradient as eluent to give the desired product (28mg, 52%) as a colourless solid.
¹H NMR (400 MHz, CDCl₃) δ 9.04 (br. s, 1H), 8.54 (br s, 1H), 8.22-8.17 (m, 1H), 7.93 (d, 1H), 7.72 (d, 1H), 3.98 (s, 3H), 1.41 (s, 18H).

### EXAMPLE P11: Synthesis of tert-butyl N-[6-(5-fluoro-3-pyridyl)-2-phenyl-3-pyridyl]carbamate (compound B112)

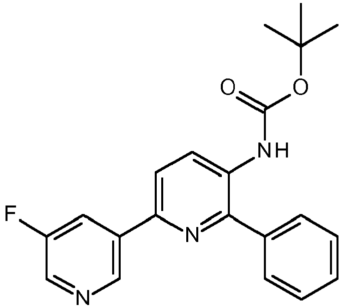

### Step 1: Synthesis of 5-chloro-2-(5-fluoro-3-pyridyl)-1-oxido-pyridin-1-ium

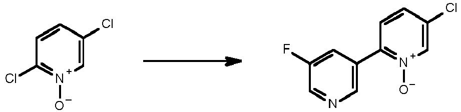

A mixture of 2,5-dichloro-1-oxido-pyridin-1-ium (0.25g, 1.52 mmol) and (5-fluoro-3-pyridyl)boronic acid (0.258g, 1.83 mmol) in EtOH (0.675 mL), toluene (2.5 mL) and water (1.15 mL) was sparged with N₂ for 30 min at rt. K₂CO₃ (0.421 g, 3.05 mmol) and Xantphos palladacycle G4 (37mg, 0.0381 mmol) were then added and the yellow solution heated to 85°C under an N₂ atmosphere for 22 hours. The reaction was allowed to cool to RT and diluted in EtOAc (150 mL) and washed with water (100 mL). The aqueous phase was further extracted with EtOAc (2 x 100 mL). The combined organic extracts were dried over MgSO₄ and evaporated to dryness under reduced pressure. The crude product was purified by flash chromatography on silica gel using a gradient of EtOAc/isohexane as eluent to give the desired product (0.21 g, 61%) as a colourless solid.
¹H NMR (400 MHz, CDCl₃) δ 8.68 (s, 1H), 8.58 (d, 1H), 8.41 (d, 1H), 8.22-8.17 (m, 1H), 7.46 (d, 1H), 7.38 (dd, 1H).

### Step 2: Synthesis of 2,3-dichloro-6-(5-fluoro-3-pyridyl)pyridine

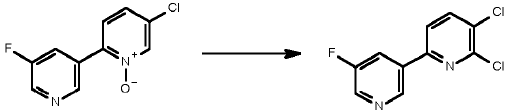

A mixture of 5-chloro-2-(5-fluoro-3-pyridyl)-1-oxido-pyridin-1-ium (0.205g, 0.913 mmol) and POCl₃ (2 mL) was heated at reflux for 90 minutes. The mixture was then cooled and quenched by dropwise addition into cooled sat. aq. NaHCO₃ (250 mL). Once gas evolution had ceased the solution was extracted with portions of EtOAc (3 x 100 mL). The combined organic extracts were dried over MgSO₄ and evaporated to dryness under reduced pressure. The crude product was purified by flash chromatography on silica gel using an EtOAc/isohexane gradient as eluent to give the desired product (112mg, 51%) as a colourless solid.
¹H NMR (400 MHz, CDCl₃) δ 8.99 (s, 1H), 8.56 (d, 1H), 8.13-8.08 (m, 1H), 7.90 (d, 1H), 7.69 (d, 1H).

### Step 3: Synthesis of 3-chloro-6-(5-fluoro-3-pyridyl)-2-phenyl-pyridine

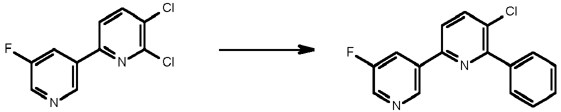

A mixture of 2,3-dichloro-6-(5-fluoro-3-pyridyl)pyridine (0.17g, 0.70 mmol) and phenylboronic acid (0.094g) in EtOH (0.46 mL), toluene (1.70 mL) and water (0.78 mL) was sparged with N₂ for 30 min at RT. K₂CO₃ (0.193g, 1.40 mmol) and Xantphos palladacycle G4 (17mg, 0.0175 mmol) were then added and the yellow solution heated to 85°C under an N₂ atmosphere for 2 hours. The reaction was cooled to RT and then diluted in EtOAc (30 mL) and washed with water (30 mL). The aqueous phase was further extracted with EtOAc (2 x 30 mL). The combined organic extracts were dried over MgSO₄ and evaporated to dryness under reduced pressure. The crude product was purified by flash chromatography on silica gel using an EtOAc/isohexane gradient as eluent to give the desired product (0.185g, 93%) as a pale yellow solid.
¹H NMR (400 MHz, CDCl₃) δ 9.05 (s, 1H), 8.53 (d, 1H), 8.19-8.15 (m, 1H), 7.92 (d, 1H), 7.86-7.82 (m, 2H), 7.71 (d, 1H), 7.53-7.47 (m, 3H).

### Step 4: Synthesis of of tert-butyl N-[6-(5-fluoro-3-pyridyl)-2-phenyl-3-pyridyl]carbamate (compound B112)

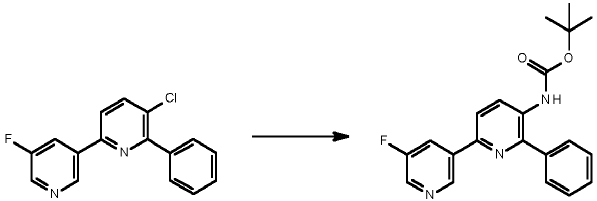

A microwave vial was charged with a mixture of 3-chloro-6-(5-fluoro-3-pyridyl)-2-phenylpyridine (150mg, 0.53 mmol) tBuBrettPhos Pd G3 (18mg, 0.021 mmol), sodium cyanate (72mg, 1.05 mmol) and anhydrous ^{t}BuOH (2 mL) and heated for 1 hour at 140°C under microwave irradiation. The reaction was cooled to RT, diluted with DCM (10 mL) and filtered through a plug of celite which was then washed with further portions of DCM (2 x 7.5 mL). The combined eluant was evaporated to dryness under reduced pressure and purified by flash chromatography on silica gel using an EtOAc/isohexane gradient as eluent to give the desired product (121 mg, 63%) as a colourless solid.
¹H NMR (400 MHz, CDCl₃) δ 9.02 (s, 1H), 8.64 (br. d, 1H), 8.46 (s, 1H), 8.14-8.08 (m, 1H), 7.75 (d, 1H), 7.68-7.63 (m, 2H), 7.61-7.55 (m, 2H), 7.54-7.49 (m, 1H), 6.81 (s, 1H), 1.50 (s, 9H).

### EXAMPLE P12: Synthesis of N-[6-(5-fluoro-3-pyridyl)-2-(trifluoromethyl)-3-pyridyl]thiazolidine-3-carboxamide (compound B68)

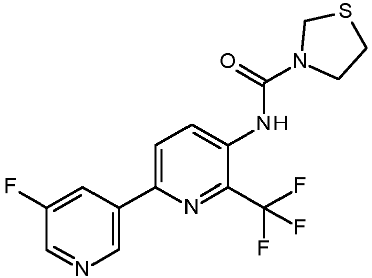

### Step 1: Synthesis of N-[6-(5-fluoro-3-pyridyl)-2-(trifluoromethyl)-3-pyridyl]thiazolidine-3-carboxamide (compound B68)

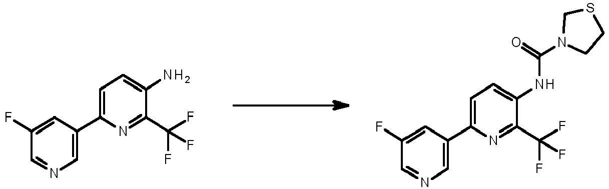

To a stirred solution of 6-(5-fluoro-3-pyridyl)-2-(trifluoromethyl)pyridin-3-amine (250 mg, 0.97 mmol) in 1,4-dioxane (6.25 mL) was added diphosgene (115mg, 0.58 mmol). The reaction mixture stirred at room temperature for 1.5hrs and thiazolidine (0.867g, 9.7204 mmol) was then added dropwise and the reaction mixture stirred at room temperature for 72 hours. The reaction mixture was evaporated to dryness and the crude material purified by mass-directed reverse phase HPLC to give the desired product (168mg, 46%) as a white solid.
¹H NMR (400 MHz, CD₃OD) δ 9.12 (s, 1H), 8.54 (d, 1H), 8.38-8.30 (m, 1H), 8.28 (d, 1H), 8.19 (d, 1H), 4.59 (s, 2H), 3.81 (t, 2H), 3.14 (t, 2H).

### EXAMPLE P13: Synthesis of N-[6-(5-fluoro-3-pyridyl)-2-(trifluoromethyl)-3-pyridyl]-1,1-dioxo-1,3-thiazolidine-3-carboxamide (compound B116) and N-[6-(5-fluoro-3-pyridyl)-2-(trifluoromethyl)-3-pyridyl]-1-oxo-1,3-thiazolidine-3-carboxamide (compound B117)

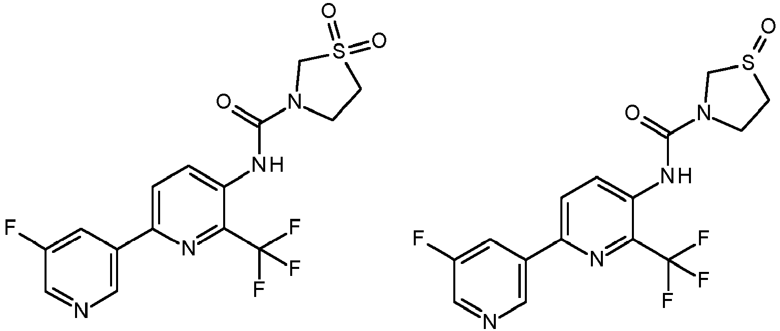

### Step 1: Synthesis of N-[6-(5-fluoro-3-pyridyl)-2-(trifluoromethyl)-3-pyridyl]-1,1-dioxo-1,3-thiazolidine-3-carboxamide (compound B116) and N-[6-(5-fluoro-3-pyridyl)-2-(trifluoromethyl)-3-pyridyl]-1-oxo-1,3-thiazolidine-3-carboxamide (compound B117)

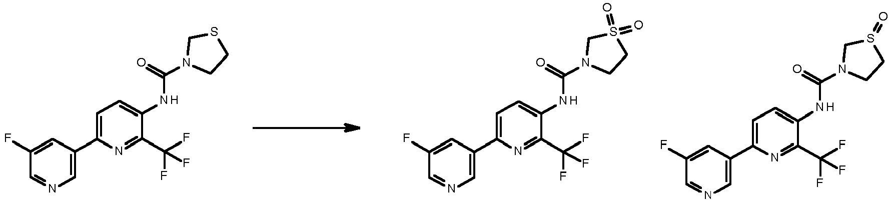

To a stirred solution of N-[6-(5-fluoro-3-pyridyl)-2-(trifluoromethyl)-3-pyridyl]thiazolidine-3-carboxamide (200 mg, 0.54 mmol) in DCM (10 mL) was added mCPBA (265mg, 1.07 mmol) and the reaction stirred at room temperature for 18 hours. The reaction mixture was diluted with DCM (20 mL) and then basified carefully with saturated aqueous sodium bicarbonate solution. The two layers were separated and the aqueous extracted again with DCM (10 mL). The combined organic extracts were washed with 10% sodium metabisulfite solution, dried over MgSO₄ and evaporated to dryness under reduced pressure. The crude product was purified by mass-directed reverse phase HPLC to give the desired products; **B116** (40mg, 19%) as a white solid; **B117** (30mg, 15%) as an offwhite solid.

**B116** ¹H NMR (400 MHz, CD₃OD) δ 9.13 (s, 1H), 8.58 (d, 1H), 8.38-8.31 (m, 1H), 8.29 (d, 1H), 8.14 (d, 1H), 4.58 (s, 2H), 4.10 (t, 2H), 3.97 (t, 2H).

**B117** ¹H NMR (400 MHz, CD₃OD) δ 9.14 (s, 1H), 8.59 (d, 1H), 8.41-8.34 (m, 1H), 8.28 (d, 1H), 8.20 (d, 1H), 4.95-4.90 (m, 1H), 4.47 (d, 1H), 4.32-4.20 (m, 1H), 4.20-4.10 (m, 1H), 3.42-3.32 (m, 1H), 3.22-3.12 (m, 1H).

### EXAMPLE P14: Synthesis of 3-[6-(5-fluoro-3-pyridyl)-2-(trifluoromethyl)-3-pyridyl]-5-methyl-1,3,5-oxadiazinan-4-one (Compound B121)

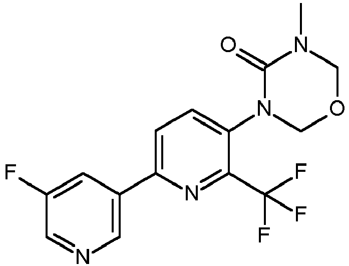

### Step 1: Synthesis of 3-[6-(5-fluoro-3-pyridyl)-2-(trifluoromethyl)-3-pyridyl]-5-methyl-1,3,5-oxadiazinan-4-one (Compound B121)

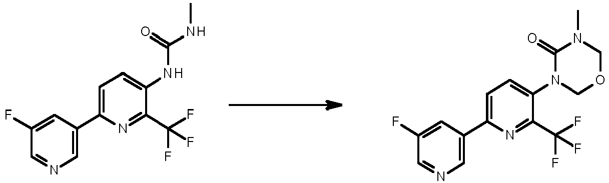

To a stirred solution of 1-[6-(5-fluoro-3-pyridyl)-2-(trifluoromethyl)-3-pyridyl]-3-methyl-urea (200 mg, 0.6365 mmol) in DCM (10 mL) were added paraformaldehyde (172mg, 1.91 mmol) and trifluoroacetic acid (0.32 mL) and the reaction mixture stirred at room temperature overnight. The reaction mixture was quenched with water (10 mL) and the two layers separated. The aqueous layer was extracted again with DCM (2 x 10 mL) and the combined organic extracts washed with brine, dried over MgSO₄ and evaporated to dryness under reduced pressure. The crude material was purified by flash chromatography on silica gel using an EtOAc/isohexane gradient followed by a MeOH/DCM gradient as eluent. The crude product was further purified by mass-directed reverse phase HPLC to give the desired compound (13mg, 6%) as a white solid.
¹H NMR (400 MHz, CD₃OD) δ 9.17 (s, 1H), 8.59 (d, 1H), 8.40-8.32 (m, 2H), 8.03 (s, 1H), 5.19-5.05 (br.m, 2H), 4.99 (s, 2H), 2.94 (s, 3H).

Further examples of the invention were made in an analogous manner using the methods described above in Examples P1 to P14, with respect to compounds B1, B15, B16, B29, B30, B32, B34, B37, B45, B76, B112, B68, B116, B117 and B121. Table 2 below, shows the structure of these compounds and the physical characterising data obtained using one or more of methods A to C as outlined below.

**Table 2: Characterising data for Compounds of formula (I) made by the methods described above**

| **Cmpd ID** | **Structure** | **¹H NMR Data (400MHz, CDCl₃ unless stated)** | **Mass/ Da** | ***m*/*z*** | **m/z method** |
|---|---|---|---|---|---|
| B1 | 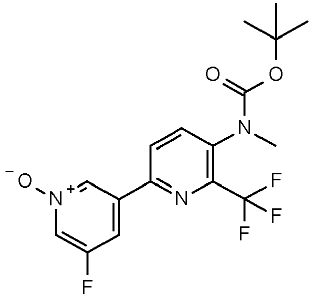 | (CD3OD, major rotamer) 8.97 (s, 1H), 8.53 (dd, 1H), 8.36 (d, 1H), 8.19 (d, 1H), 8.09 (d, 1H), 3.12 (s, 3H), 1.32 (s, 9H) | 387.1 | [MH]+ 388; tr 0.86mi ns | B |
| B2 | 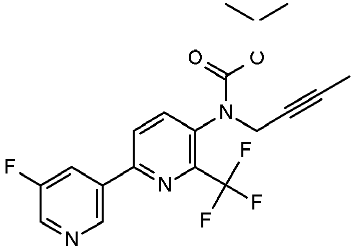 | (major rotamer) 9.08 (1H, s), 8.56 (s, 1H), 8.22 (br.d, 1H), 8.02 (d, 1H), 7.93 (d,1H), 4.79 (br.d, 1H), 3.92 (br.d, 1H), 1.79 (s, 3H), 1.33 (s, 9H) | 409.1 | [MH]+ 410; tr 0.88 mins | C |
| B3 | 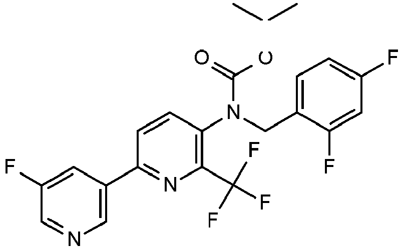 | (major rotamer) 9.02 (br. s, 1H), 8.54 (s, 1H), 8.19 (br.d, 1H), 7.88 (d, 1H) 7.7.54-7.29 (br.m, 2H), 6.87 (m, 1H), 6.72 (br.m, 1H), 5.17 (br.d, 1H), 4.43 (br.d, 1H), 1.34 (br. s, 9H) | 483.1 | [MH]+ 484 ; tr 0.96 mins | C |
| B4 | 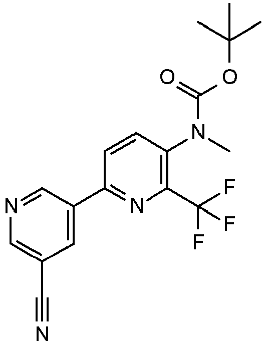 | (major rotamer) 9.44 (br.s, 1H), 8.96 (s, 1H), 8.72 (br.s, 1H), 8.02 (d, 1H), 7.81 (d, 1H), 3.21 (s, 3H), 1.33(br.s, 9H) | 378.1 | [MH]+ 379 ; tr 0.77 mins | C |
| B5 | 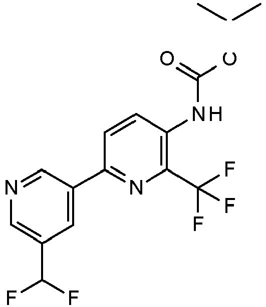 | 9.36 (s, 1H), 8.87-8.79 (m, 2H), 8.59 (s, 1H), 7.99 (d, 1H), 7.03 (br.s, 1H), 6.83 (t, 1H), 1.55 (s, 9H) | 389.1 | - | - |
| B6 | 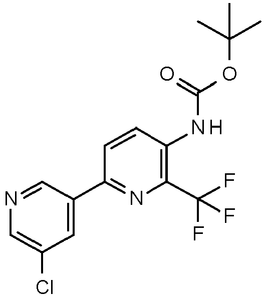 | 9.06 (d, 1H), 8.78 (d, 1H), 8.61 (d, 1H), 8.36 (m, 1H), 7.91 (d, 1H), 7.01 (br.s, 1H), 1.55 (s, 9H) | 373.1 | - | - |
| B7 | 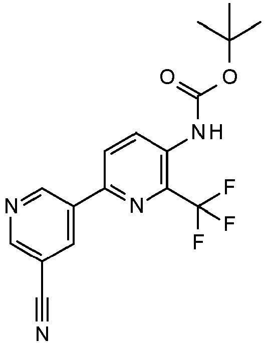 | 9.39 (d, 1H), 8.90 (d, 1H), 8.84 (m, 1H), 8.64 (s, 1H), 7.96 (d, 1H), 7.03 (br.s, 1H), 1.56 (s, 9H) | 364.1 | - | - |
| B9 | 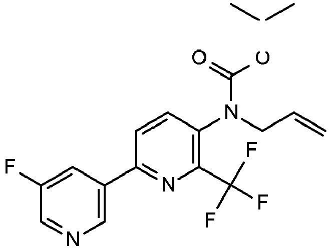 | (major rotamer) 9.04 (s, 1H), 8.56 (d, 1H), 8.19 (br.d, 1H), 7.97 (d, 1H), 7.68 (br.d, 1H), 5.99-5.87 (m, 1H), 5.23-5.03 (m, 2H), 4.60 (br.d, 1H), 3.85-3.63 (br.m, 1H), 1.34 (br.s, 9H) | 397.1 | [MH]+ 398 ; tr 1.28 mins | B |
| B10 | 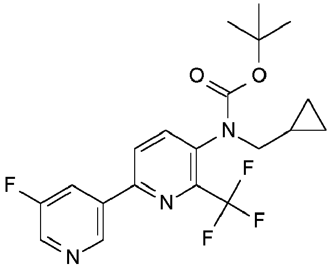 | (major rotamer) 9.05 (br.s, 1H), 8.54 (s, 1H), 8.21 (br.d, 1H), 7.99 (d, 1H), 7.82 (br.d, 1H), 3.91-3.80 (m, 1H), 3.20-3.10 (m, 1H), 1.32 (br.s, 9H), 1.00 (br. s, 1H), 0.48 (br.d, 2H), 0.13 (br.d, 2H) | 411.2 | [MH]+ 412 ; tr 0.91 mins | C |
| B11 | 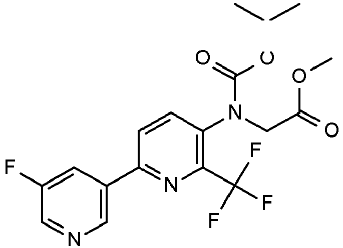 | (CD3OD, major rotamer) 9.16 (s, 1H), 8.58 (d, 1H), 8.39-8.31 (m, 2H), 8.29-8.23 (m, 1H), 4.62 (d, 1H), 3.92 (d, 1H), 3.78 (s, 3H), 1.32 (s, 9H) | 429.1 | [MH]+ 430; tr 0.81 mins | C |
| B12 | 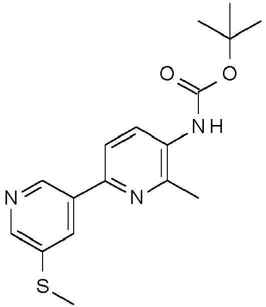 | 8.90 (d, 1H), 8.49 (d, 1H), 8.31 (br. d, 1H), 8.19 (m, 1H), 7.59 (d, 1H), 6.39 (br.s, 1H), 2.58 (s, 3H), 2.55 (s, 3H), 1.54 (s, 9H) | 331.1 | - | - |
| B13^{#} | 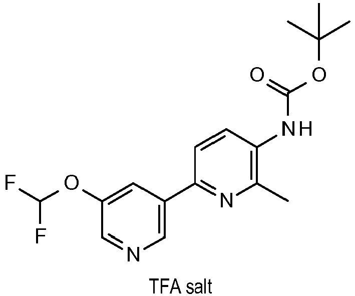 | 12.4 (br.s, 1H), 9.11 (br.s, 1H), 8.54 (br.s, 1H), 8.42 (d, 1H), 8.37 (s, 1H), 7.64 (d, 1H), 6.71 (t, 1H), 6.62 (br.s, 1H), 2.59 (s, 3H), 1.55 (s, 9H) | 351.1 | - | - |
| B14 | 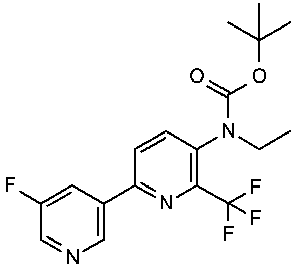 | (major rotamer) 9.07 (br.s, 1H), 8.57 (d, 1H), 8.21 (br.d, 1H), 8.01 (d, 1H), 7.72 (br.d, 1H), 3.91 (m, 1H), 3.47 (m, 1H), 1.59-1.21 (m, 12H) | 385.1 | [MH]+ 386 ; 1.25 mins | B |
| B15 | 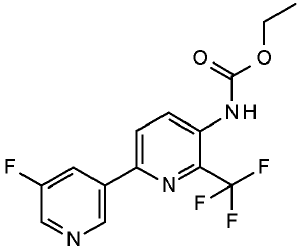 | 9.03 (d, 1H), 8.79 (d, 1H), 8.53 (d, 1H), 8.12 (m, 1H), 7.97 (d, 1H), 7.14 (br.s, 1H), 4.30 (q, 2H), 1.37 (t, 3H) | 329.1 | - | - |
| B16 | 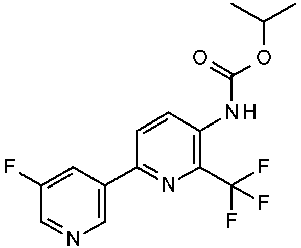 | 9.02 (d, 1H), 8.80 (d, 1H), 8.52 (d, 1H), 8.12 (m, 1H), 7.97 (d, 1H), 7.09 (br.s, 1H), 5.07 (m, 1H), 1.36 (d, 6H) | 343.1 | - | - |
| B17 | 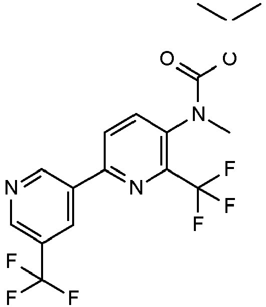 | (major rotamer) 9.44 (br.s, 1H), 9.97 (s, 1H), 8.64 (br.s, 1H), 8.04 (d, 1H), 7.79 (d, 1H), 3.22 (s, 3H), 1.33 (s, 9H) | 421.1 | [MH]+ 422 ; tr 0.90 mins | C |
| B18 | 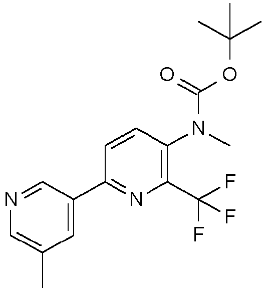 | (major rotamer) 9.02 (br.s, 1H), 8.52 (s, 1H), 8.25 (br.d, 1H), 7.98 (d, 1H), 7.72(d, 1H), 3.21 (s, 3H), 2.44 (s, 3H), 1.33(s,9H) | 367.2 | [MH]+ 368 ; tr 0.68 min | C |
| B19 | 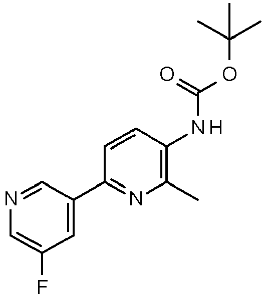 | 8.97 (s, 1H), 8.44 (d, 1H), 8.34 (br.d, 1H), 8.09-8.02 (m, 1H), 7.61 (d, 1H), 6.41 (br.s, 1H), 2.58 (s, 3H), 1.54 (s, 9H) | 303.1 | - | - |
| B20 | 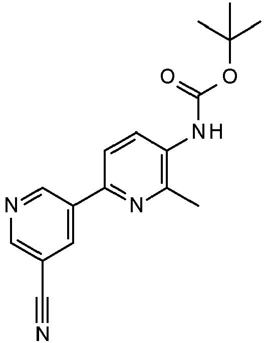 | 9.34 (d,1H), 8.83 (d, 1H), 8.61 (m, 1H), 8.41 (d, 1H), 7.63 (d, 1H), 6.42 (br.s, 1H), 2.59 (s, 3H), 1.57 (s, 9H) | 310.1 | - | - |
| B21 | 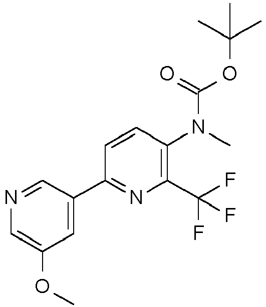 | (major rotamer) 8.81 (br.s, 1H), 8.39 (s, 1H), 8.02-7.89 (m, 2H), 7.79 (d, 1H), 3.96 (s, 3H), 3.21 (s, 3H), 1.33(s,9H) | 383.1 | [MH]+ 384 ; tr 0.74 mins | C |
| B22 | 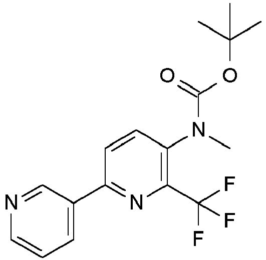 | (major rotamer) 9.27 (br.d, 1H), 8.69 (d, 1H), 8.44 (br.d, 1H), 8.00 (d, 1H), 7.73(d, 1H), 7.49-7.41 (m, 1H), 3.21 (s, 3H), 1.32 (s, 9H) | 353.1 | [MH]+ 354; tr 0.66 mins | C |
| B23 | 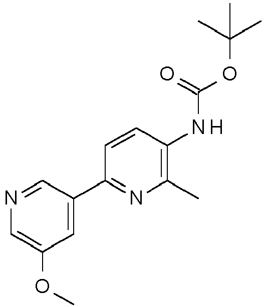 | 8.72 (s, 1H), 8.35-8.28 (m, 2H), 7.83 (d, 1H), 7.60 (d, 1H), 6.39 (br.s, 1H), 3.93 (s, 3H), 2.59 (s, 3H), 1.54 (s,9H) | 315.2 | - | - |
| B24 | 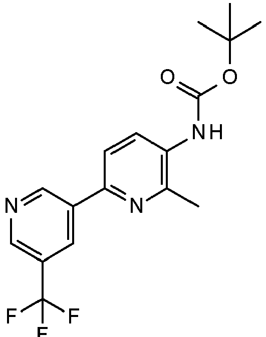 | 9.32 (s, 1H), 8.86 (s, 1H), 8.56 (s, 1H), 8.39 (br. d, 1H), 7.67 (d, 1H), 6.41 (br.s, 1H), 2.59 (s, 3H), 1.54 (s, 9H) | 353.1 | - | - |
| B25 | 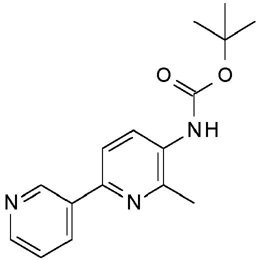 | 9.16 (d, 1H), 8.61 (m, 1H), 8.21-8.13 (m, 2H),7.59 (d, 1H), 7.37 (m, 1H), 6.37(br.s, 1H), 2.59 (s, 3H), 1.52 (s, 9H) | 285.1 | - | - |
| B26 | 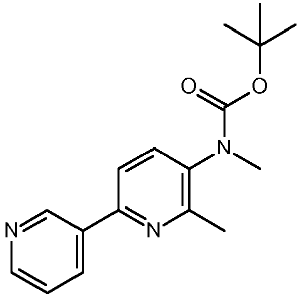 | (major rotamer) 9.38 (s, 2H), 9.24 (s, 1H), 7.64-7.50 (m, 2H), 3.20 (s, 3H), 2.53 (s, 3H), 1.37 (s, 9H) | 300.2 | - | - |
| B27 | 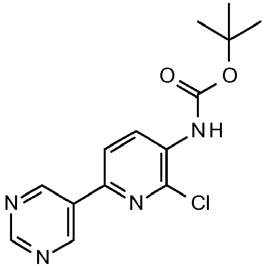 | 9.28 (s, 2H), 9.23 (s, 1H), 8.67 (d, 1H), 7.70 (d, 1H), 7.14 (br.s, 1H), 1.56 (s, 9H) | 306.1 | - | - |
| B28 | 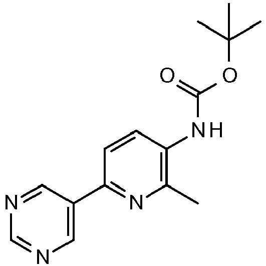 | 9.29 (s, 2H), 9.21 (s, 1H), 8.39 (d, 1H), 7.61 (d, 1H), 6.42 (br.s, 1H), 2.59 (s, 3H), 1.56 (s, 9H) | 286.1 | - | - |
| B29 | 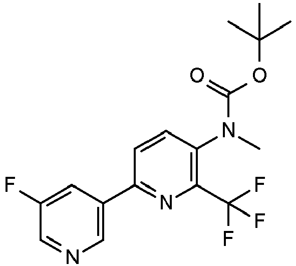 | (major rotamer) 9.07 (s, 1H), 8.57 (d, 1H), 8.20 (br.d, 1H), 8.01 (d, 1H), 7.76 (d, 1H), 3.22 (s, 3H), 1.33 (s, 9H) | 371.1 | [MH]+ 372; tr 1.17 min | B |
| B30 | 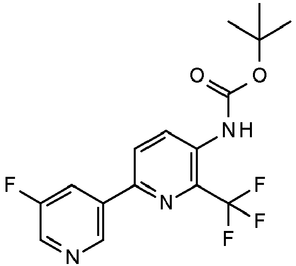 | 9.02 (dd, 1H), 8.79 (d, 1H), 8.52 (d, 1H), 8.12 (m, 1H), 7.94 (d, 1H), 7.01 (br.s, 1H), 1.56 .(s, 9H) | 357.1 | [MH]+ 358; tr 1.18 mins | B |
| B31 | 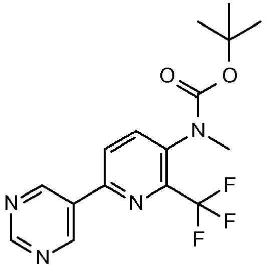 | (major rotamer) 9.41 (s, 2H), 9.32 (s, 1H), 8.07 (d,1H),7.74 (d, 1H), 3.22 (s, 3H), 1.34 (s, 9H) | 354.1 | [MH]+ 355 ; tr 0.96 mins | B |
| B32 | 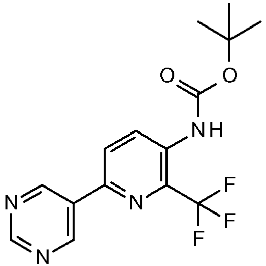 | 9.33 (s, 2H), 9.27 (s, 1H), 8.81 (d, 1H), 7.92 (d, 1H), 7.02 (br.s, 1H), 1.54 (s, 9H) | 340.1 | [MH]+ 341 ; tr 0.97 mins | B |
| B33 | 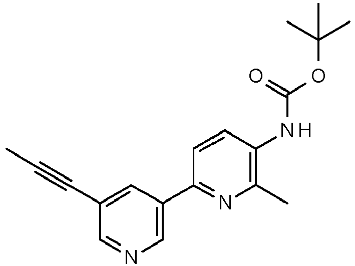 | 9.03 (d, 1H), 8.57 (d, 1H), 8.31 (br. d, 1H), 8.26 (m, 1H), 7.57 (d, 1H), 6.39 (br.s, 1H), 2.58 (s, 3H), 2.09 (s, 3H), 1.55 (s, 9H) | 323.2 | - | - |
| B34 | 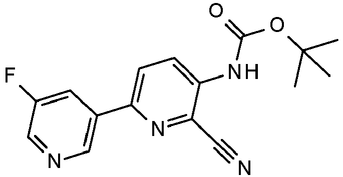 | 9.06-8.90 (m, 1H), 8.78 (d, 1H), 8.53 (d, 1H), 8.15-8.00 (m, 1H), 7.95 (d, 1H), 7.18 (br.s, 1H), 1.58 (s, 9H) | 314.1 | - | - |
| B35 | 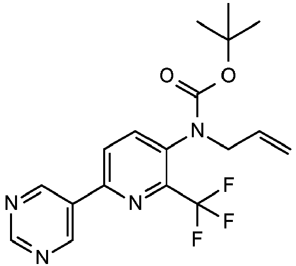 | (major rotamer) 9.45 (br.s, 2H), 9.30 (s, 1H), 8.05 (d, 1H), 7.82 (d, 1H), 5.95 (m, 1H), 5.25-5.05 (m, 2H), 4.58 (br.d, 1H), 3.75 (br. m, 1H), 1.33 (s, 9H) | 380.1 | [MH]+ 381; tr 0.75 mins | C |
| B36 | 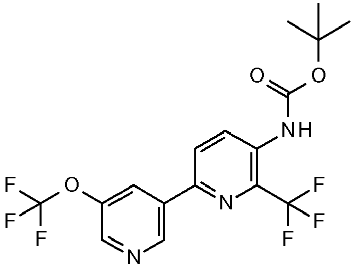 | 9.15 (d, 1H), 8.81 (d, 1H), 8.59 (d, 1H), 8.25 (s, 1H), 7.94 (d, 1H), 7.05 (br.s, 1H), 1.57 (s, 9H) | 423.1 | - | - |
| B37 | 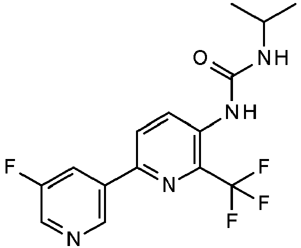 | 9.01 (m, 1H), 8.81 (d, 1H), 8.50 (d, 1H), 8.13-8.08 (m, 1H), 7.92 (d, 1H), 6.68 (br.s, 1H), 4.69 (br.s, 1H), 4.05-3.94 (m, 1H), 1.25 (m, 6H) | 342.1 | [MH]+ 343 ; tr 0.61 mins | C |
| B38 | 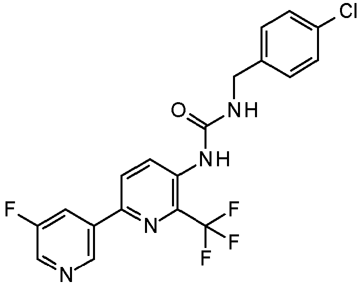 | (CD₃OD) 9.12 (s, 1H), 8.64 (d, 1H), 8.53 (d, 1H), 8.35-8.28 (m, 1H), 8.22 (d, 1H), 7.40-7.32 (m, 4H), 4.42 (s, 2H) | 424.1 | [MH]+ 425 ; tr 0.74 mins | C |
| B39 | 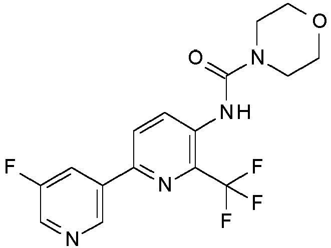 | (CDCl₃) 9.01 (s, 1H), 8.75 (d, 1H), 8.52 (d, 1H), 8.13-8.09 (m, 1H), 7.94 (d, 1H), 7.00 (br.s, 1H), 3.82-3.75 (m, 4H), 3.58-3.49 (m, 4H) | 370.1 | [MH]+ 371 ; tr 0.49 mins | C |
| B40 | 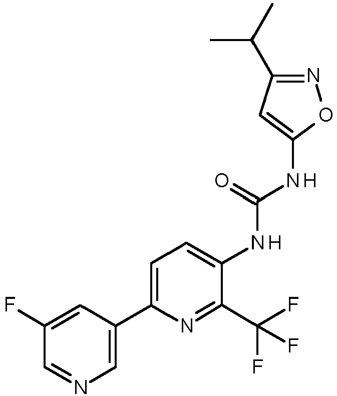 | (CD₃OD) 9.15 (s, 1H), 8.73 (d, 1H), 8.56 (d, 1H), 8.38-8.30 (m, 1H), 8.30 (d, 1H), 6.14 (s, 1H), 3.06-2.94 (m, 1H), 1.30 (d, 6H) | 409.1 | [MH]+ 410 ; tr 0.69 mins | C |
| B41 | 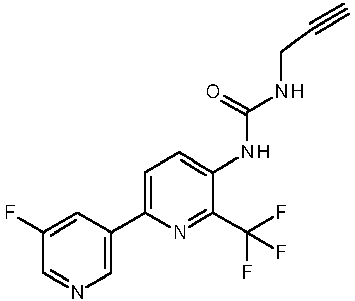 | 9.05 (s, 1H), 8.76 (d, 1H), 8.52 (s, 1H), 8.29-8.20 (m, 1H), 7.92 (d, 1H), 7.21 (s, 1H), 6.02 (br.s, 1H), 4.09 (d, 2H), 2.29 (dd, 1H) | 338.1 | [MH]+ 339 ; tr 0.54 mins | C |
| B42 | 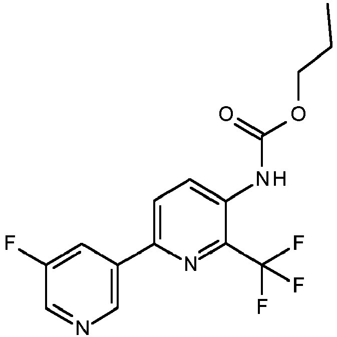 | 9.02 (s, 1H), 8.78 (d, 1H), 8.51 (d, 1H), 8.15-8.09 (m, 1H), 7.97 (d, 1H), 7.13 (br.s, 1H), 4.19 (t, 2H), 1.80-1.69 (m, 2H), 1.01 (t, 3H) | 343.1 | [MH]+ 344 ; tr 0.75 mins | C |
| B43 | 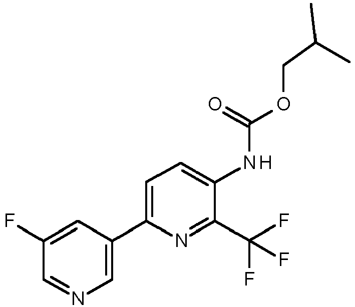 | 9.02 (s, 1H), 8.75 (d, 1H), 8.51 (d, 1H), 8.14-8.08 (m, 1H), 7.97 (d, 1H), 7.18 (br.s, 1H), 4.02 (d, 2H), 2.09-1.97 (m, 1H), 0.99 (d, 6H) | 357.1 | [MH]+ 358 ; tr 0.81 mins | C |
| B44 | 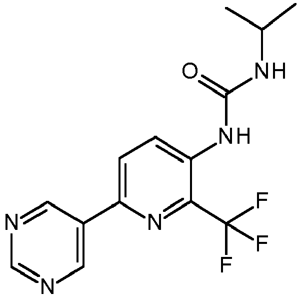 | (CD₃CN) 9.32 (s, 2H), 9.19 (s, 1H), 8.74 (d, 1H), 8.09 (d, 1H),7.05 (br.s, 1H), 5.85 (br.s, 1H), 3.88 (m, 1H), 1.17 (d, 6H) | 325.1 | | |
| B45 | 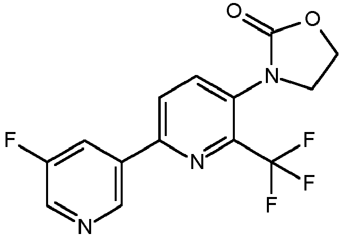 | 9.07 (s, 1H), 8.61 (d, 1H), 8.19 (m, 1H), 8.10 (d, 1H), 8.00 (d, 1H), 4.63 (dd, 2H), 4.05 (dd, 2H) | 327.1 | [MH]+ 328 ; tr 0.68 mins | B |
| B46 | 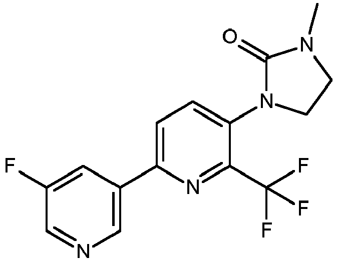 | 9.03 (s, 1H), 8.54 (d, 1H), 8.15 (m, 1H), 8.00 (d, 1H), 7.92 (d, 1H), 3.78 (m, 2H), 3.57 (m, 2H), 2.92 (s, 3H) | 340.1 | [MH]+ 341 ; tr 0.69 mins | B |
| B47 | 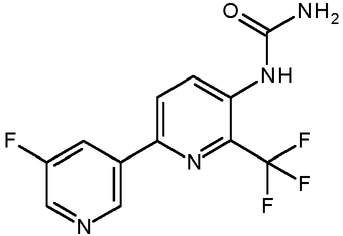 | (CD₃OD) 9.10 (s, 1H), 8.59 (d, 1H), 8.52 (d, 1H), 8.32-8.24 (m, 1H), 8.21 (d, 1H) | | | |
| B48 | 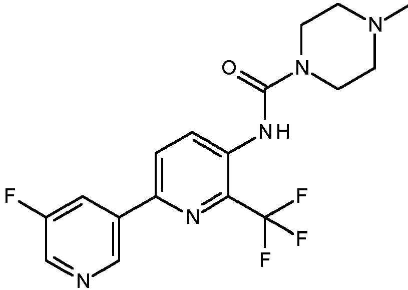 F | (CD₃OD) 9.12 (s, 1H), 8.54 (d, 1H), 8.37-8.29 (m, 1H), 8.27 (d, 1H), 8.09 (d, 1H), 3.64-3.52 (m, 4H), 2.56-2.45 (m, 4H), 2.34 (s, 3H) | | | |
| B49 | 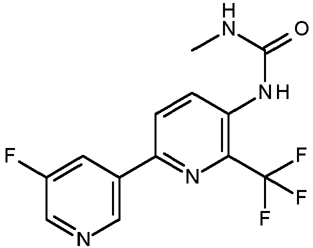 | (CD₃OD) 9.11 (s, 1H), 8.60 (d, 1H), 8.53 (d, 1H), 8.36-8.29 (m, 1H), 8.21 (d, 1H), 2.81 (s, 3H) | | | |
| B50 | 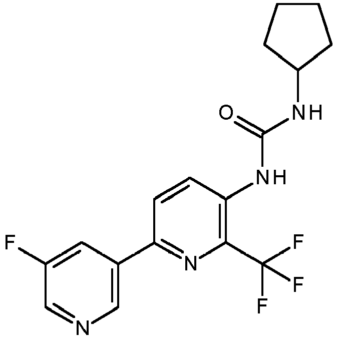 | (CD₃OD) d 9.09 (s, 1H), 8.64 (d, 1H), 8.50 (d, 1H), 8.32-8.24 (m, 1H), 8.19 (d, 1H), 4.12-4.03 (m, 1H), 2.03-1.91 (m, 2H), 1.82-1.59 (m, 4H), 1.55-1.44 (m, 2H) | | | |
| B51 | 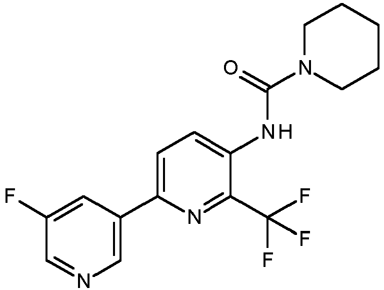 | (CD₃OD) 9.11 (s, 1H), 8.53 (d, 1H), 8.36-8.29 (m, 1H), 8.21 (d, 1H), 8.09 (d, 1H), 3.59-3.45 (m, 4H), 1.76-1.56 (m, 6H) | | | |
| B52 | 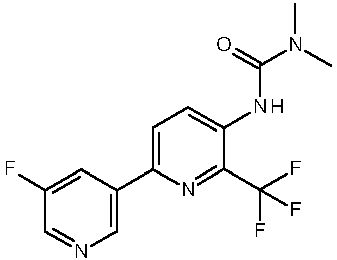 | (CD₃OD) 9.12 (s, 1H), 8.53 (d, 1H), 8.36-8.29 (m, 1H), 8.25 (d, 1H), 8.19 (d, 1H), 3.06 (s, 6H) | | | |
| B53 | 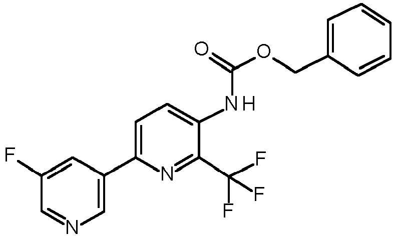 | 9.03 (s, 1H), 8.80 (d, 1H), 8.53 (d, 1H), 8.15-8.10 (m, 1H), 7.98 (d, 1H), 7.47-7.38 (m, 5H), 7.21 (br. s, 1H), 5.27 (s, 2H) | | | |
| B54 | 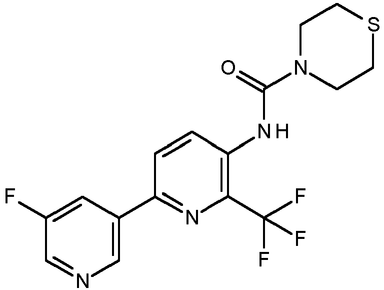 | (CD₃OD) 9.12 (s, 1H), 8.54 (d, 1H), 8.38-8.29 (m, 1H), 8.27 (d, 1H), 8.11-8.02 (m, 1H), 3.89-3.78 (m, 4H), 2.72-2.61 (m, 4H) | | | |
| B55 | 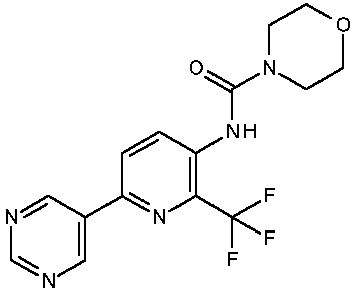 | 9.35 (s, 2H), 9.28 (s, 1H), 8.79 (d, 1H), 7.92 (d, 1H), 7.01 (br. s, 1H), 3.79 (m, 4H), 3.52 (m, 4H) | | | |
| B56 | 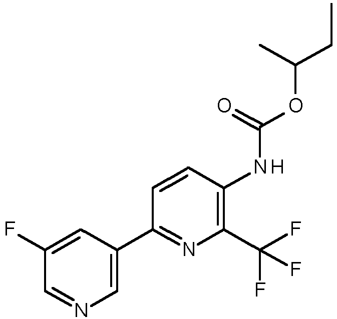 | (CD₃OD) 9.10 (s, 1H), 8.52 (d, 1H), 8.34 (d, 1H), 8.32-8.26 (m, 1H), 8.21 (d, 1H), 4.89-4.79 (m, 1H), 1.74-1.55 (m, 2H), 1.29 (d, 3H), 0.95 (t, 3H) | | | |
| B57 | 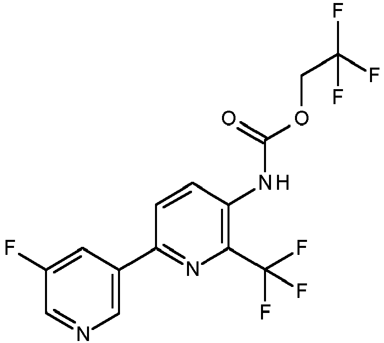 | (CD₃OD) 9.12 (s, 1H), 8.54 (d, 1H), 8.37-8.23 (m, 3H), 4.72 (q, 2H) | | | |
| B58 | 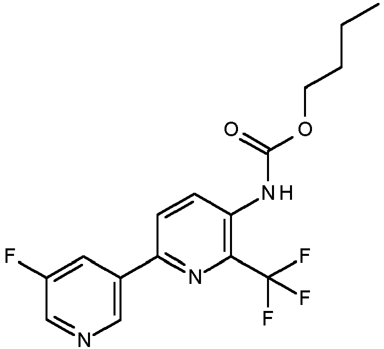 | (CD₃OD) 9.12 (s, 1H), 8.54 (d, 1H), 8.40-8.22 (m, 3H), 4.20 (t, 2H), 1.74-1.65 (m, 2H), 1.51-1.39 (m, 2H), 0.99 (t, 3H) | | | |
| B59 | 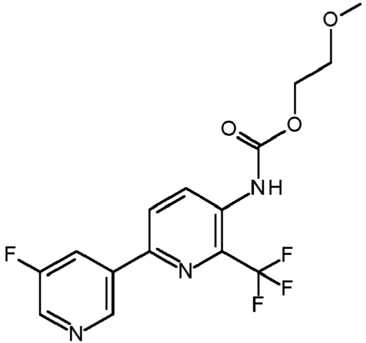 | (CD₃OD) 9.12 (s, 1H), 8.54 (d, 1H), 8.39 (d, 1H), 8.34-8.29 (m, 1H), 8.28 (d, 1H), 4.32 (t, 2H), 3.66 (t, 2H), 3.39 (s, 3H) | | | |
| B60 | 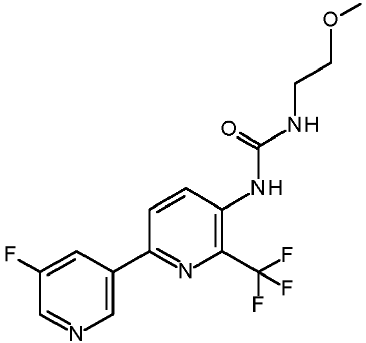 | (CD₃OD) 9.10 (s, 1H), 8.61 (d, 1H), 8.51 (d, 1H), 8.32-8.27 (m, 1H), 8.19 (d, 1H), 3.50 (t, 2H), 3.39 (t, 2H), 3.35 (s, 3H) | | | |
| B61 | 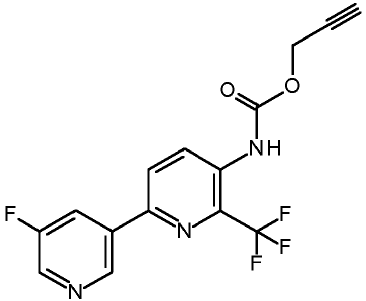 | (CD₃OD) 9.13 (s, 1H), 8.56 (d, 1H), 8.41-8.26 (m, 3H), 4.82 (d, 2H), 2.98 (t, 1H) | | | |
| B62 | 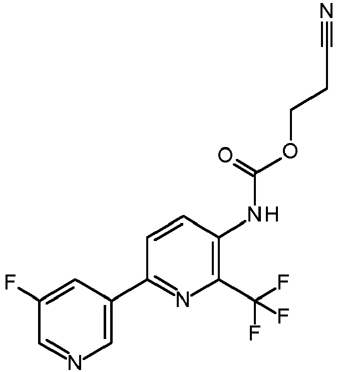 | (CD₃OD) 9.12 (s, 1H), 8.54 (d, 1H), 8.39-8.25 (m, 3H), 4.40 (t, 2H), 2.91 (t, 2H) | | | |
| B63 | 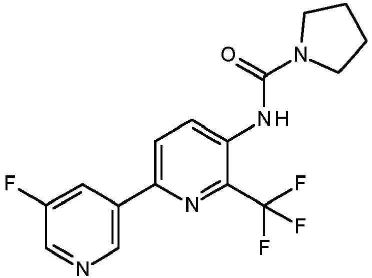 | 9.12 (s, 1H), 8.53 (d, 1H), 8.35-8.28 (m, 2H), 8.23 (d, 1H), 3.54-3.42 (m, 4H), 2.00 (br, 4H) | | | |
| B64 | 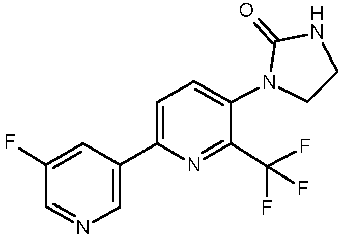 | 9.03 (s, 1H), 8.57 (d, 1H), 8.16 (d, 1H), 8.02 (d, 1H), 7.93 (d, 1H), 4.92 (br. s, 1H), 3.91 (t, 2H), 3.69 (t, 2H). | | | |
| B65 | 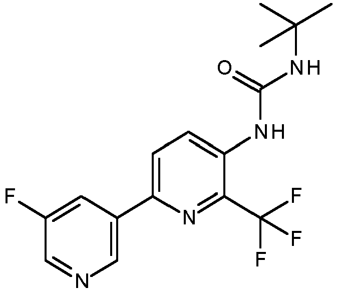 | (CD₃OD) 9.10 (s, 1H), 8.60 (d, 1H), 8.51 (d, 1H), 8.33-8.28 (m, 1H), 8.19 (d, 1H), 1.39 (s, 9H) | | | |
| B66 | 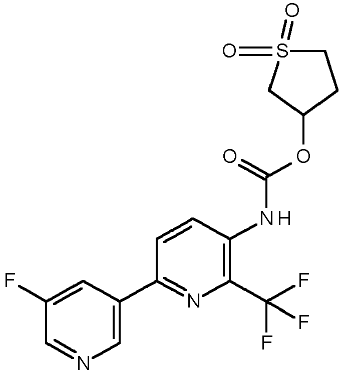 | (CD₃OD) 9.13 (s, 1H), 8.55 (d, 1H), 8.41-8.22 (m, 3H), 5.62-5.53 (m, 1H), 3.58-3.47 (m, 1H), 3.40-3.19 (m, 3H), 2.66-2.49 (m, 2H) | | | |
| B67 | 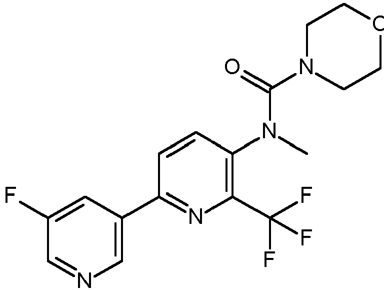 | (CD₃OD) 9.18 (s, 1H), 8.59 (d, 1H), 8.41-8.31 (m, 2H), 8.11 (d, 1H), 3.63-3.55 (m, 4H), 3.32-3.29 (m, 4H), 3.28 (s, 3H) | | | |
| B68 | 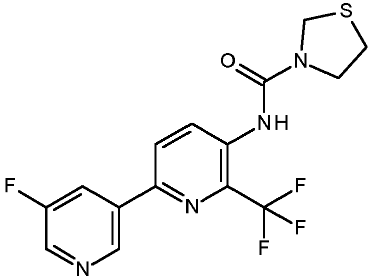 | (CD₃OD) 9.12 (s, 1H), 8.54 (d, 1H), 8.38-8.30 (m, 1H), 8.28 (d, 1H), 8.19 (d, 1H), 4.59 (s, 2H), 3.81 (t, 2H), 3.14 (t, 2H) | | | |
| B69 | 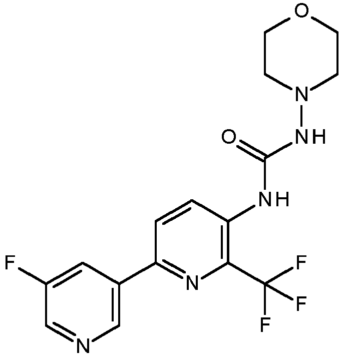 | 9.18 (s, 1H), 9.02 (s, 1H), 8.91 (d, 1H), 8.52 (d, 1H), 8.15-8.10 (m, 1H), 7.95 (d, 1H), 5.69 (s, 1H), 4.01-3.92 (m, 2H), 3.79-3.65 (m, 2H), 3.10-3.00 (m, 2H), 2.80-2.69 (m, 2H) | | | |
| B70 | 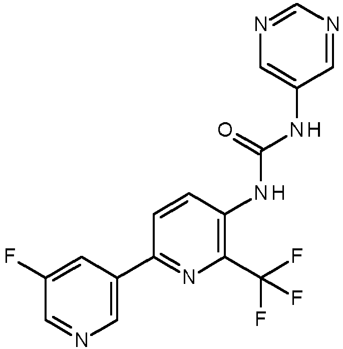 | (CD₃OD) 9.13 (s, 1H), 8.98 (s, 2H), 8.82 (s, 1H), 8.72 (d, 1H), 8.51 (d, 1H), 8.35-8.23 (m, 2H) | | | |
| B71 | 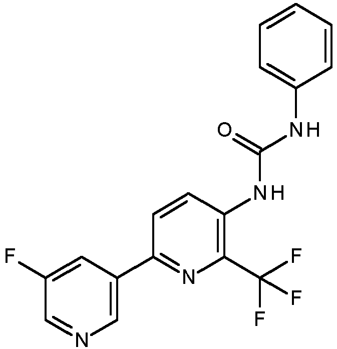 | (CD₃OD) 9.12 (s, 1H), 8.72 (d, 1H), 8.53 (d, 1H), 8.38-8.29 (m, 1H), 8.24 (d, 1H), 7.48 (d, 2H), 7.31 (t, 2H), 7.06 (t, 1H) | | | |
| B72 | 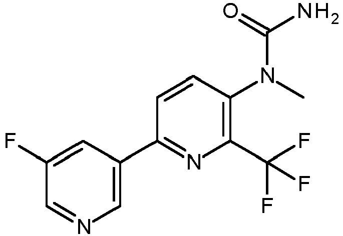 | (CD₃OD) 9.19 (s, 1H), 8.61 (d, 1H), 8.46-8.39 (m, 1H), 8.35 (d, 1H), 8.03 (d, 1H), 3.22 (s, 3H) | | | |
| B73 | 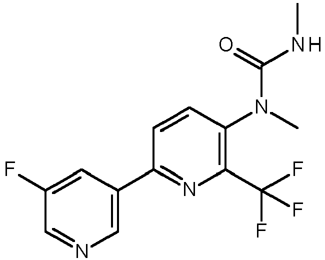 | (CD₃OD) 9.16 (s, 1H), 8.60 (d, 1H), 8.42-8.38 (m, 1H), 8.35 (d, 1H), 8.02 (d, 1H), 3.22 (s, 3H), 2.71 (s, 3H) | | | |
| B74 | 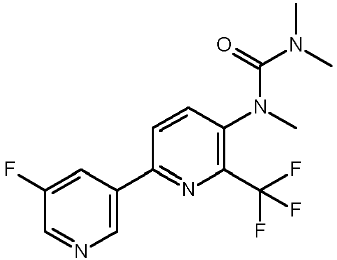 | (CD₃OD) 9.15 (s, 1H), 8.58 (d, 1H), 8.38-8.31 (m, 2H), 8.02 (d, 1H), 3.23 (s, 3H), 2.79 (s, 6H) | | | |
| B75 | 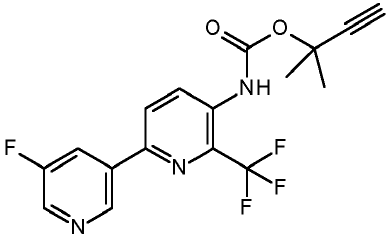 | (CD₃OD) 9.12 (s, 1H), 8.52 (d, 1H), 8.37-8.22 (m, 3H), 2.99 (s, 1H), 1.74 (s, 6H) | | | |
| B76 | 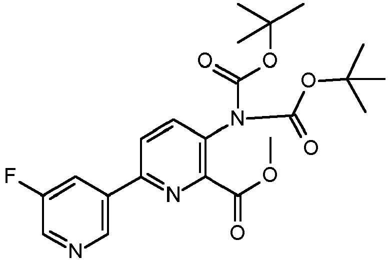 | 9.04 (br. s, 1H), 8.54 (br. s, 1H), 8.22-8.17 (m, 1H), 7.93 (d, 1H), 7.72 (d, 1H), 3.98 (s, 3H), 1.41 (s, 18H). | | | |
| B77 | 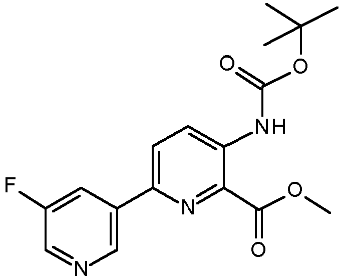 | 10.26 (s, 1H), 9.07-8.97 (m, 2H), 8.52 (br. s, 1H), 8.13-8.07 (m, 1H), 7.92 (d, 1H), 4.06 (s, 3H), 1.56 (s, 9H) | | | |
| B78 | 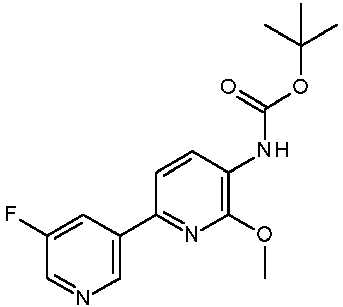 | 9.05 (s, 1H), 8.45 (s, 1H), 8.40 (dd, 1H), 8.05 (dd, 1H), 7.40 (d, 1H), 7.10 (s, 1H), 4.15 (s, 3H), 1.55 (s, 9H) | | | |
| B79 | 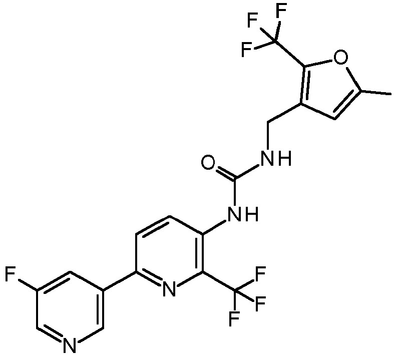 | (500 MHz, CD₃OD) 9.11 (s, 1H), 8.62 (d, 1H), 8.51 (d, 1H), 8.31-8.26 (m, 1H), 8.21 (s, 1H), 6.23 (s, 1H), 4.34 (s, 2H), 2.31 (s, 3H) | | | |
| B80 | 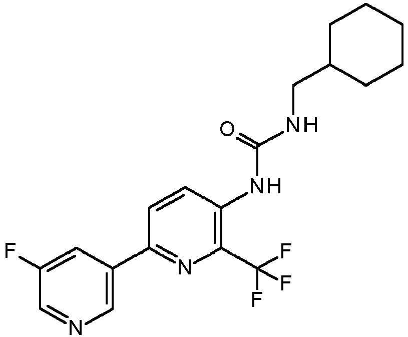 | (500 MHz, CD₃OD) 9.11 (s, 1H), 8.60 (d, 1H), 8.51 (d, 1H), 8.32-8.25 (m, 1H), 8.20 (d, 1H), 3.08 (d, 2H), 1.84-1.62 (m, 5H), 1.56-1.44 (m, 1H), 1.38-1.17 (m, 3H), 1.08-0.96 (m, 2H) | | | |
| B81 | 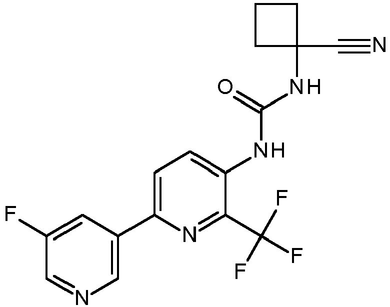 | (500 MHz, CD₃OD) 9.10 (s, 1H), 8.64 (d, 1H), 8.52 (d, 1H), 8.31-8.25 (m, 1H), 8.21 (d, 1H), 2.79-2.71 (m, 2H), 2.48-2.39 (m, 2H), 2.23-2.12 (m, 2H) | | | |
| B83 | 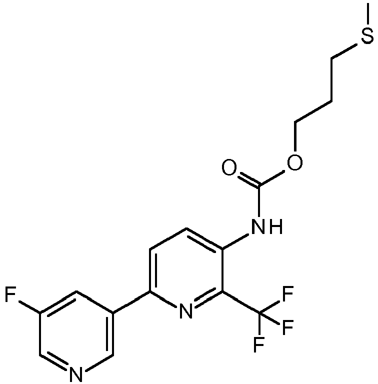 | (500 MHz, CD₃OD) 9.12 (s, 1H), 8.53 (s, 1H), 8.36 (d, 1H), 8.32-8.29 (m, 1H), 8.27 (d, 1H), 4.30 (t, 2H), 2.62 (t, 2H), 2.11 (s, 3H), 2.03-1.96 (m, 2H) | | | |
| B85 | 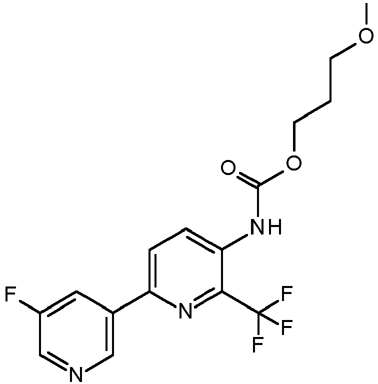 | (500 MHz, CD₃OD) 9.12 (s, 1H), 8.53 (d, 1H), 8.39 (d, 1H), 8.33-8.29 (m, 1H), 8.27 (d, 1H), 4.28 (t, 2H), 3.52 (t, 2H), 3.34 (s, 3H), 2.01-1.91 (m, 2H) | | | |
| B88 | 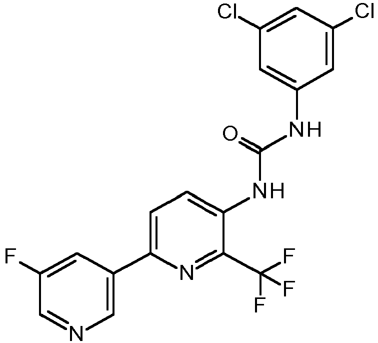 | (500 MHz, CD₃OD) 9.12 (s, 1H), 8.71 (d, 1H), 8.52 (d, 1H), 8.35-8.29 (m, 1H), 8.28 (d, 1H), 7.51 (s, 1H), 7.10 (s, 2H) | | | |
| B89 | 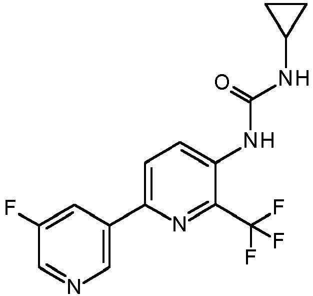 | (CD₃OD) 9.12 (s, 1H), 8.63 (d, 1H), 8.52 (d, 1H), 8.35-8.27 (m, 1H), 8.22 (d, 1H), 2.68-2.59 (m, 1H), 0.79 (d, 2H), 0.54 (d, 2H) | | | |
| B90 | 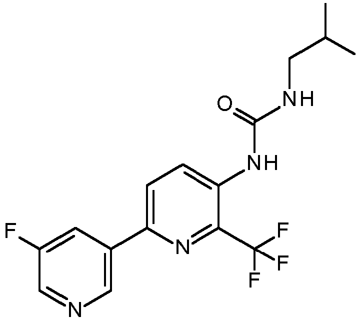 | (500 MHz, CD₃OD) 9.09 (s, 1H), 8.59 (d, 1H), 8.51 (d, 1H), 8.31-8.24 (m, 1H), 8.19 (d, 1H), 3.08 (d, 2H), 1.86-1.75 (m, 1H), 0.97 (d, 6H) | | | |
| B91 | 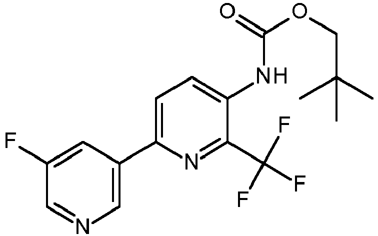 | (500 MHz, CD₃OD) 9.12 (s, 1H), 8.54 (d, 1H), 8.34-8.27 (m, 3H), 3.92 (s, 2H), 0.99 (s, 9H) | | | |
| B92 | 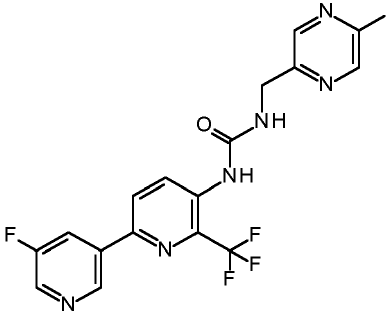 | (500 MHz, CD₃OD) 9.09 (s, 1H), 8.59 (d, 1H), 3.53-8.48 (m, 3H), 8.30-8.25 (m, 1H), 8.19 (d, 1H), 4.57 (s, 2H), 2.54 (s, 3H) | | | |
| B93 | 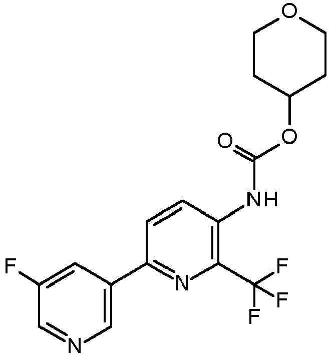 | 9.02 (s, 1H), 8.76 (d, 1H), 8.52 (d, 1H), 8.13 (m, 1H), 7.98 (d, 1H), 7.13 (br.s, 1H), 5.00 (m, 1H), 3.98 (m, 2H), 3.57 (m, 2H), 2.03 (m, 2H), 1.81 (m, 2H) | | | |
| B94 | 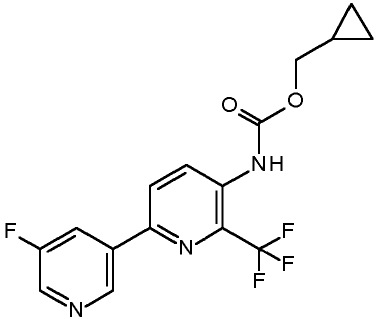 | 9.02 (s, 1H), 8.78 (d, 1H), 8.52 (d, 1H), 8.12 (m, 1H), 7.98 (d, 1H), 7.17 (br. s, 1H), 4.05 (d, 2H), 1.22 (m ,1H), 0.67-0.62 (2H, m), 0.37-0.34 (2H, m) | | | |
| B95 | 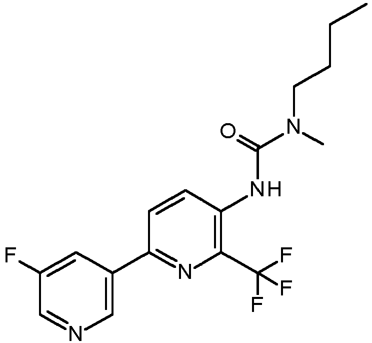 | (500 MHz, CD₃OD) 9.12 (s, 1H), 8.53 (d, 1H), 8.34-8.29 (m, 1H), 8.25 (d, 1H), 8.19 (d, 1H), 3.42 (t, 2H), 3.07 (s, 3H), 1.68-1.58 (m, 2H), 1.44-1.33 (m, 2H), 1.01 (t, 3H) | | | |
| B96 | 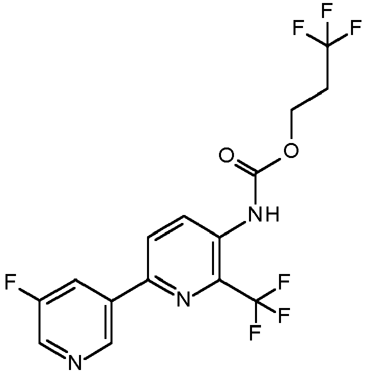 | 9.02 (t, 1H), 8.71 (d, 1H), 8.53 (d, 1H), 8.13 (m, 1H), 7.99 (d, 1H), 7.17 (br. s, 1H), 4.48 (t, 2H), 2.58 (m, 2H) | | | |
| B97 | 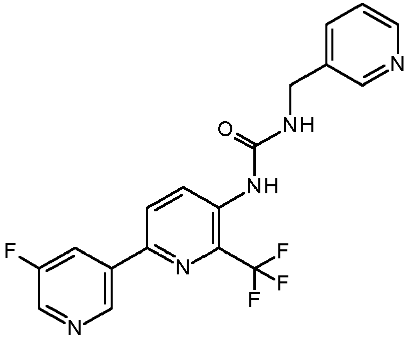 | (500 MHz, CD₃OD) 9.11 (s, 1H), 8.78 (s, 1H), 8.72-8.68 (m, 1H), 8.59 (d, 1H), 8.52 (d, 1H), 8.41 (d, 1H), 8.31-8.27 (m, 1H), 8.21 (d, 1H), 7.93-7.89 (m, 1H), 4.61 (s, 2H) | | | |
| B98 | 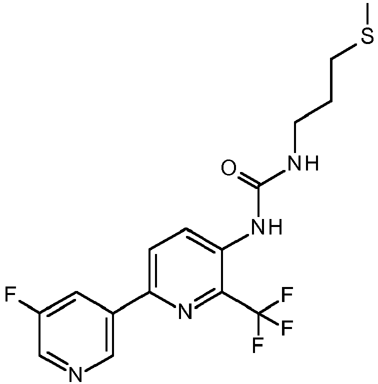 | (500 MHz, CD₃OD) 9.09 (s, 1H), 8.61 (d, 1H), 8.51 (d, 1H), 8.30-8.26 (m, 1H), 8.19 (d, 1H), 3.34 (t, 2H), 2.58 (t, 2H), 2.11 (s, 3H), 1.89-1.81 (m, 2H) | | | |
| B102 | 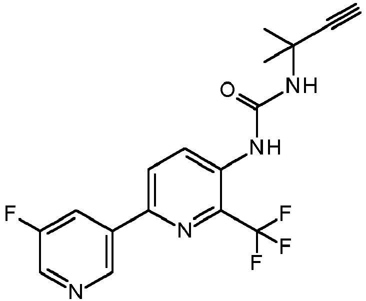 | (CD₃OD) 9.10 (s, 1H), 8.67-8.61 (m, 1H), 8.51 (d, 1H), 8.31-8.24 (m, 1H), 8.19 (d, 1H), 2.69 (s, 1H), 1.63 (s, 6H) | | | |
| B104 | 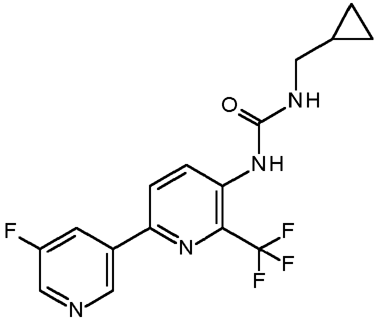 | (500 MHz, CD₃OD) 9.10 (s, 1H), 8.62 (d, 1H), 8.51 (d, 1H), 8.31-8.24 (m, 1H), 8.18 (d, 1H), 3.11 (d, 2H), 1.11-0.99 (m, 1H), 0.59-0.50 (m, 2H), 0.29-0.22 (m, 2H) | | | |
| B105 | 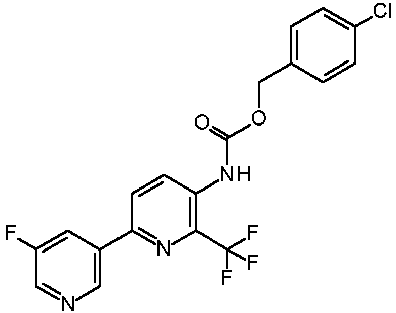 | (500 MHz, CD₃OD) 9.12 (s, 1H), 8.53 (d, 1H), 8.36 (d, 1H), 8.33-8.29 (m, 1H), 8.28 (d, 1H), 7.42 (d, 2H), 7.39 (d, 2H), 5.22 (s, 2H) | | | |
| B106 | 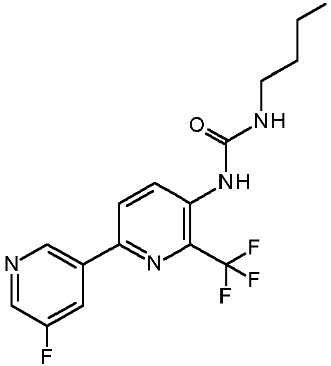 | (500 MHz, CD₃OD) 9.09 (s, 1H), 8.61 (d, 1H), 8.51 (s, 1H), 8.31-8.25 (m, 1H), 8.19 (d, 1H), 3.23 (t, 2H), 1.59-1.50 (m, 2H), 1.49-1.39 (m, 2H), 0.99 (t, 3H) | | | |
| B107 | 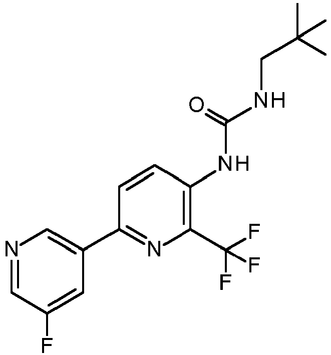 | (500 MHz, CD₃OD) 9.11 (s, 1H), 8.59 (d, 1H), 8.51 (d, 1H), 8.31-8.27 (m, 1H), 8.19 (d, 1H), 3.06 (s, 2H), 0.97 (s, 9H) | | | |
| B108 | 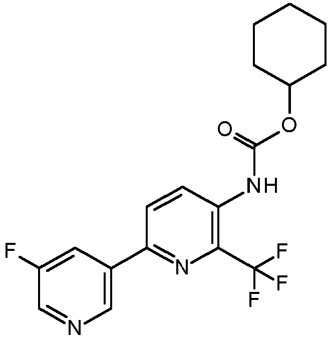 | 9.03 (s, 1H), 8.72 (d, 1H), 8.53 (d, 1H), 8.13 (m, 1H), 7.91 (d, 1H), 7.07 (br. s, 1H), 4.78 (m, 1H), 1.99-1.91 (m, 2H), 1.83-1.71 (m, 2H), 1.66-1.18 (6H, m) | | | |
| B109 | 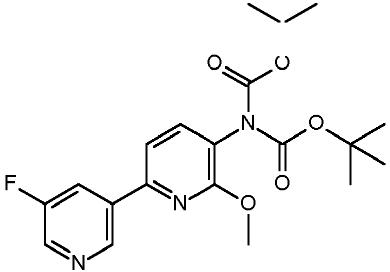 | 9.05 (s, 1H), 8.50 (s, 1H), 8.10 (dd, 1H), 7.55 (dd, 1H), 7.40 (d, 1H), 4.15 (s, 3H), 1.40 (s, 18H) | | | |
| B110 | 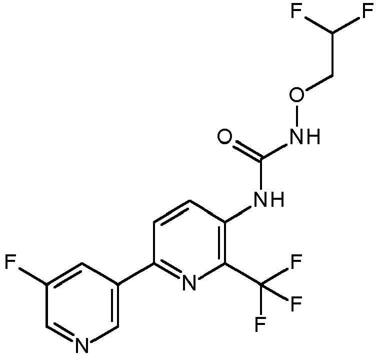 | 8.98 (s, 1H), 8.77 (d, 1H), 8.47 (s, 1H), 8.22 (s, 1H), 8.10-8.03 (m, 1H), 7.92 (d, 1H), 7.62 (s, 1H), 6.01 (tt, 1H), 4.12 (td, 2H) | | | |
| B111 | 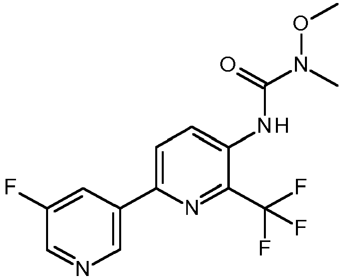 | 8.95 (s, 1H), 8.83 (d, 1H), 8.45 (d, 1H), 8.39 (br, 1H), 8.08-8.01 (m, 1H), 7.89 (d, 1H), 3.74 (s, 3H), 3.18 (s, 3H) | | | |
| B112 | 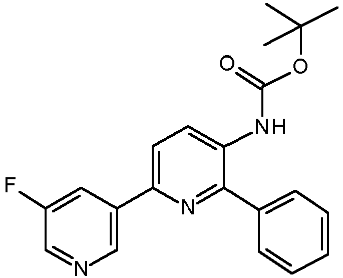 | 9.02 (s, 1H), 8.64 (br d, 1H), 8.46 (s, 1H), 8.14-8.08 (m, 1H), 7.75 (d, 1H), 7.68-7.63 (m, 2H), 7.61-7.55 (m, 2H), 7.54-7.49 (m, 1H), 6.81 (s, 1H), 1.50 (s, 9H) | | | |
| B113 | 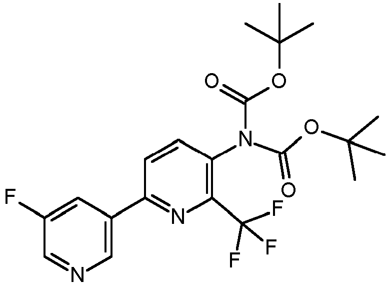 | 9.08 (dd, 1H), 8.57 (d, 1H), 8.22 (m, 1H), 8.03 (d, 1H), 7.75 (d, 1H), 1.41 (s, 18H) | | | |
| B114 | 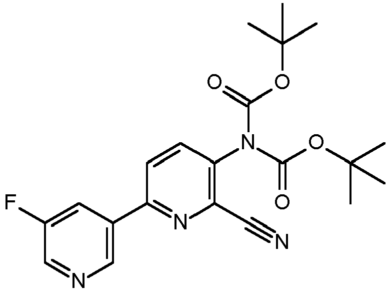 | 9.04 (s, 1H), 8.59 (d, 1H), 8.16 (m, 1H), 8.03 (d, 1H), 7.80 (d, 1H), 1.49 (s, 18H) | | | |
| B115 | 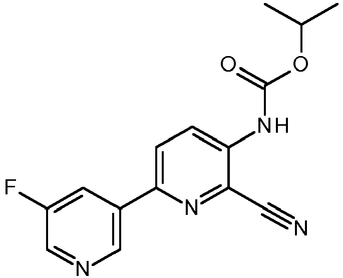 | 8.98 (t, 1H), 8.81 (d, 1H), 8.52 (d, 1H), 8.18 (m, 1H), 7.92 (d, 1H), 7.18 (br.s, 1H), 5.06 (m, 1H), 1.38 (d, 6H) | | | |
| B116 | 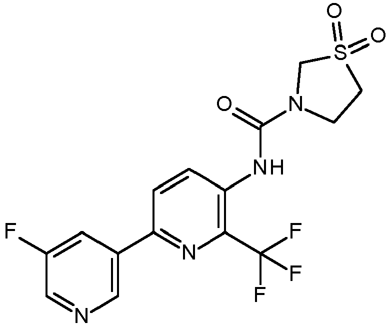 | (CD₃OD) 9.13 (s, 1H), 8.58 (d, 1H), 8.38-8.31 (m, 1H), 8.29 (d, 1H), 8.14 (d, 1H), 4.58 (s, 2H), 4.10 (t, 2H), 3.97 (t, 2H) | | | |
| B117 | 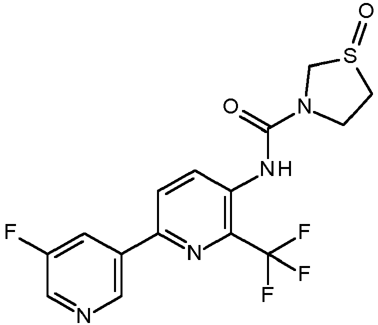 | (CD₃OD) 9.14 (s, 1H), 8.59 (d, 1H), 8.41-8.34 (m, 1H), 8.28 (d, 1H), 8.20 (d, 1H), 4.95-4.90 (m, 1H), 4.47 (d, 1H), 4.32-4.20 (m, 1H), 4.20-4.10 (m, 1H), 3.42-3.32 (m, 1H), 3.22-3.12 (m, 1H) | | | |
| B118 | 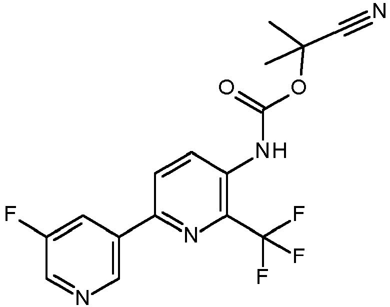 | (CD₃OD) 9.21 (s, 1H), 8.62 (d, 1H), 8.49 (d, 1H), 8.43-8.38 (m, 1H), 8.29 (d, 1H), 1.75 (s, 3H), 1.69 (s, 3H) | | | |
| B119 | 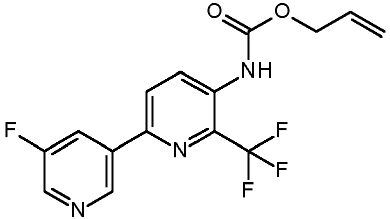 | (CD₃OD) 9.12 (s, 1H), 8.53 (d, 1H), 8.40-8.23 (m, 3H), 6.07-5.94 (m, 1H), 5.38 (dd, 1H), 5.26 (dd, 1H), 4.69 (dd, 2H) | | | |
| B121 | 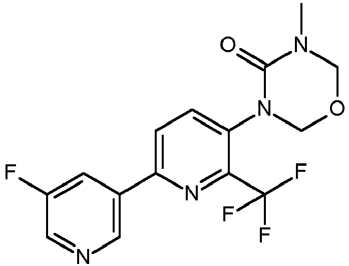 | (CD₃OD) 9.17 (s, 1H), 8.59 (d, 1H), 8.40-8.32 (m, 2H), 8.03 (s, 1H), 5.19-5.05 (br.m, 2H), 4.99 (s, 2H), 2.94 (s, 3H) | | | |
| B123 | 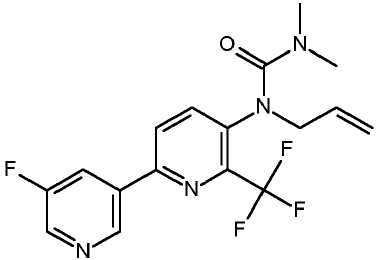 | 9.14 (s, 1H), 8.57 (d, 1H), 8.38-8.32 (m, 1H), 8.31 (d, 1H), 8.00 (d, 1H), 6.03-5.92 (m, 1H), 5.16-5.09 (m, 2H), 4.26 (d, 2H), 2.77 (s, 6H) | | | |
| B125 | 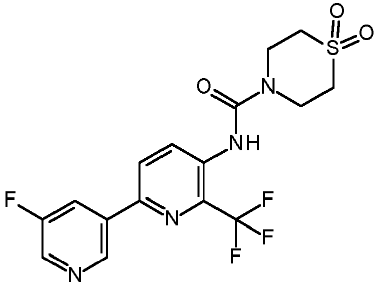 | (CD₃OD) 9.14 (s, 1H), 8.57 (d, 1H), 8.38-8.31 (m, 1H), 8.29 (d, 1H), 8.11 (d, 1H), 4.11-4.00 (m, 4H), 3.26-3.14 (m, 4H) | | | |

### Physical characterisation

Compounds of the invention were characterised using one or more of the following methods.

### NMR

NMR spectra contained herein were recorded on either a 400MHz Bruker AVANCE III HD equipped with a Bruker SMART probe or a 500MHz Bruker AVANCE III equipped with a Bruker Prodigy probe. Chemical shifts are expressed as ppm downfield from TMS, with an internal reference of either TMS or the residual solvent signals. The following multiplicities are used to describe the peaks: s = singlet, d = doublet, t = triplet, dd = double doublet, m = multiplet. Additionally br. is used to describe a broad signal and app. is used to describe an apparent multiplicity.

### LCMS

LCMS data contained herein consists of the molecular ion [MH+] and the retention time (tr) of the peak recorded on the chromatogram. The following instruments, methods and conditions were used to obtain LCMS data:

### Method A

*Instrumentation:* Waters Acquity UPLC-MS using a Sample Organizer with Sample Manager FTN, H-Class QSM, Column Manager, 2 x Column Manager Aux, Photodiode Array (Wavelength range (nm): 210 to 400, ELSD and SQD 2 equipped with a Waters HSS T3 C18 column (column length 30 mm, internal diameter of column 2.1 mm, particle size 1.8 micron).

*Ionisation method:* Electrospray positive and negative: Capillary (kV) 3.00, Cone (V) 30.00, Source Temperature (°C) 500, Cone Gas Flow (L/Hr.) 10, Desolvation Gas Flow (L/Hr.) 1000. Mass range (Da): positive 95 to 800, negative 115 to 800.

The analysis was conducted using a two minute run time, according to the following gradient table at 40°C:

| **Time (mins)** | **Solvent A (%)** | **Solvent B (%)** | **Flow (ml/mn)** |
|---|---|---|---|
| 0.00 | 95.0 | 5.0 | 0.7 |
| 1.75 | 0.0 | 100 | 0.7 |
| 1.76 | 0.0 | 100 | 0.7 |
| 2.0 | 0.0 | 5.0 | 0.7 |
| 2.01 | 95.0 | 5.0 | 0.7 |
| 2.11 | 95.0 | 5.0 | 0.7 |

| | | | |
|---|---|---|---|
| Solvent A: H₂O with 0.05% TFA Solvent B: CH₃CN with 0.05% TFA | | | |

### Method B (2 min method)

*Instrumentation:* Either (a) Waters Acquity UPLC system with Waters SQD2 single-quad MS detector, Photodiode Array Detector (Absorbance Wavelength: 254 nm, 10 pts/sec, Time Constant: 0.2000 sec), Charged Aerosol Detector (Corona) and Waters CTC 2770 auto-sampler unit (injection volume: 2 microliters, 1 min seal wash); or (b) Waters Acquity UPLC system with Waters QDa single-quad MS detector, Photodiode Array Detector (Absorbance Wavelength: 254 nm, 10 pts/sec, Time Constant: 0.2000 sec), Charged Aerosol Detector (Corona) and Waters CTC 2770 auto-sampler unit (injection volume: 2 microliters, 1 min seal wash).

### LC-Method:

Phenomenex 'Kinetex C18 100A' column (50 mm x 4.6 mm, particle size 2.6 micron),
Flow rate: 2 mL/min at 313K (40 Celsius),
Gradient (Solvent A: H₂O with 0.1% Formic Acid; Solvent B: Acetonitrile with 0.1% Formic Acid):
The analysis was conducted using a two minute run time, according to the following gradient table at 40°C.

| **Time (mins)** | **Solvent A (%)** | **Solvent B (%)** | **Flow (ml/mn)** |
|---|---|---|---|
| Initial | 70.0 | 30.0 | 2.000 |
| 1.20 | 10.0 | 90.0 | 2.000 |
| 1.70 | 10.0 | 90.0 | 2.000 |
| 1.80 | 70.0 | 30.0 | 2.000 |
| 2.00 | 70.0 | 30.0 | 2.000 |
| 2.20 | 70.0 | 30.0 | 2.000 |

### Method C (1 min method)

*Instrumentation:* Either (a) Waters Acquity UPLC system with Waters SQD2 single-quad MS detector, Photodiode Array Detector (Absorbance Wavelength: 254 nm, 10 pts/sec, Time Constant: 0.2000 sec), Charged Aerosol Detector (Corona) and Waters CTC 2770 auto-sampler unit (injection volume: 2 microliters, 1 min seal wash); or (b) Waters Acquity UPLC system with Waters QDa single-quad MS detector, Photodiode Array Detector (Absorbance Wavelength: 254 nm, 10 pts/sec, Time Constant: 0.2000 sec), Charged Aerosol Detector (Corona) and Waters CTC 2770 auto-sampler unit (injection volume: 2 microliters, 1 min seal wash).

### LC-Method:

Phenomenex 'Kinetex C18 100A' column (50 mm x 4.6 mm, particle size 2.6 micron), Flow rate: 2 mL/min at 313K (40 Celsius),
Gradient (Solvent A: H₂O with 0.1% Formic Acid; Solvent B: Acetonitrile with 0.1% Formic Acid):
The analysis was conducted using a one minute run time, according to the following gradient table at 40°C.

| **Time (mins)** | **Solvent A (%)** | **Solvent B (%)** | **Flow (ml/mn)** |
|---|---|---|---|
| Initial | 60.0 | 40.0 | 2.000 |
| 0.80 | 0.0 | 100.0 | 2.000 |
| 0.95 | 0.0 | 100.0 | 2.000 |
| 1.00 | 60.0 | 40.0 | 2.000 |
| 1.10 | 60.0 | 40.0 | 2.000 |
| 1.25 | 60.0 | 40.0 | 2.000 |

### BIOLOGICIAL EXAMPLES

### B1 Pre-emergence herbicidal activity

Seeds of a variety of test species were sown in standard soil in pots: *Triticum aestivium* (TRZAW), *Avena fatua* (AVEFA), *Alopecurus myosuroides* (ALOMY), *Echinochloa crus-galli* (ECHCG), *Lolium perenne* (LOLPE), *Zea Mays* (ZEAMX), *Abutilon theophrasti* (ABUTH), *Amaranthus retroflexus* (AMARE) and *Setaria faberi* (SETFA). After cultivation for one day (pre-emergence) under controlled conditions in a glasshouse (at 24/16°C, day/night; 14 hours light; 65% humidity), the plants were sprayed with an aqueous spray solution derived from the formulation of the technical active ingredient in acetone / water (50:50) solution containing 0.5% Tween 20 (polyoxyethelyene sorbitan monolaurate, CAS RN 9005-64-5). The test plants were then grown in a glasshouse under controlled conditions (at 24/16°C, day/night; 14 hours light; 65% humidity) and watered twice daily. After 13 days, the test was evaluated (5= total damage to plant; 0 = no damage to plant). Results are shown in Tables B1a and B1b.

### Tables B1a and B1b Control of weed species by compound of Formula (I) after pre-emergence application

**Table B1a: Test 1a**

| **Compound ID** | **Rate (g/ha)** | **LOLPE** | **SETFA** | **ALOMY** | **ECHCG** | **AVEFA** | **TRAZW** |
|---|---|---|---|---|---|---|---|
| B1 | 1000 | 1 | 4 | 0 | 2 | 2 | 0 |
| B3 | 1000 | 0 | 0 | 0 | 0 | 0 | 0 |
| B4 | 1000 | 0 | 4 | 0 | 1 | 0 | 1 |
| B5 | 1000 | 0 | 4 | 0 | 2 | 0 | 0 |
| B6 | 1000 | 1 | 5 | 1 | 3 | 2 | 0 |
| B7 | 1000 | 1 | 5 | 0 | 2 | 1 | 0 |
| B9 | 1000 | 2 | 5 | 1 | 3 | 2 | 0 |
| B10 | 1000 | 1 | 5 | 1 | 3 | 2 | 0 |
| B11 | 1000 | 0 | 1 | 0 | 0 | 0 | 0 |
| B12 | 1000 | 1 | 3 | 1 | 1 | 1 | 0 |
| B33 | 1000 | 1 | 4 | 0 | 2 | 2 | 0 |
| B34 | 1000 | 1 | 5 | 0 | 4 | 0 | 0 |
| B35 | 1000 | 1 | 4 | 0 | 2 | 0 | 0 |
| B36 | 1000 | 0 | 5 | 0 | 3 | 2 | 0 |
| B37 | 1000 | 1 | 5 | 0 | 4 | 2 | 0 |
| B38 | 1000 | 0 | 3 | 0 | 1 | 0 | 0 |
| B39 | 1000 | 1 | 5 | 0 | 4 | 3 | 0 |
| B40 | 1000 | 1 | 1 | 0 | 1 | 0 | 0 |
| B41 | 1000 | 1 | 5 | 0 | 4 | 1 | 0 |
| B42 | 1000 | 1 | 5 | 1 | 4 | 3 | 0 |
| B43 | 1000 | 1 | 4 | 1 | 3 | 1 | 1 |
| B44 | 1000 | 1 | 3 | 0 | 3 | 2 | 0 |
| B45 | 1000 | 0 | 3 | 0 | 3 | 0 | 0 |
| B46 | 1000 | 0 | 4 | 0 | 2 | 1 | 0 |
| B47 | 1000 | 1 | 5 | 0 | 4 | 1 | 0 |
| B48 | 1000 | 1 | 4 | 0 | 3 | 1 | 0 |
| B49 | 1000 | 0 | 4 | 0 | 3 | 2 | 0 |
| B50 | 1000 | 1 | 4 | 0 | 2 | 1 | 0 |
| B51 | 1000 | 1 | 5 | 0 | 2 | 1 | 0 |
| B52 | 1000 | 1 | NT | 0 | 3 | 2 | 0 |
| B53 | 1000 | 1 | 4 | 0 | 2 | 1 | 0 |
| B54 | 1000 | 1 | 3 | 0 | 3 | 2 | 0 |
| B55 | 1000 | 0 | 1 | 0 | 1 | 1 | 0 |
| B56 | 1000 | 0 | 2 | 0 | 1 | 1 | 0 |
| B57 | 1000 | 1 | 3 | 0 | 2 | 1 | 0 |
| B58 | 1000 | 1 | 2 | 0 | 2 | 1 | 0 |
| B59 | 1000 | 2 | 3 | 1 | 4 | 2 | 0 |
| B60 | 1000 | 1 | 2 | 0 | 4 | 1 | 0 |
| B61 | 1000 | 1 | 2 | 1 | 3 | 2 | 0 |
| B62 | 1000 | 1 | 3 | 1 | 3 | 2 | 0 |
| B63 | 1000 | 0 | 2 | 1 | 2 | 0 | 0 |
| B64 | 1000 | 0 | 2 | 0 | 1 | 0 | 0 |
| B65 | 1000 | 1 | 3 | 1 | 2 | 2 | 0 |
| B66 | 1000 | 1 | 5 | 0 | 3 | 2 | 0 |
| B67 | 1000 | 1 | 4 | 0 | 3 | 0 | 0 |
| B68 | 1000 | 1 | 4 | 1 | 3 | 2 | 0 |
| B69 | 1000 | 1 | 4 | 0 | 2 | 1 | 0 |
| B70 | 1000 | 0 | 2 | 0 | 1 | 0 | 0 |
| B71 | 1000 | 0 | 1 | 0 | 1 | 0 | 0 |
| B72 | 1000 | 0 | 4 | 0 | 2 | 0 | 0 |
| B73 | 1000 | 1 | 4 | 0 | 2 | 0 | 0 |
| B74 | 1000 | 1 | 5 | 1 | 4 | 2 | 1 |
| B76 | 1000 | 0 | 3 | 0 | 1 | 0 | 0 |
| B77 | 1000 | 0 | NT | 0 | 1 | 0 | 0 |
| B78 | 1000 | 1 | 4 | 0 | 2 | 0 | 0 |
| B79 | 1000 | 1 | 1 | 0 | 2 | 0 | 0 |
| B80 | 250 | 2 | 2 | 0 | 1 | 1 | 0 |
| B81 | 1000 | 1 | 5 | 1 | 5 | 1 | 0 |
| B83 | 1000 | 2 | 5 | 0 | 4 | 2 | 0 |
| B88 | 250 | 2 | 2 | 0 | 1 | 0 | 0 |
| B89 | 1000 | 1 | 5 | 0 | 4 | 2 | 0 |
| B90 | 250 | 1 | 5 | 0 | 4 | 1 | 0 |
| B91 | 250 | 1 | 5 | 0 | 2 | 1 | 1 |
| B92 | 250 | 0 | 5 | 0 | 1 | 1 | 0 |
| B93 | 250 | 1 | 5 | 0 | 4 | 2 | 0 |
| B94 | 1000 | 2 | 5 | 1 | 4 | 3 | 0 |
| B95 | 250 | 1 | 4 | 0 | 1 | 0 | 0 |
| B96 | 250 | 1 | 5 | 0 | 4 | 1 | 0 |
| B97 | 250 | 0 | 0 | 0 | 0 | 0 | 0 |
| B98 | 250 | 1 | 4 | 0 | 1 | 1 | 0 |
| B102 | 1000 | 2 | 5 | 0 | 4 | 3 | 0 |
| B104 | 250 | 1 | 5 | 0 | 2 | 0 | 0 |
| B105 | 1000 | 1 | 5 | 0 | 2 | 1 | 0 |
| B106 | 250 | 1 | 5 | 0 | 1 | 1 | 0 |
| B107 | 250 | 3 | 4 | 1 | 3 | 1 | 1 |
| B108 | 1000 | 2 | 5 | 0 | 3 | 2 | 0 |
| B109 | 1000 | 0 | 3 | 0 | 0 | 1 | 0 |
| B110 | 1000 | 0 | 4 | 0 | 3 | 0 | 0 |
| B111 | 1000 | 0 | 3 | 0 | 3 | 2 | 0 |
| B112 | 1000 | 0 | 4 | 0 | 3 | 0 | 0 |
| B113 | 1000 | 1 | 5 | 1 | 5 | 3 | 0 |
| B114 | 1000 | 1 | 5 | 0 | 5 | 1 | 0 |
| B115 | 1000 | 0 | 5 | 0 | 4 | 2 | 0 |
| B116 | 1000 | 1 | 5 | 0 | 4 | 2 | 0 |
| B117 | 1000 | 1 | 5 | 0 | 4 | 3 | 0 |
| B118 | 1000 | 0 | 4 | 0 | 2 | 0 | 0 |
| B119 | 1000 | 0 | 5 | 0 | 3 | 2 | 0 |
| B121 | 1000 | 0 | 4 | 0 | 2 | 0 | 0 |
| B123 | 1000 | 1 | 5 | 0 | 3 | 1 | 0 |

**Table B1b: Test 1b**

| **Compound ID** | **Rate (g/ha)** | **LOLPE** | **AMARE** | **SETFA** | **ECHCG** | **ZEAMX** | **ABUTH** |
|---|---|---|---|---|---|---|---|
| B13 | 1000 | 1 | 0 | 3 | 2 | 2 | 0 |
| B14 | 1000 | 0 | 0 | 0 | 0 | 0 | 4 |
| B15 | 1000 | 0 | 0 | 4 | 1 | 4 | 0 |
| B16 | 1000 | 1 | 0 | 4 | 3 | 5 | 0 |
| B17 | 1000 | 0 | 0 | 0 | 0 | 0 | 0 |
| B18 | 1000 | 0 | 1 | 1 | 0 | 0 | 1 |
| B19 | 1000 | 3 | 1 | 4 | 4 | 5 | 1 |
| B20 | 1000 | 1 | 1 | 4 | 5 | 5 | 0 |
| B21 | 1000 | 0 | 1 | 1 | 0 | 0 | 1 |
| B22 | 1000 | 1 | 1 | 4 | 3 | 0 | 1 |
| B23 | 1000 | 1 | 1 | 4 | 1 | 1 | 1 |
| B24 | 1000 | 0 | 1 | 1 | 0 | 1 | 1 |
| B25 | 1000 | 2 | 1 | 5 | 2 | 3 | 1 |
| B26 | 1000 | 0 | 0 | 4 | 1 | 5 | 0 |
| B27 | 1000 | 1 | 0 | 4 | 4 | 2 | 0 |
| B28 | 1000 | 1 | 0 | 2 | 2 | 3 | 0 |
| B29 | 1000 | 1 | 2 | 4 | 4 | 3 | 1 |
| B30 | 1000 | 1 | 2 | 4 | 3 | 5 | 0 |
| B31 | 1000 | 1 | 1 | 5 | 3 | 4 | 0 |
| B32 | 1000 | 1 | 0 | 5 | 3 | 5 | 0 |

Compounds that score 4 or 5 on one or more plant species are particularly preferred.

### B2 Post-emergence herbicidal activity

Seeds of a variety of test species were sown in standard soil in pots: *Triticum aestivium* (TRZAW), *Avena fatua* (AVEFA), *Alopecurus myosuroides* (ALOMY), *Echinochloa crus-galli* (ECHCG), *Lolium perenne* (LOLPE), *Zea Mays* (ZEAMX), *Abutilon theophrasti* (ABUTH), *Amaranthus retroflexus* (AMARE) and *Setaria faberi* (SETFA). After 8 days cultivation (post-emergence) under controlled conditions in a glasshouse (at 24/16°C, day/night; 14 hours light; 65% humidity), the plants were sprayed with an aqueous spray solution derived from the formulation of the technical active ingredient in acetone / water (50:50) solution containing 0.5% Tween 20 (polyoxyethelyene sorbitan monolaurate, CAS RN 9005-64-5). The test plants were then grown in a glasshouse under controlled conditions (at 24/16°C, day/night; 14 hours light; 65% humidity) and watered twice daily. After 13 days, the test was evaluated (5 = total damage to plant; 0 = no damage to plant). Results are shown in Tables B2a and B2b.

### Tables B2a and B2b Control of weed species by compound of Formula (I) after post-emergence application

**Table B2a Test 2a**

| **Compound ID** | **Rate (g/ha)** | **LOLPE** | **SETFA** | **ALOMY** | **ECHCG** | **AVEFA** | **TRAZW** |
|---|---|---|---|---|---|---|---|
| B1 | 1000 | 2 | 5 | 1 | 4 | 4 | 0 |
| B3 | 1000 | 0 | 3 | 0 | 2 | 2 | 0 |
| B4 | 1000 | 1 | 5 | 1 | 4 | 2 | 2 |
| B5 | 1000 | 1 | 4 | 1 | 3 | 1 | 0 |
| B6 | 1000 | 4 | 5 | 1 | 4 | 3 | 1 |
| B7 | 1000 | 2 | 5 | 1 | 4 | 4 | 1 |
| B9 | 1000 | 4 | 5 | 1 | 5 | 3 | 1 |
| B10 | 1000 | 3 | 5 | 1 | 4 | 3 | 1 |
| B11 | 1000 | 1 | 3 | 0 | 2 | 1 | 0 |
| B12 | 1000 | 2 | 3 | 1 | 2 | 2 | 1 |
| B33 | 1000 | 3 | 5 | 2 | 2 | 4 | 2 |
| B34 | 1000 | 1 | 4 | 0 | 5 | 2 | 1 |
| B35 | 1000 | 2 | 5 | 0 | 3 | 2 | 1 |
| B36 | 1000 | 1 | NT | 0 | 3 | 3 | 0 |
| B37 | 1000 | 2 | NT | 0 | 5 | 3 | 0 |
| B38 | 1000 | 2 | 4 | 0 | 2 | 2 | 0 |
| B39 | 1000 | 3 | 5 | 1 | 5 | 4 | 1 |
| B40 | 1000 | 1 | 3 | 0 | 1 | 2 | 0 |
| B41 | 1000 | 2 | 5 | 0 | 4 | 3 | 0 |
| B42 | 1000 | 3 | 5 | 1 | 5 | 4 | 2 |
| B43 | 1000 | 3 | 5 | 1 | 4 | 4 | 2 |
| B44 | 1000 | 2 | 4 | 1 | 4 | 3 | 1 |
| B45 | 1000 | 2 | 4 | 1 | 4 | 3 | 1 |
| B46 | 1000 | 2 | 4 | 0 | 4 | 3 | 0 |
| B47 | 1000 | 2 | 5 | 0 | 4 | 3 | 0 |
| B48 | 1000 | 1 | 5 | 0 | 4 | 2 | 0 |
| B49 | 1000 | 2 | 4 | 0 | 4 | 2 | 0 |
| B50 | 1000 | 1 | 5 | 0 | 4 | 3 | 0 |
| B51 | 1000 | 3 | 5 | 0 | 4 | 3 | 0 |
| B52 | 1000 | 3 | 5 | 0 | 4 | 3 | 0 |
| B53 | 1000 | 2 | 5 | 1 | 5 | 4 | 1 |
| B54 | 1000 | 3 | 4 | 1 | 4 | 4 | 1 |
| B55 | 1000 | 1 | 4 | 0 | 3 | 2 | 0 |
| B56 | 1000 | 3 | 4 | 0 | 4 | 3 | 0 |
| B57 | 1000 | 2 | 3 | 0 | 4 | 3 | 0 |
| B58 | 1000 | 2 | 5 | 0 | 3 | 2 | 0 |
| B59 | 1000 | 3 | 4 | 1 | 5 | 3 | 1 |
| B60 | 1000 | 2 | 5 | 1 | 5 | 2 | 1 |
| B61 | 1000 | 2 | 4 | 1 | 4 | 2 | 0 |
| B62 | 1000 | 2 | 4 | 0 | 5 | 3 | 0 |
| B63 | 1000 | 2 | 3 | 0 | 5 | 2 | 0 |
| B64 | 1000 | 1 | 4 | 0 | 4 | 3 | 0 |
| B65 | 1000 | 3 | 4 | 1 | 5 | 3 | 0 |
| B66 | 1000 | NT | 5 | 0 | 4 | NT | 1 |
| B67 | 1000 | 1 | 4 | 1 | 4 | 2 | 1 |
| B68 | 1000 | 2 | 5 | 0 | 5 | 3 | 2 |
| B69 | 1000 | 2 | 4 | 0 | 4 | 3 | 1 |
| B70 | 1000 | 1 | 3 | 0 | 3 | 1 | 1 |
| B71 | 1000 | 2 | 3 | 0 | 3 | 2 | 1 |
| B72 | 1000 | 1 | 4 | 1 | 4 | 1 | 1 |
| B73 | 1000 | 2 | 4 | 1 | 4 | 3 | 1 |
| B74 | 1000 | NT | 5 | 0 | 4 | NT | 0 |
| B76 | 1000 | 1 | 3 | 0 | 1 | 0 | 0 |
| B77 | 1000 | 1 | 2 | 0 | 1 | 1 | 0 |
| B78 | 1000 | 1 | 5 | 0 | 2 | 2 | 0 |
| B79 | 1000 | 1 | 2 | 0 | 1 | 2 | 0 |
| B80 | 250 | 1 | 2 | 0 | 1 | 2 | 0 |
| B81 | 1000 | 2 | 5 | 1 | 5 | 3 | 1 |
| B83 | 1000 | 3 | 5 | 0 | 5 | 4 | 1 |
| B88 | 250 | 0 | 1 | 0 | 1 | 1 | 0 |
| B89 | 1000 | 2 | 5 | 0 | 5 | 4 | 1 |
| B90 | 250 | 2 | 5 | 0 | 4 | 3 | 0 |
| B91 | 250 | 1 | 5 | 0 | 2 | 2 | 0 |
| B92 | 250 | 1 | 4 | 0 | 3 | 3 | 0 |
| B93 | 250 | 3 | 5 | 0 | 5 | 4 | 0 |
| B94 | 1000 | 3 | 5 | 0 | 5 | 3 | 0 |
| B95 | 250 | 1 | 5 | 0 | 2 | 3 | 0 |
| B96 | 250 | 2 | 5 | 0 | 5 | 3 | 0 |
| B97 | 250 | 1 | 1 | 0 | 1 | 2 | 0 |
| B98 | 250 | 0 | 5 | 0 | 2 | 3 | 0 |
| B102 | 1000 | 3 | 5 | 1 | 5 | 4 | 1 |
| B104 | 250 | 1 | 5 | 1 | 3 | 3 | 0 |
| B105 | 1000 | 1 | 5 | 0 | 4 | 3 | 0 |
| B106 | 250 | 1 | 5 | 0 | 3 | 3 | 0 |
| B107 | 250 | 1 | 5 | 0 | 3 | 3 | 0 |
| B108 | 1000 | 1 | 5 | 0 | 0 | 3 | 0 |
| B109 | 1000 | 0 | 1 | 0 | 1 | 1 | 0 |
| B110 | 1000 | 1 | 4 | 0 | 3 | 3 | 0 |
| B111 | 1000 | 1 | 4 | 0 | 3 | 2 | 0 |
| B112 | 1000 | 0 | 3 | 0 | 1 | 1 | 0 |
| B113 | 1000 | 3 | 5 | 1 | 5 | 4 | 2 |
| B114 | 1000 | 3 | 5 | 0 | 5 | 4 | 1 |
| B115 | 1000 | 3 | 5 | 1 | 5 | 4 | 1 |
| B116 | 1000 | 3 | 5 | 1 | 4 | 3 | 1 |
| B117 | 1000 | 4 | 5 | 1 | 5 | 4 | 3 |
| B118 | 1000 | 1 | 4 | 0 | 3 | 3 | 0 |
| B119 | 1000 | 3 | 5 | 0 | 3 | 4 | 0 |
| B121 | 1000 | 2 | 5 | 0 | 3 | 3 | 0 |
| B123 | 1000 | 1 | 5 | 2 | 3 | 2 | 1 |

**Table B2b: Test 2b**

| **Compound ID** | **Rate (g/ha)** | **LOLPE** | **AMARE** | **SETFA** | **ECHCG** | **ZEAMX** | **ABUTH** |
|---|---|---|---|---|---|---|---|
| B13 | 1000 | 2 | 1 | 5 | 3 | 5 | 0 |
| B14 | 1000 | 1 | 0 | 4 | 2 | 4 | 0 |
| B15 | 1000 | 2 | 1 | 4 | 3 | 5 | 0 |
| B16 | 1000 | 3 | 0 | 5 | 4 | 5 | 0 |
| B17 | 1000 | 0 | 0 | 1 | 1 | 1 | 0 |
| B18 | 1000 | 1 | 0 | 3 | 2 | 2 | 0 |
| B19 | 1000 | 4 | 2 | 5 | 4 | 5 | 1 |
| B20 | 1000 | 3 | 1 | 5 | 4 | 4 | 0 |
| B21 | 1000 | 1 | 0 | 3 | 2 | 2 | 0 |
| B22 | 1000 | 3 | 0 | 4 | 4 | 2 | 0 |
| B23 | 1000 | 2 | 1 | 3 | 2 | 5 | 1 |
| B24 | 1000 | 1 | 2 | 2 | 2 | 2 | 1 |
| B25 | 1000 | 4 | 2 | 5 | 3 | 4 | 2 |
| B26 | 1000 | 2 | 2 | 5 | 5 | 5 | 1 |
| B27 | 1000 | 2 | 2 | 5 | 4 | 3 | 2 |
| B28 | 1000 | 4 | 0 | 4 | 4 | 5 | 0 |
| B29 | 1000 | 3 | 1 | 5 | 4 | 5 | 1 |
| B30 | 1000 | 4 | 0 | 5 | 5 | 5 | 0 |
| B31 | 1000 | 2 | 1 | 5 | 4 | 5 | 1 |
| B32 | 1000 | 2 | 0 | 5 | 4 | 5 | 0 |

Compounds which score 4 or 5 on one or more plant species are particularly preferred.

## Claims

1. Use of a compound of Formula **(I)** 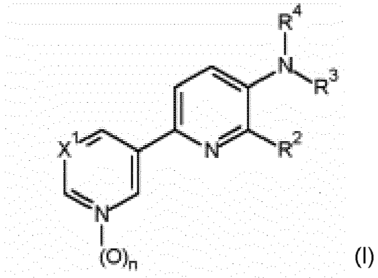 or a salt thereof, wherein,
X¹ is N or CR¹;
R¹ is selected from the group consisting of hydrogen, halogen, cyano, C₁-C₆alkyl, C₃-C₆cycloalkyl, C₂-C₆alkenyl, C₂-C₆alkynyl, C₁-C₆alkoxy, -C(O)OC₁-C₆alkyl, -S(O)ₚC₁-C₆alkyl, NR⁶R⁷, C₁-C₆haloalkoxy and C₁-C₆haloalkyl;
R² is selected from the group consisting of halogen, cyano, nitro, C₁-C₆alkyl, C₁-C₆haloalkyl, C₂-C₆alkenyl, C₂-C₆alkynyl, C₃-C₆cycloalkyl, -C(O)OC₁-C₆alkyl, -S(O)ₚ(C₁-C₆alkyl), C₁-C₆alkoxy, C₁-C₆haloalkoxy and phenyl;
R³ is -C(O)X²R¹²;
X² is O or NR¹⁰;
when X² is O, R¹² is selected from the group consisting of C₁-C₆alkyl, CᵣalkoxyCₛalkyl, C₁-C₆haloalkyl, CralkoxyCshaloalkyl, CralkylthioCₛalkyl, C₂-C₆alkenyl, C₂-C₆alkynyl, and - (CR^{a}R^{b})qR¹¹;
when X² is NR¹⁰, R¹² is selected from the group consisting of hydrogen, C₁-C₆alkyl, C₁-C₆alkoxy, C₁-C₆haloalkyl, C₁-C₆haloalkoxy, CralkylthioCₛalkyl, C₂-C₆alkenyl, C₂-C₆alkynyl, and -(CR^{a}R^{b})_{q}R¹¹;
R¹⁰ is selected from the group consisting of hydrogen, C₁-C₆alkyl, C₃-C₆ cycloalkyl;
or, R¹⁰ and R¹² together with the nitrogen atom to which they are joined, can form a 5-, 6-, or 7-membered ring, optionally containing 1 to 3 additional heteroatoms each independently selected from O, N or S, wherein when said ring contains a ring sulphur said ring sulphur is in the form S(O)ₚ ;
R⁴ is selected from the group consisting of hydrogen, C₁-C₆alkyl, C₁-C₆alkoxy, C₁-C₆haloalkyl, C₁-C₆haloalkoxy, C₃-C₆cyloalkyl, C₃-C₆alkenyl, C₃-C₆alkynyl, -C(O)R⁹ and - (CR^{a}R^{b})_{q}R⁵;
R^{a} is hydrogen or C₁-C₂ alkyl;
R^{b} is hydrogen or C₁-C₂ alkyl;
R⁵ is cyano, -C(O)OC₁-C₆alkyl, -C₃-C₆cycloalkyl, -aryl or -heteroaryl wherein said aryl and heteroaryl are optionally substituted by 1 to 3 independent R⁸;
R⁶ and R⁷ are independently selected from the group consisting of hydrogen and C₁-C₆alkyl;
each R⁸ is independently selected from the group consisting of halogen, C₁-C₆ alkyl and C₁-C₆alkoxy-, C₁-C₆ haloalkyl, C₁-C₆ haloalkoxy-, cyano and S(O)ₚ(C₁-C₆alkyl);
R⁹ is selected from the group consisting of hydrogen, C₁-C₆alkyl, C₁-C₆alkoxy, C₁-C₆haloalkyl, C₁-C₆haloalkoxy, C₂-C₆alkenyl, C₂-C₆alkynyl, and -(CR^{a}R^{b})_{q}R¹¹;
or R⁴ and R¹⁰ together with the atoms to which they are joined form a 5-7 membered ring system optionally containing from 1 to 3 additional heteroatoms independently selected from S, O and N;
or R⁴ and R¹² together with the atoms to which they are joined form a 5-7 membered ring system optionally containing from 1 to 3 additional heteroatoms independently selected from S, O and N;
R¹¹ is cyano, -C₃-C₆cycloalkyl, or an -aryl, -heteroaryl or -heterocyclyl ring, wherein said ring is optionally substituted by 1 to 3 independent R⁸, and wherein when said ring contains a ring sulphur, said ring sulphur is in the form S(O)ₚ;
n is 0 or 1;
p is 0, 1, or 2;
q is 0, 1, 2, 3, 4, 5 or 6;
r is 1, 2, 3, 4, or 5, s is 1, 2, 3, 4, or 5, and the sum of r+s is less than or equal to 6,
as a herbicide,
with the proviso that the compound of Formula (I) is not
(i) *tert*-butyl N-[2-methyl-6-(3-pyridyl)-3-pyridyl]carbamate.

2. The use according to claim 1, wherein X¹ is N.

3. The use according to claim 1, wherein X¹ is CR¹ and R¹ is selected from the group consisting of hydrogen, cyano, halogen, C₁-C₃alkyl, C₃-C₄alkynyl, C₁-C₃alkoxy, C₁-C₃haloalkyl, C₁-C₃haloalkoxy, and C₁-C₃thioalkyl.

4. The use according to any one of the preceding claims, wherein R² is halogen, cyano, C₁-C₆alkyl, C₁-C₆haloalkyl, C(O)OC₁-C₆alkyl or phenyl.

5. The use according to any one of the preceding claims, wherein X² is O.

6. The use according to claim 5, wherein R¹² is selected from the group consisting of: C₁-C₆alkyl, CralkoxyCsalkyl, C₁-C₆haloalkyl, CralkoxyCshaloalkyl, CralkylthioCₛalkyl, C₂-C₆alkenyl, C2-C6alkynyl, and
-(CR^{a}R^{b})_{q}R¹¹

7. The use according to any one of claims 1 to 4, wherein X² is NR¹⁰.

8. The use according to claim 7, wherein R¹⁰ is: hydrogen or C₁-C₆alkyl;
or wherein R¹⁰ together with R⁴ and the atoms to which R¹⁰ and R⁴ are joined form a 5-7 membered ring system optionally containing from 1 to 3 additional heteroatoms independently selected from S, O and N;
or wherein R¹⁰ together with R¹² and the nitrogen atom to which R¹⁰ and R¹² are joined form a 5-7 membered ring system optionally containing from 1 to 3 additional heteroatoms independently selected from S in the form of S(O)ₚ, O and N.

9. The use according to any one of the preceding claims, wherein R⁴ is selected from the group consisting of hydrogen, methyl, ethyl, allyl, but-2-ynyl, C(O)R⁹ and -(CH₂)_{q}R⁵.

10. A compound of formula (I) as defined in claim 1, wherein X² is NR¹⁰ and either:
R¹⁰ together with R⁴ and the atoms to which R¹⁰ and R⁴ are joined form a 5-7 membered ring system optionally containing from 1 to 3 additional heteroatoms independently selected from S, O and N,
or R¹⁰ together with R¹² and the nitrogen atom to which R¹⁰ and R¹² are joined form a 5-7 membered ring system optionally containing from 1 to 3 additional heteroatoms independently selected from S in the form of S(O)ₚ, O and N.

11. A herbicidal composition comprising a herbicidally effective amount of a compound according to claim 10 and an agriculturally acceptable formulation adjuvant.

12. The herbicidal composition according to claim 11, further comprising at least one additional pesticide.

13. The herbicidal composition according to claim 12, wherein the additional pesticide is a herbicide or herbicide safener.

14. A method of controlling weeds at a locus comprising application to the locus of a weed controlling amount of a compound of formula (I) as defined in any one of claims 1 to 10, or a weed controlling amount of a composition as defined in any one of claims 11 to 13.

## Patentansprüche

1. Verwendung einer Verbindung der Formel (I) 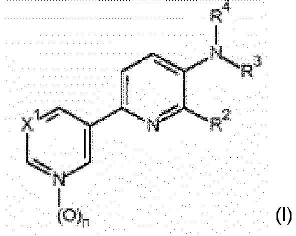 oder eines Salzes davon, wobei:
X¹ für N oder CR¹ steht;
R¹ aus der Gruppe bestehend aus Wasserstoff, Halogen, Cyano, C₁-C₆-Alkyl, C₃-C₆-Cycloalkyl, C₂-C₆-Alkenyl, C₂-C₆-Alkinyl, C₁-C₆-Alkoxy, -C(O)O-C₁-C₆-Alkyl, -S(O)ₚ-C₁-C₆-Alkyl, NR⁶R⁷, C₁-C₆-Halogenalkoxy und C₁-C₆-Halogenalkyl ausgewählt ist;
R² aus der Gruppe bestehend aus Halogen, Cyano, Nitro, C₁-C₆-Alkyl, C₁-C₆-Halogenalkyl, C₂-C₆-Alkenyl, C₂-C₆-Alkinyl, C₃-C₆-Cycloalkyl, -C(O)O-C₁-C₆-Alkyl, -S(O)ₚ-C₁-C₆-Alkyl, C₁-C₆-Alkoxy, C₁-C₆-Halogenalkoxy und Phenyl ausgewählt ist;
R³ für -C(O)X²R¹² steht;
X² für O oder NR¹⁰ steht;
dann, wenn X² für O steht, R¹² aus der Gruppe bestehend aus C₁-C₆-Alkyl, Cᵣ-Alkoxy-Cₛ-alkyl, C₁-C₆-Halogenalkyl, Cᵣ-Alkoxy-Cₛ-halogenalkyl, Cᵣ-Alkylthio-Cₛ-alkyl, C₂-C₆-Alkenyl, C₂-C₆-Alkinyl und -(CR^{a}R^{b})_{q}R¹¹ ausgewählt ist;
dann, wenn X² für NR¹⁰ steht, R¹² aus der Gruppe bestehend aus Wasserstoff, C₁-C₆-Alkyl, C₁-C₆-Alkoxy, C₁-C₆-Halogenalkyl, C₁-C₆-Halogenalkoxy, Cᵣ-Alkylthio-Cₛ-alkyl, C₂-C₆-Alkenyl, C₂-C₆-Alkinyl und -(CR^{a}R^{b})_{q}R¹¹ ausgewählt ist;
R¹⁰ aus der Gruppe bestehend aus Wasserstoff, C₁-C₆-Alkyl und C₃-C₆-Cycloalkyl ausgewählt ist;
oder R¹⁰ und R¹² zusammen mit dem Stickstoffatom, an das sie gebunden sind, einen 5-, 6- oder 7-gliedrigen Ring, der gegebenenfalls 1 bis 3 zusätzliche Heteroatome, die jeweils unabhängig aus O, N oder S ausgewählt sind, enthält, bilden können, wobei dann, wenn der Ring einen Ringschwefel enthält, der Ringschwefel in der Form S(O)ₚ vorliegt;
R⁴ aus der Gruppe bestehend aus Wasserstoff, C₁-C₆-Alkyl, C₁-C₆-Alkoxy, C₁-C₆-Halogenalkyl, C₁-C₆-Halogenalkoxy, C₃-C₆-Cycloalkyl C₃-C₆-Alkenyl, C₃-C₆-Alkinyl, -C(O)R⁹ und -(CR^{a}R^{b})_{q}R⁵ ausgewählt ist;
R^{a} für Wasserstoff oder C₁-C₂-Alkyl steht;
R^{b} für Wasserstoff oder C₁-C₂-Alkyl steht;
R⁵ für Cyano, -C(O)O-C₁-C₆-Alkyl, -C₃-C₆-Cycloalkyl, -Aryl oder -Heteroaryl steht, wobei das Aryl und Heteroaryl gegebenenfalls durch 1 bis 3 unabhängige R⁸ substituiert sind;
R⁶ und R⁷ unabhängig aus der Gruppe bestehend aus Wasserstoff und C₁-C₆-Alkyl ausgewählt sind;
R⁸ jeweils unabhängig aus der Gruppe bestehend aus Halogen, C₁-C₆-Alkyl und C₁-C₆-Alkoxy-, C₁-C₆-Halogenalkyl, C₁-C₆-Halogenalkoxy-, Cyano und S(O)ₚ-C₁-C₆-Alkyl ausgewählt ist;
R⁹ aus der Gruppe bestehend aus Wasserstoff, C₁-C₆-Alkyl, C₁-C₆-Alkoxy, C₁-C₆-Halogenalkyl, C₁-C₆-Halogenalkoxy, C₂-C₆-Alkenyl, C₂-C₆-Alkinyl und -(CR^{a}R^{b})_{q}R¹¹ ausgewählt ist;
oder R⁴ und R¹⁰ zusammen mit den Atomen, an die sie gebunden sind, ein 5-7-gliedriges Ringsystem, das gegebenenfalls 1 bis 3 zusätzliche Heteroatome, die jeweils unabhängig aus S, O und N ausgewählt sind, enthält, bilden;
oder R⁴ und R¹² zusammen mit den Atomen, an die sie gebunden sind, ein 5-7-gliedriges Ringsystem, das gegebenenfalls 1 bis 3 zusätzliche Heteroatome, die jeweils unabhängig aus S, O und N ausgewählt sind, enthält, bilden;
R¹¹ für Cyano, -C₃-C₆-Cycloalkyl oder einen -Aryl-, -Heteroaryl- oder -Heterocyclylring steht, wobei der Ring gegebenenfalls durch 1 bis 3 unabhängige R⁸ substituiert ist und wobei dann, wenn der Ring einen Ringschwefel enthält, der Ringschwefel in der Form S(O)ₚ vorliegt;
n für 0 oder 1 steht;
p für 0, 1 oder 2 steht;
q für 0, 1, 2, 3, 4, 5 oder 6 steht;
r für 1, 2, 3, 4 oder 5 steht, s für 1, 2, 3, 4 oder 5 steht und die Summe von r + s kleiner als oder gleich 6 ist,
als Herbizid,
mit der Maßgabe, dass es sich bei der Verbindung der Formel (I) nicht um
(i) N-[2-Methyl-6-(3-pyridyl)-3-pyridyl]carbamidsäure-tert-butylester
handelt.

2. Verwendung nach Anspruch 1, wobei X¹ für N steht.

3. Verwendung nach Anspruch 1, wobei X¹ für CR¹ steht und R¹ aus der Gruppe bestehend aus Wasserstoff, Cyano, Halogen, C₁-C₃-Alkyl, C₃-C₄-Alkinyl, C₁-C₃-Alkoxy, C₁-C₃-Halogenalkyl, C₁-C₃-Halogenalkoxy und C₁-C₃-Thioalkyl ausgewählt ist.

4. Verwendung nach einem der vorhergehenden Ansprüche, wobei R² für Halogen, Cyano, C₁-C₆-Alkyl, C₁-C₆-Halogenalkyl, C(O)O-C₁-C₆-Alkyl oder Phenyl steht.

5. Verwendung nach einem der vorhergehenden Ansprüche, wobei X² für O steht.

6. Verwendung nach Anspruch 5, wobei R¹² aus der Gruppe bestehend aus C₁-C₆-Alkyl, Cᵣ-Alkoxy-Cₛalkyl, C₁-C₆-Halogenalkyl, Cᵣ-Alkoxy-Cₛhalogenalkyl, Cᵣ-Alkylthio-Cₛ-alkyl, C₂-C₆-Alkenyl, C₂-C₆-Alkinyl und -(CR^{a}R^{b})_{q}R¹¹ ausgewählt ist.

7. Verwendung nach einem der Ansprüche 1 bis 4, wobei X² für NR¹⁰ steht.

8. Verwendung nach Anspruch 7, wobei R¹⁰ für Wasserstoff oder C₁-C₆-Alkyl steht;
oder wobei R¹⁰ zusammen mit R⁴ und den Atomen, an die R¹⁰ und R⁴ gebunden sind, ein 5-7-gliedriges Ringsystem, das gegebenenfalls 1 bis 3 zusätzliche Heteroatome, die jeweils unabhängig aus S, O und N ausgewählt sind, enthält, bildet;
oder wobei R¹⁰ zusammen mit R¹² und dem Stickstoffatom, an das R¹⁰ und R¹² gebunden sind, ein 5-7-gliedriges Ringsystem, das gegebenenfalls 1 bis 3 zusätzliche Heteroatome, die jeweils unabhängig aus S in der Form von S(O)ₚ, O und N ausgewählt sind, enthält, bildet.

9. Verwendung nach einem der vorhergehenden Ansprüche, wobei R⁴ aus der Gruppe bestehend aus Wasserstoff, Methyl, Ethyl, Allyl, But-2-inyl, C(O)R⁹ und -(CH₂)_{q}R⁵ ausgewählt ist.

10. Verbindung der Formel (I) gemäß Anspruch 1, wobei X² für NR¹⁰ steht und entweder:
R¹⁰ zusammen mit R⁴ und den Atomen, an die R¹⁰ und R⁴ gebunden sind, ein 5-7-gliedriges Ringsystem, das gegebenenfalls 1 bis 3 zusätzliche Heteroatome, die jeweils unabhängig aus S, O und N ausgewählt sind, enthält, bildet,
oder R¹⁰ zusammen mit R¹² und dem Stickstoffatom, an das R¹⁰ und R¹² gebunden sind, ein 5-7-gliedriges Ringsystem, das gegebenenfalls 1 bis 3 zusätzliche Heteroatome, die jeweils unabhängig aus S in der Form von S(O)ₚ, O und N ausgewählt sind, enthält, bildet.

11. Herbizide Zusammensetzung, umfassend eine herbizid wirksame Menge einer Verbindung nach Anspruch 10 und ein landwirtschaftlich unbedenkliches Formulierungshilfsmittel.

12. Herbizide Zusammensetzung nach Anspruch 11, ferner umfassend mindestens ein zusätzliches Pestizid.

13. Herbizide Zusammensetzung nach Anspruch 12, wobei es sich bei dem zusätzlichen Pestizid um ein Herbizid oder einen Herbizid-Safener handelt.

14. Verfahren zur Bekämpfung von Unkräutern an einem Standort, bei dem man eine unkrautbekämpfende Menge einer Verbindung der Formel (I) gemäß einem der Ansprüche 1 bis 10 oder eine unkrautbekämpfende Menge einer Zusammensetzung gemäß einem der Ansprüche 11 bis 13 auf den Standort ausbringt.

## Revendications

1. Utilisation d'un composé de formule (I) 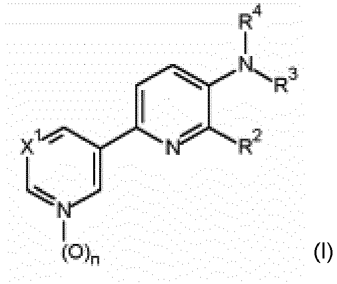 ou un sel de celui-ci, dans lequel,
X¹ est N ou CR¹ ;
R¹ est choisi dans le groupe constitué d'hydrogène, halogène, cyano, alkyle en C₁-C₆, -(cycloalkyle en C₃-C₆), alcényle en C₂-C₆, alcynyle en C₂-C₆, alcoxy en C₁-C₆, -C(O)O (alkyle en C₁-C₆), -S(O)ₚ(alkyle en C₁-C₆), NR⁶R⁷, halogénoalcoxy en C₁-C₆ et halogénoalkyle en C₁-C₆ ; R² est choisi dans le groupe constitué d'halogène, cyano, nitro, alkyle en C₁-C₆, halogénoalkyle en C₁-C₆, alcényle en C₂-C₆, alcynyle en C₂-C₆, cycloalkyle en C₃-C₆, -C(O)O(alkyle en C₁-C₆), -S(O)ₚ(alkyle en C₁-C₆), alcoxy en C₁-C₆, halogénoalcoxy en C₁-C₆ et phényle ;
R³ est -C(O)X²R¹² ;
X² est O ou NR¹⁰ ;
lorsque X² est O, R¹² est choisi dans le groupe constitué d'alkyle en C₁-C₆, (alcoxy en Cᵣ) (alkyle en Cₛ), halogénoalkyle en C₁-C₆, (alcoxy en Cᵣ) (halogénoalkyle en Cₛ), (alkylthio en Cᵣ) (alkyle en Cₛ), alcényle en C₂-C₆, alcynyle en C₂-C₆ et - (CR^{a}R^{b}) _{q}R¹¹ ;
lorsque X² est NR¹⁰, R¹² est choisi dans le groupe constitué d'hydrogène, alkyle en C₁-C₆, alcoxy en C₁-C₆, halogénoalkyle en C₁-C₆, halogénoalcoxy en C₁-C₆, (alkylthio en Cᵣ) (alkyle en Cₛ), alcényle en C₂-C₆, alcynyle en C₂-C₆ et -(CR^{a}R^{b})_{q}R¹¹ ;
R¹⁰ est choisi dans le groupe constitué d'hydrogène, alkyle en C₁-C₆, cycloalkyle en C₃-C₆ ;
ou, R¹⁰ et R¹², conjointement avec l'atome d'azote auquel ils sont liés, peuvent former un cycle de 5, 6 ou 7 chaînons, contenant facultativement 1 à 3 hétéroatomes supplémentaires, chacun indépendamment choisi parmi O, N ou S, où, lorsque ledit cycle contient un soufre cyclique, ledit soufre cyclique est sous la forme S(O)ₚ ;
R⁴ est choisi dans le groupe constitué d'hydrogène, alkyle en C₁-C₆, alcoxy en C₁-C₆, halogénoalkyle en C₁-C₆, halogénoalcoxy en C₁-C₆, cycloalkyle en C₃-C₆, alcényle en C₃-C₆, alcynyle en C₃-C₆, -C(O)R⁹ et - (CR^{a}R^{b})_{q}R⁵ ;
R^{a} est hydrogène ou alkyle en C₁-C₂ ;
R^{b} est hydrogène ou alkyle en C₁-C₂ ;
R⁵ est cyano, -C(O)O (alkyle en C₁-C₆), -(cycloalkyle en C₃-C₆), -aryle ou-hétéroaryle où lesdits aryle et hétéroaryle sont facultativement substitués par 1 à 3 R⁸ indépendants ;
R⁶ et R⁷ sont indépendamment choisis dans le groupe constitué d'hydrogène et alkyle en C₁-C₆ ;
chaque R⁸ est indépendamment choisi dans le groupe constitué d'halogène, alkyle en C₁-C₆ et (alcoxy en C₁-C₆)-, halogénoalkyle en C₁-C₆, (halogénoalcoxy en C₁-C₆)-, cyano et S(O)ₚ(alkyle en C₁-C₆) ;
R⁹ est choisi dans le groupe constitué d'hydrogène, alkyle en C₁-C₆, alcoxy en C₁-C₆, halogénoalkyle en C₁-C₆, halogénoalcoxy en C₁-C₆, alcényle en C₂-C₆, alcynyle en C₂-C₆ et - (CR^{a}R^{b})_{q}R¹¹ ;
ou R⁴ et R¹⁰, conjointement avec les atomes auxquels ils sont liés, forment un système cyclique de 5 à 7 chaînons contenant facultativement de 1 à 3 hétéroatomes supplémentaires indépendamment choisis parmi S, O et N ;
ou R⁴ et R¹², conjointement avec les atomes auxquels ils sont liés, forment un système cyclique de 5 à 7 chaînons contenant facultativement de 1 à 3 hétéroatomes supplémentaires indépendamment choisis parmi S, O et N ; R¹¹ est cyano, -(cycloalkyle en C₃-C₆), ou un cycle -aryle, -hétéroaryle ou -hétérocyclyle, où ledit cycle est facultativement substitué par 1 à 3 R⁸ indépendants, et où, lorsque ledit cycle contient un soufre cyclique, ledit soufre cyclique est sous la forme S(O)ₚ ;
n est 0 ou 1 ;
p est 0, 1 ou 2 ;
q est 0, 1, 2, 3, 4, 5 ou 6 ;
r est 1, 2, 3, 4 ou 5, s est 1, 2, 3, 4 ou 5, et la somme de r+s est inférieure ou égale à 6,
en tant qu'herbicide,
à condition que le composé de formule (I) ne soit pas (i) N-[2-méthyl-6-(3-pyridyl)-3-pyridyl]carbamate de tert-butyle.

2. Utilisation selon la revendication 1, dans laquelle X¹ est N.

3. Utilisation selon la revendication 1, dans laquelle X¹ est CR¹ et R¹ est choisi dans le groupe constitué d'hydrogène, cyano, halogène, alkyle en C₁-C₃, alcynyle en C₃-C₄, alcoxy en C₁-C₃, halogénoalkyle en C₁-C₃, halogénoalcoxy en C₁-C₃ et thioalkyle en C₁-C₃.

4. Utilisation selon l'une quelconque des revendications précédentes, dans laquelle R² est halogène, cyano, alkyle en C₁-C₆, halogénoalkyle en C₁-C₆, C(O)O(alkyle en C₁-C₆) ou phényle.

5. Utilisation selon l'une quelconque des revendications précédentes, dans laquelle X² est O.

6. Utilisation selon la revendication 5, dans laquelle R¹² est choisi dans le groupe constitué de : alkyle en C₁-C₆, (alcoxy en Cᵣ) (alkyle en Cₛ), halogénoalkyle en C₁-C₆, (alcoxy en Cᵣ) (halogénoalkyle en Cₛ), (alkylthio en Cᵣ) (alkyle en Cₛ), alcényle en C₂-C₆, alcynyle en C₂-C₆, et
-(CR^{a}R^{b})_{q}R¹¹.

7. Utilisation selon l'une quelconque des revendications 1 à 4, dans laquelle X² est NR¹⁰.

8. Utilisation selon la revendication 7, dans laquelle R¹⁰ est : hydrogène ou alkyle en C₁-C₆ ;
ou dans laquelle R¹⁰, conjointement avec R⁴ et les atomes auxquels R¹⁰ et R⁴ sont liés, forment un système cyclique de 5 à 7 chaînons contenant facultativement de 1 à 3 hétéroatomes supplémentaires indépendamment choisis parmi S, O et N ;
ou dans laquelle R¹⁰, conjointement avec R¹² et l'atome d'azote auquel R¹⁰ et R¹² sont liés, forment un système cyclique de 5 à 7 chaînons contenant facultativement de 1 à 3 hétéroatomes supplémentaires indépendamment choisis parmi S sous la forme de S(O)ₚ, O et N.

9. Utilisation selon l'une quelconque des revendications précédentes, dans laquelle R⁴ est choisi dans le groupe constitué d'hydrogène, méthyle, éthyle, allyle, but-2-ynyle, C(O)R⁹ et -(CH₂)_{q}R⁵.

10. Composé de formule (I) tel que défini dans la revendication 1, dans lequel X² est NR¹⁰ et soit :
R¹⁰, conjointement avec R⁴ et les atomes auxquels R¹⁰ et R⁴ sont liés, forment un système cyclique de 5 à 7 chaînons contenant facultativement de 1 à 3 hétéroatomes supplémentaires indépendamment choisis parmi S, O et N, ou R¹⁰, conjointement avec R¹² et l'atome d'azote auquel R¹⁰ et R¹² sont liés, forment un système cyclique de 5 à 7 chaînons contenant facultativement de 1 à 3 hétéroatomes supplémentaires indépendamment choisis parmi S sous la forme de S(O)ₚ, O et N.

11. Composition herbicide comprenant une quantité herbicide efficace d'un composé selon la revendication 10 et un adjuvant de formulation acceptable en agriculture.

12. Composition herbicide selon la revendication 11, comprenant en outre au moins un pesticide supplémentaire.

13. Composition herbicide selon la revendication 12, dans laquelle le pesticide supplémentaire est un herbicide ou un phytoprotecteur.

14. Procédé de lutte contre les mauvaises herbes à un emplacement comprenant l'application sur l'emplacement d'une quantité efficace contre les mauvaises herbes d'un composé de formule (I) tel que défini dans l'une quelconque des revendications 1 à 10, ou d'une quantité efficace contre les mauvaises herbes d'une composition telle que définie dans l'une quelconque des revendications 11 à 13.
